# EUROPEAN PATENT APPLICATION

(11) **EP 1 806 584 A1**
(43) Date of publication of application: **11.07.2007**
(21) Application number: 07250072.1
(22) Date of filing: 09.01.2007
(51) Int. Cl.: G01N 33/543

(54) **Compositions for separation of a plurality of distinct targets from a sample**

(30) Priority: 09.01.2006 US 328403; 27.10.2006 US 553729
(71) Applicant: Sigma-Aldrich Co., St Louis, Missouri 63103 (US)
(72) Inventor: Schuchard, Mark D., Eureka, Missouri 63025 (US); Melm, Christopher D., Glen Carbon, Illinois 62034 (US); Crawford, Angela S., Arnold, Missouri 63010 (US); Chapman, Holly A., St. Louis, Missouri 63104 (US); Chen, D. Er, Chesterfield, Missouri 63017 (US); Dapron, John G., St. Louis, Missouri 63129 (US); Mehigh, Richard J., St. Louis, Missouri 63129 (US); Scott, Graham B. I., Wentzville, Missouri 63385 (US)
(74) Representative: Roberts, Michael Austin

(57) **Abstract**

The present invention generally relates to compositions that may be used to separate targets from non-targets. More specifically, the present invention relates to the separation of at least two distinct targets from a sample containing a mixture of targets and non-targets by contacting the sample with a composition comprising a plurality of heterogeneous epitope binding agents having affinity for the distinct targets.

## Description

### FIELD OF THE INVENTION

The present invention generally relates to compositions that may be used to separate targets from non-targets. More specifically, the present invention relates to the separation of at least two distinct targets from a sample containing a mixture of targets and non-targets by contacting the sample with a composition comprising a plurality of heterogeneous epitope binding agents having affinity for the distinct targets.

### BACKGROUND OF THE INVENTION

Diagnostic, research and therapeutic applications often require the purification or separation of one or more targets from more complex mixtures, for example mixtures derived from biological tissues, biological fluids, cell cultures, environmental samples and other samples. Various separation techniques typically exploit differences in physical properties between targets in the mixture, such as affinity for a particular ligand, charge, hydrophobicity, size, or shape.

The process of purification or fractionation generally involves the binding and separation of a target on a stationary phase or other medium. The non-target fraction of the mixture will not become bound. If desired, the target can be recovered from the solid medium in a process known as elution. For example, in fractionation based on affinity, a mixture containing a target is contacted with an affinity matrix that binds the target. The affinity matrix may comprise any number of epitope binding agents with affinity for the target, such as aptamers, antibodies, or other proteins. Affinity fractionation or purification may take various forms, for example column chromatography. In column chromatography, a sample containing a mixture of targets is typically passed through an affinity matrix comprising a stationary bed of chromatographic resin; in general, targets having relatively less affinity for the resin elute first, followed by biomolecules having relatively greater affinity for the resin. Note that in some types of affinity chromatography, the capture or binding of target molecule(s) can be engineered to be essentially quantitative, i.e. approximating an ON/OFF switch. The captured target molecules are then typically eluted in substantially one step by disrupting the chemical bonds between resin and targets via the use of an appropriate "competing" reagent (e.g., imidazole in the case of chromatographic resins for purifying HIS-tagged proteins). Alternatively, in batch chromatography, an affinity matrix comprising free-flowing chromatographic resin is added directly to a sample to form a suspension of the resin in the sample and the suspension is then agitated or gently mixed. The target-bound resin is removed from the remainder of the sample by centrifugation or filtration, leaving unbound targets in solution.

The process of separation is particularly useful in the study of regulatory proteins, cytokines, biomarkers, drug targets, or any other biomolecules that may be masked in a sample by more abundant biomolecules, such as albumin. In plasma, for example, the dozen or so most abundant proteins, accounting for more than 95% of the mass of total plasma and present at the mg/mL level, constitute less than 0.1 % of the various types of proteins. In contrast, low abundance proteins, such as biomarkers for various diseases, are typically present at ng/mL or pg/mL levels. Most analytical techniques used to study low abundance proteins, such as two dimensional gel electrophoresis (2DE) and mass spectrometry (MS), require processes for first separating abundant biomolecules from the non-abundant biomolecules in order to detect and visualize low abundance biomarkers.

Certain affinity separation compositions have been used to deplete multiple high abundance targets from a sample. These separation compositions, however, have significant disadvantages. Typically the compositions are only capable of separating a small fraction of the high abundance targets present in a sample. The compositions are also expensive because they are typically made by generating a series of individual antibody resins having the antibody conjugated to the resin in an oriented manner. The individual resins are then combined in order to produce the final product. As such, there is a need for cost effective separation compositions that are capable of efficiently separating a significant percentage of high abundance targets from a sample.

### SUMMARY OF THE INVENTION

According to the present invention there is provided a composition, process, method and kit as defined in the accompanying claims. In further detail, among the several aspects of the current invention is the provision of a separation composition that may be utilized to separate a plurality of distinct targets, such as high abundance targets, from a sample. The separation composition of the invention comprises a plurality of heterogeneous epitope binding agents (such as antibodies) stochastically conjugated *en masse* to a solid support. Because the separation composition is made by conjugating the epitope binding agents (e.g. antibodies) *en masse* to the solid support in a stochastic manner, the composition is produced in a cost effective and efficient manner compared to separation compositions where individual epitope binding agents (e.g. antibodies) are conjugated to a resin in an oriented manner and then the resins are mixed to form the final product. Advantageously, the separation compositions of the present invention typically have higher binding capacities and, as such, can be used to separate increased numbers of distinct targets relative to separation compositions made via the aforementioned multi step process.

Another aspect of the invention encompasses a process for preparing a composition capable of separating at least two distinct targets from a sample comprising a mixture of targets and non-targets. The process generally comprises combining a plurality of heterogeneous epitope binding agents (e.g. antibodies) to form an epitope binding agent (e.g. antibody) mixture. The epitope binding agent (e.g. antibody mixture) is contacted with a solid support under conditions such that the plurality of epitope binding agents (such as antibodies) are stochastically conjugated *en masse* to the solid support.

A further aspect of the invention provides a method for separating at least two distinct targets from a sample comprising a mixture of targets and non-targets. The method comprises contacting the sample with a separation composition of the invention comprising a plurality of heterogeneous epitope binding agents (e.g. antibodies) stochastically conjugated *en masse* to a solid support. The epitope binding agents (e.g. antibodies) have affinity for the targets, such that when they contact the targets, they bind the targets, thereby separating the targets from the non-targets.

Yet another aspect of the invention provides a method for separating at least two distinct targets from a sample and simultaneously fractionating at least two distinct non-targets from the sample. The method comprises contacting the sample with a composition comprising a plurality of heterogeneous epitope binding agents (e.g. antibodies) stochastically conjugated *en mass* to a solid support. The epitope binding agents (e.g. antibodies) have affinity for the distinct targets, such that when the epitope binding agents (e.g. antibodies) contact the targets they bind to the targets, thereby separating the targets from the sample. The method further comprises collecting at least two fractions of the target-depleted sample, such that each fraction comprises at least one distinct non-target.

Another aspect of the invention encompasses a *complete* kit, (including all concomitant equilibration and elution buffers etc.) for the separation of at least two distinct targets from a sample comprising a mixture of targets and non-targets. The *complete* kit comprises a separation composition of the invention, all purpose designed and built companion solutions, (including preservatives), equilibration and elution buffers and detailed instructions for use.

Other aspects and features of the invention will be in part apparent and in part pointed out hereinafter.

### BRIEF DESCRIPTION OF THE FIGURES

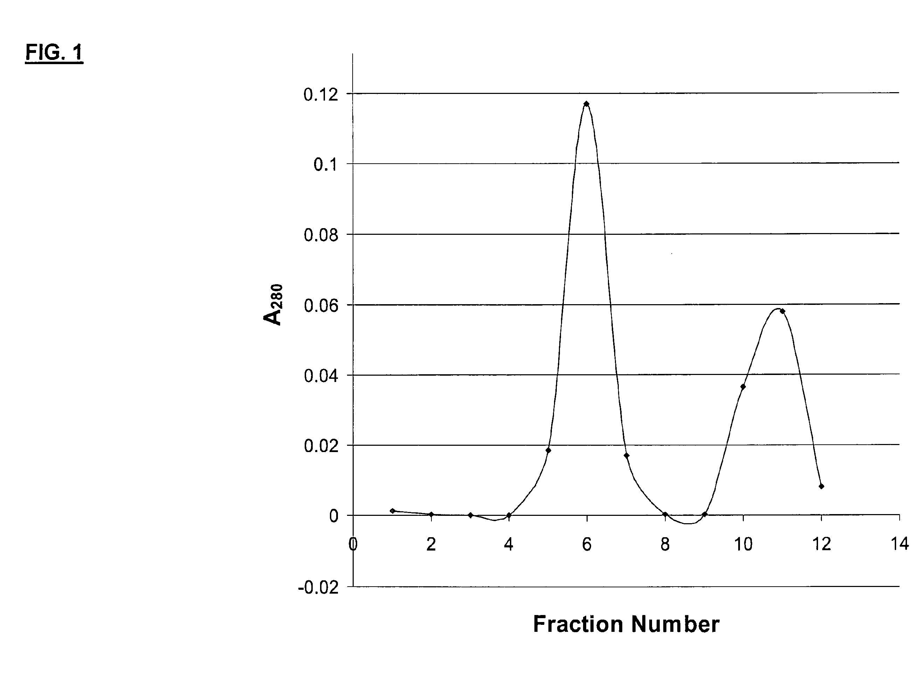

**Figure 1** is a plot depicting the absorbance at 280 nm (A₂₈₀) of fractions collected during gel filtration of S-acetylthioacetic acid succinimide (SATA)-activated antibodies over a Sephadex G25 column.

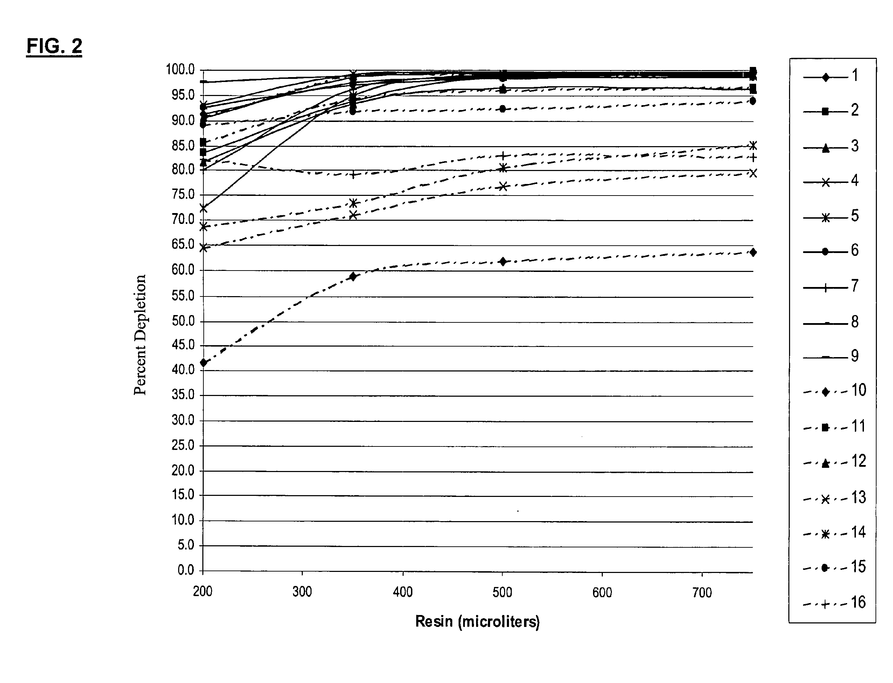

**Figure 2** is a plot illustrating the depletion of sixteen high abundance plasma proteins as a function of resin volume. A fixed volume of plasma (25 µl) was applied to various volumes of the antibody-conjugated resin (200, 350, 500 and 750 µl). The percent depletion was determined using ELISA analyses. 1 = IgG, 2 = human serum albumin (HSA), 3 = alpha-2-macroglobulin, 4 = IgM, 5 = IgA, 6 = fibrinogen, 7 = alpha-1-antitrypsin, 8 = transferrin, 9 = haptoglobin, 10 = alpha-1-acid glycoprotein, 11 = ceruloplasmin, 12 = IgD, 13 = apolipoprotein A1 (ApoA1), 14 = complement C3, 15 = complement C1q, and 16 = HDL.

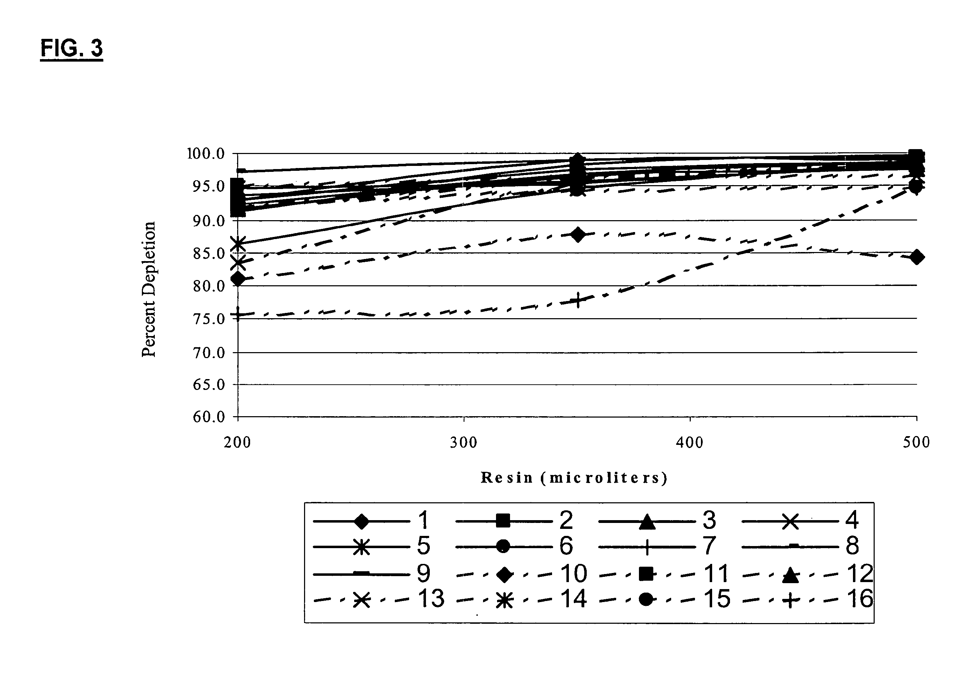

**Figure 3** is a plot depicting the depletion of sixteen high abundance plasma proteins as a function of resin volume. The amounts of some antibodies conjugated to the resin were adjusted relative to the amounts conjugated to the resin used in Figure 2. A fixed volume of plasma (25 µl) was applied to various volumes of the antibody-conjugated resin (200, 350, and 500 µl). The percent depletion was determined using ELISA analyses. 1 = lgG, 2 = HSA, 3 = alpha-2-macroglobulin, 4 = lgM, 5 = lgA, 6 = fibrinogen, 7 = alpha-1-antitrypsin, 8 = transferrin, 9 = haptoglobin, 10 = alpha-1-acid glycoprotein, 11 = ceruloplasmin, 12 = lgD, 13 = ApoA1, 14 = complement C3, 15 = complement C1q, and 16 = HDL.

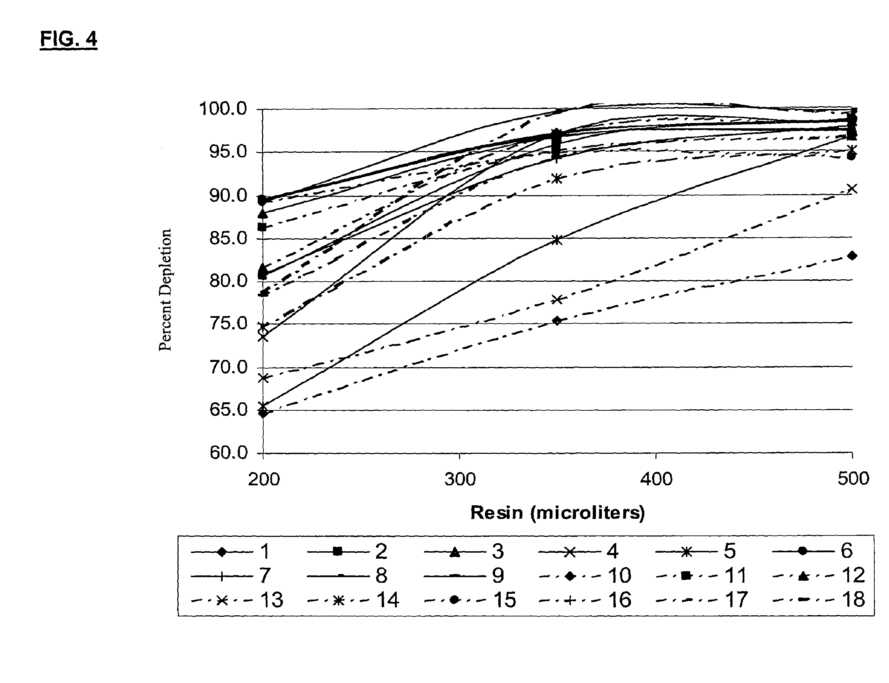

**Figure 4** is a plot illustrating the depletion of eighteen high abundance plasma proteins as a function of resin volume. A fixed volume of plasma (25 µl) was applied to various volumes of the antibody-conjugated resin (200, 350, and 500 µl). The percent depletion was determined using ELISA analyses. 1 = IgG, 2 = HSA, 3 = alpha-2-macroglobulin, 4 = IgM, 5 = IgA, 6 = fibrinogen, 7 = alpha-1-antitrypsin, 8 = transferrin, 9 = haptoglobin, 10 = alpha-1-acid glycoprotein, 11 = ceruloplasmin, 12 = IgD, 13 = ApoA1, 14 = complement C3, 15 = complement C1q, 16 = HDL, 17 = prealbumin, and 18 = ApoB.

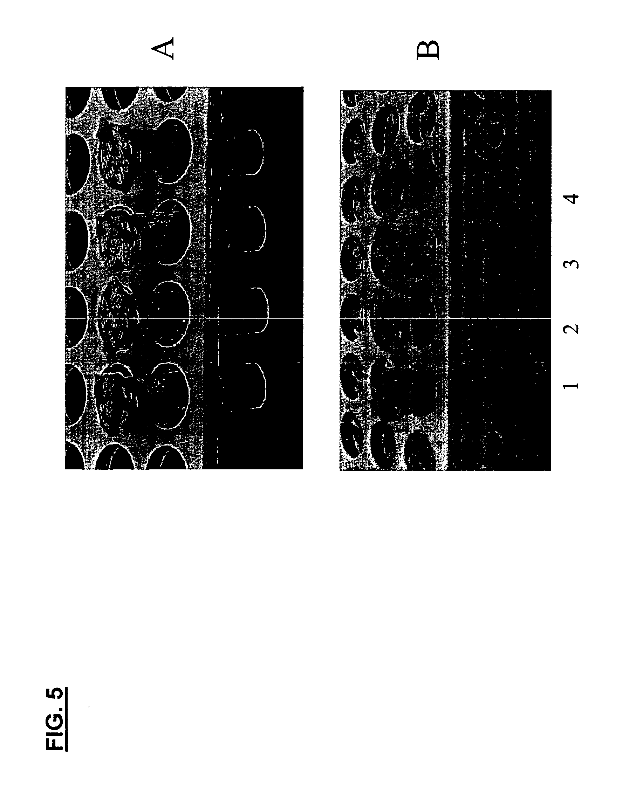

**Figure 5** is a photograph of blank resins exposed to various conjugation conditions (1-4) and incubated overnight at 37 °C. Panel A shows tubes containing the respective resins at the start of the experiment, and panel B presents the tubes after the incubation period. Condition 1 is resin that was bromoacetamide-activated using 3 mM bromoacetic acid-NHS ester. Condition 2 is resin that was maleimide-activated using 1 mM maleimide-NHS ester. Condition 3 is resin that was maleimide-activated using 3 mM maleimide-NHS ester. Condition 4 is resin that was maleimide-activated using 3 mM maleimide-NHS ester and conjugated to 20 antibodies.

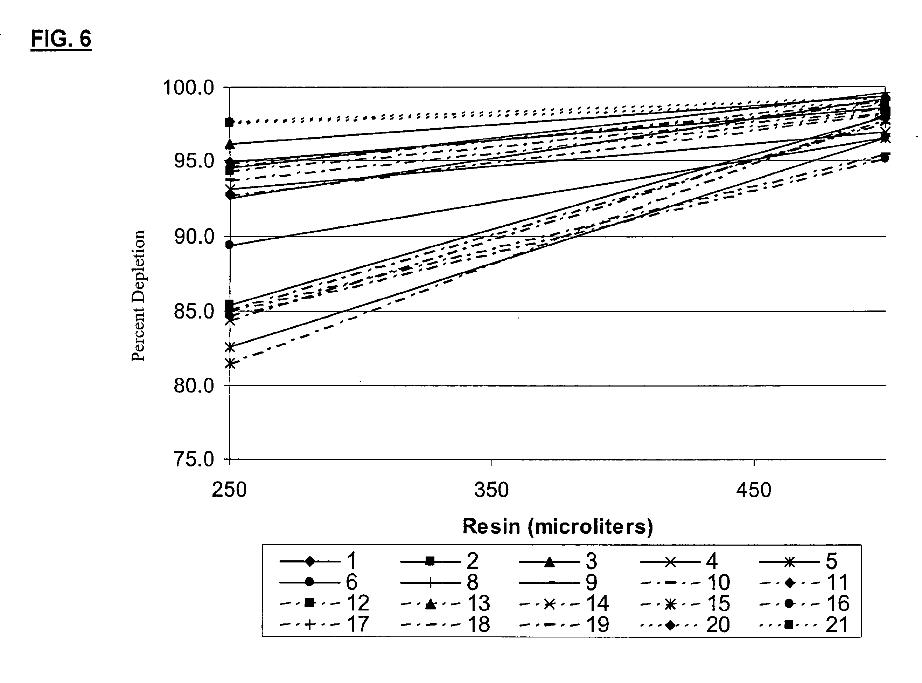

**Figure 6** is a plot illustrating the depletion of twenty high abundance plasma proteins as a function of resin volume. A fixed volume of plasma (25 µl) was applied to various volumes of the antibody-conjugated resin (250 and 500 µl). The percent depletion was determined using ELISA analyses. 1 = lgG, 2 = HSA, 3 = alpha-2-macroglobulin, 4 = lgM, 5 = lgA, 6 = fibrinogen, 7 = alpha-1-antitrypsin, 8 = transferrin, 9 = haptoglobin, 10 = alpha-1-acid glycoprotein, 11 = ceruloplasmin, 12 = IgD, 13 = ApoA1, 14 = complement C3, 15 = complement C1q, 16 = HDL, 17 = prealbumin, 18 = ApoB, 19 = plasminogen, and 20 = complement C4).

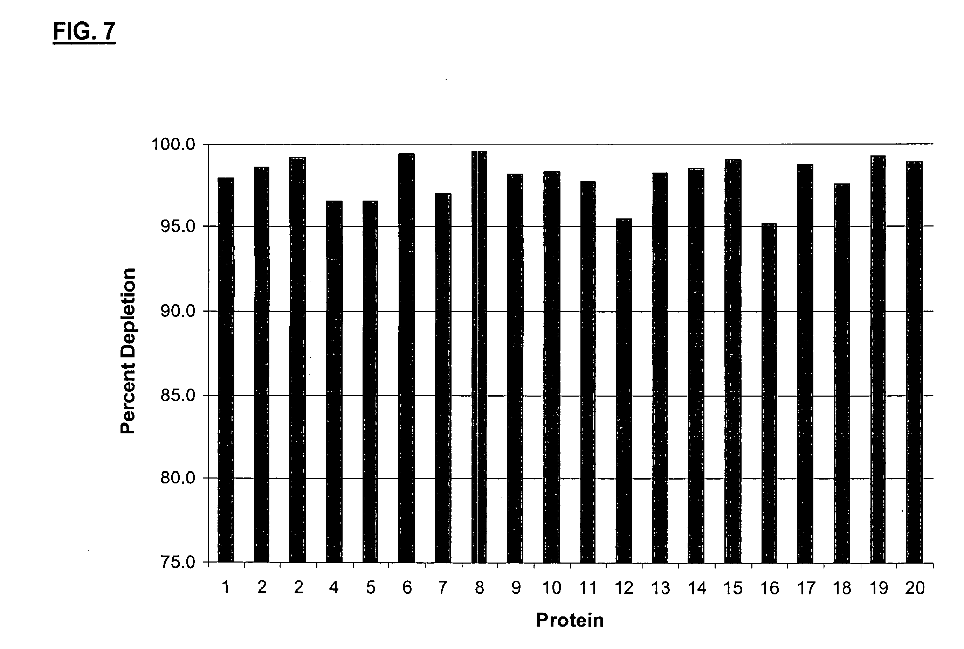

**Figure 7** is a bar graph depicting the depletion of twenty high abundance plasma proteins following application of 25 µl of plasma to 500 µl of maleimide-activated resin. The percent depletion was determined using ELISA analyses. 1 = HSA, 2 = IgG, 3 = transferrin, 4 = fibrinogen, 5 = IgA, 6 = alpha-2-macroglobulin, 7 = IgM, 8 = alpha-1-antitrypsin, 9 = complement C3, 10 = haptoglobin, 11 = ApoA1, 12 = ApoB, 13 = alpha-1-acid glycoprotein, 14 = ceruloplasmin, 15 = prealbumin, 16 = complement C1q, 17 = IgD, 18 = HDL, 19 = plasminogen, and 20 = complement C4.

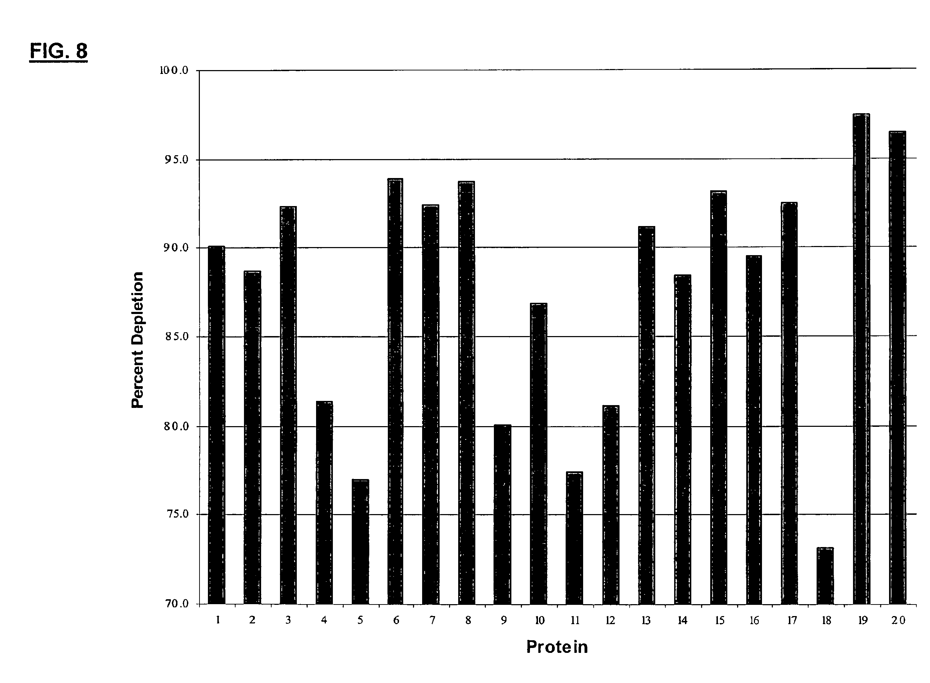

**Figure 8** is a bar graph illustrating the depletion of twenty high abundance plasma proteins following application of 25 µl of plasma to 500 µl of bromoacetamide-activated resin. The percent depletion was determined using ELISA analyses. 1 = HSA, 2 = IgG, 3 = transferrin, 4 = fibrinogen, 5 = IgA, 6 = alpha-2-macroglobulin, 7 = IgM, 8 = alpha-1-antitrypsin, 9 = complement C3, 10 = haptoglobin, 11 = ApoA1, 12 = ApoB, 13 = alpha-1-acid glycoprotein, 14 = ceruloplasmin, 15 = prealbumin, 16 = complement C1q, 17 = IgD, 18 = HDL, 19 = plasminogen, and 20 = complement C4.

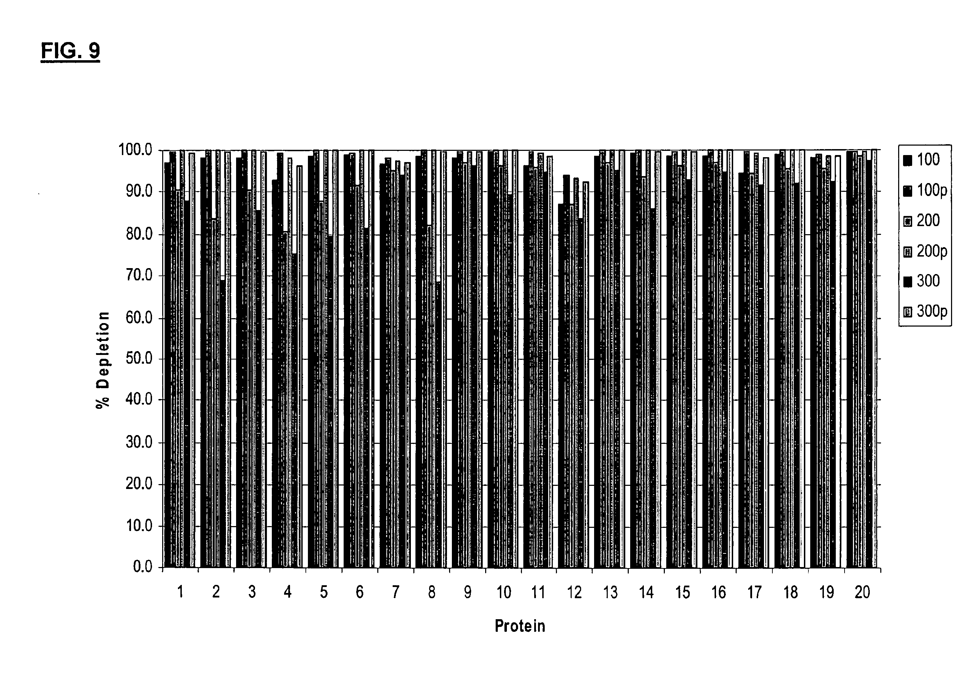

**Figure 9** is a bar graph depicting the depletion of twenty high abundance plasma proteins following application of 100, 200 or 300 µl of plasma to 3.75 ml of bromoacetamide-activated resin for one or two sequential cycles. The second cycle (p) represents a reapplication of the depleted plasma from the first cycle. The percent depletion was determined using ELISA analyses. 1 = HSA, 2 = IgG, 3 = transferrin, 4 = fibrinogen, 5 = IgA, 6 = alpha-2-macroglobulin, 7 = IgM, 8 = alpha-1-antitrypsin, 9 = haptoglobin, 10 = C3 complement, 11 = ApoA1, 12 = ApoB, 13 = alpha-1-acid glycoprotein, 14 = ceruloplasmin, 15 = prealbumin, 16 = IgD, 17 = ApoA2, 18 = plasminogen, 19 = complement C4 and 20 = complement C1q.

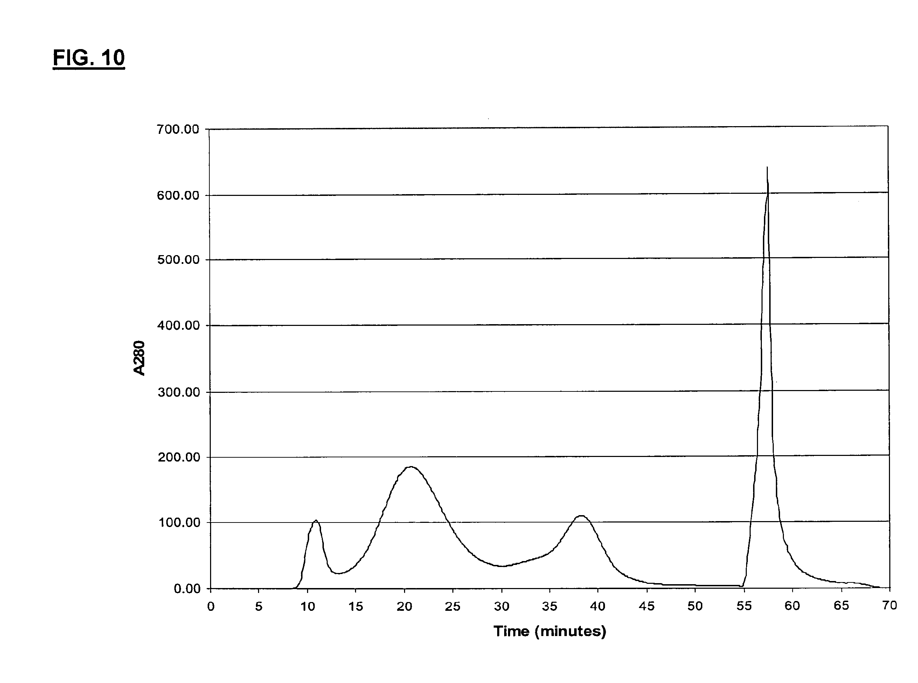

**Figure 10** is a plot of the absorbance at 280 nm versus time during immunodepletion column chromatography. Human citrated plasma (100 µl) was injected at 0.30 ml/min into a 10 ml column of immunoaffinity Sepharose 6B resin. Fractions (1 ml) of the flow-through (depleted plasma) were collected from t = 0 to t = 53 minutes. The 20 high abundance plasma proteins bound to the column resin were eluted with 0.1 M glycine-HCl, pH 2.5, and 0.1 % (w/v) octyl β-D-glucopyranoside at 3 ml/min from t = 53 minutes to t = 63 minutes. At t = 63 minutes, the column was reequilibrated at 3 ml/min.

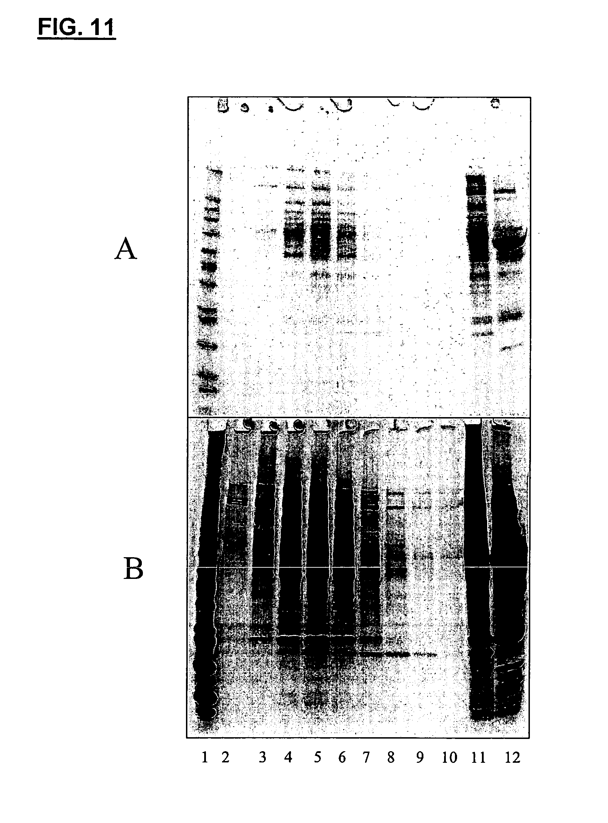

**Figure 11** presents photographic images of the depleted plasma fractions resolved by SDS-PAGE. Fractions were collected during immunodepletion chromatography to remove the 20 high abundance proteins (see Figure 10). Panel A shows a gel stained with Coomassie blue, and panel B shows a silver-stained gel. Lane 1 has protein molecular markers; lanes 2-10 contain fractions 4-12, respectively; lane 11 contains a pool of fractions 4-12; and lane 12 contains starting whole plasma.

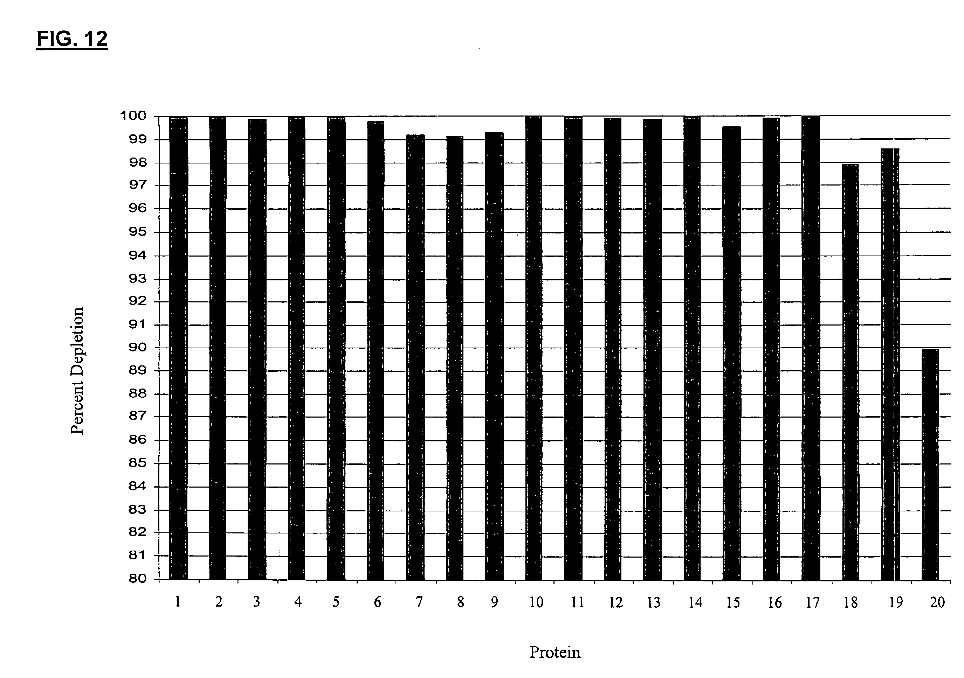

**Figure 12** is a plot illustrating the depletion of twenty high abundance proteins from 100 µl of plasma using a 10 ml immunoaffinity column. The percent depletion was determined using ELISA analyses. 1 = HSA, 2 = IgG, 3 = transferrin, 4 = IgA, 5 = alpha-1-antitrypsin, 6 = haptoglobin, 7 = fibrinogen, 8 = alpha-2-macroglobulin, 9 = IgM, 10 = C3 complement, 11 = alpha-1-acid glycoprotein, 12 = ceruloplasmin, 13 = prealbumin, 14 = IgD, 15 = plasminogen, 16 = complement C4, 17 = complement C1q, 18 = ApoB, 19 = ApoA2, and 20 = ApoA1.

### DETAILED DESCRIPTION OF THE INVENTION

The present invention provides compositions that may be utilized to separate a plurality of distinct targets from a sample having targets and non-targets. Generally speaking, the compositions of the invention are utilized to separate high abundance targets, and in particular, high abundance proteins, from a biological sample. The invention also provides a process for preparing the separation composition, methods for using the composition to separate distinct targets from a sample, methods for removing distinct targets and separate non-targets from a samples, and complete kits containing the separation composition.

### (I) Separation Compositions

A separation composition of the present invention generally comprises a plurality of heterogeneous epitope binding agents stochastically conjugated *en masse,* according to the process detailed in section (II) below, to a solid support. The epitope binding agents may each be conjugated directly to the solid support (e.g. via a CNBr coupling methodology). Alternatively, the epitope binding agents may each be conjugated to the solid support via one or more linkers. It is also possible, depending upon the embodiment, that some of the epitope binding agents may be conjugated directly to the solid support and some may be conjugated to the solid support via one or more linkers.

### (a) Epitope Binding Agents

A variety of individual epitope binding agents are suitable for use in the present invention to form a mixture having a plurality of heterogeneous epitope binding agents. Typically, individual epitope binding agents are selected so that the composition as a whole has affinity for a plurality of distinct targets. In this context, the phrase "heterogeneous" means that individual epitope binding agents within the plurality of epitope binding agents have affinity for different targets, thereby forming a separation composition that is capable of separating a multitude of distinct targets from a sample comprising a mixture of targets and non-targets. As will be appreciated by the skilled person, the number of distinct targets recognized by the separation composition of the invention can and will vary depending upon the binding affinities of the individual epitope binding agents comprising the heterogeneous epitope binding agent mixture. By way of non-limiting example, the epitope binding agents of the composition may bind from about 2 to about 1000 distinct targets. In another embodiment, the epitope binding agents of the composition may bind from about 2 to about 500 distinct targets. In a further embodiment, the epitope binding agents of the composition may bind from about 5 to about 100 distinct targets. In an additional embodiment, the epitope binding agents of the composition may bind from about 5 and to about 75 distinct targets. In still a further embodiment, the epitope binding agents of the composition may bind from about 2 to about 50 distinct targets. In an exemplary embodiment the epitope binding agents of the composition may bind about 20 targets. Non-limiting examples of targets are described below.

Epitope binding agents generally are selected so that the separation composition has binding affinity for desired targets. Examples of epitope binding agents suitable for use in the invention include, but are not limited to, small molecules, polynucleotides, and proteins or peptides.

In one embodiment, the epitope binding agent may be a small molecule. Small molecules may include metal ions, hormones, amino acids, cofactors, dyes, nucleic acids, and drugs. For example, metal ions typically utilized in immobilized metal affinity chromatography may be used in certain aspects of the invention. In this embodiment, the affinity of certain regions of a target for metal ions is used to separate the targets (e.g., the affinity of amino acid residues in a protein target for metal ions). Suitable metal ions include nickel (Ni²⁺), zinc (Zn²⁺), copper (Cu²⁺), iron (Fe³⁺), cobalt (Co²⁺), calcium (Ca²⁺), aluminum (Al³⁺), magnesium (Mg²⁺), manganese (Mn²⁺), or gallium (Ga³⁺). In another alternative embodiment, the metal ion is nickel, copper, cobalt, iron, or zinc. In still another alternative embodiment, the metal ion is nickel, iron, or copper.

In yet another embodiment, the epitope binding agent may be a polynucleotide, nucleotide, or oligonucleotide (e.g., oligo dT). Non-limiting examples of polynucleotide epitope binding agents include aptamers and double-stranded DNA. Aptamers may comprise DNA or RNA nucleotides. Additionally, aptamers may contain modified nucleotides, such as 2'-fluoro nucleotides, 2'-amino nucleotides, 5'-aminoallyl-2'-fluoro nucleotides, inosine, 2,6-diaminopurine, deoxyuridine, peptide nucleic acids (PNA), locked nucleic acids (LNA), and phosphorothioate nucleotides.

In still another embodiment, the epitope binding agent may be a protein or polypeptide. Suitable examples of protein epitope binding agents include, but are not limited to, receptors and fragments of receptors, ligands and fragments of ligands, peptides, coenzymes, coregulators, DNA-binding proteins, scaffolding proteins, Protein A, and Protein G.

In an exemplary embodiment, the epitope binding agent is an antibody mixture. In particular embodiments of the invention, the composition comprises a plurality of heterogeneous antibodies. Suitable antibodies include, but are not limited to, immunoglobulins, i.e., lgG, lgA, lgM, lgD, lgE, lgY, monoclonal antibodies, polyclonal antibodies, recombinant antibodies, single chain antibodies, antibody fragments and peptides. In certain embodiments of the invention, the heterogeneous antibody mixture comprises monoclonal antibodies. Suitable monoclonal antibodies include chimeric antibodies and humanized antibodies. In another embodiment of the invention, the heterogeneous antibody mixture comprises polyclonal antibodies. In other embodiments, the heterogeneous antibody mixture comprises recombinant antibodies. Suitable recombinant antibodies include chimeric antibodies, humanized antibodies, recombinant antibody fragments, i.e., Fv fragments, single chain Fv fragments, disulfide stabilized Fv (dsFv) fragments, single domain antigen binding fragments, i.e., V_{H}S, recombinant Ilama heavy chain antibody fragments, camelized antibodies, and antibody fusion proteins. In other embodiments, the heterogeneous antibody mixture comprises single chain antibodies. Examples of single chain antibodies include single chain Fv fragments and single domain antigen binding fragments, i.e., V_{H}S. In further embodiments of the invention, the heterogeneous antibody mixture comprises antibody fragments. Suitable antibody fragments include, but are not limited to, single chain antibody fragments, i.e., single chain Fv fragments, recombinant Ilama heavy chain antibody fragments, recombinant antibody fragments, i.e., Fv fragments, disulfide stabilized Fv (dsFv) fragments, single domain antigen binding fragments, i.e., V_{H}S, Fc fragments, and Fab fragments. Antibodies utilized in the invention may be either purchased commercially or made in accordance with methods generally known in the art. For example, antibodies, methods of generating antibodies, including suitable expression systems for the large scale production of antibody fragments, and applications of antibodies are discussed in "The Production of Antibody Fragments and Antibody Fusion Proteins by Yeasts and Filamentous Fungi," Joosten, et al., Microbial Cell Factories, 2003, 2:1.

As will be appreciated by the skilled person, in certain embodiments it may be desirable to have a diverse mixture of molecules that comprise the plurality of epitope binding agents. For example, the heterogeneous epitope binding mixture may comprise protein and polynucleotide epitope binding agents. Alternatively, in other embodiments, it may be desirable to have the same type of molecule comprising the plurality of epitope binding agents. For example, the heterogeneous epitope binding agent mixture may comprise protein epitope binding agents. In an additional embodiment, the heterogeneous epitope binding mixture may comprise antibody and antibody fragment epitope binding agents. In a further embodiment, the epitope binding agents comprise immunoglobulin, monoclonal antibody, polyclonal antibody, recombinant antibody, single chain antibody, an antibody fragment, or a combination thereof. In yet a further embodiment of the invention, the epitope binding agents may comprise IgG, IgY, and single chain antibodies. In still a further embodiment, epitope binding agents comprise IgG, IgY, and recombinant Ilama heavy chain antibody fragments.

### (b) Solid Support

To facilitate the separation of targets from non-targets, the epitope binding agents are stochastically conjugated to a solid support either directly or via one or more linkers. Depending upon the particular embodiment, the epitope binding agents may be conjugated to the solid support by a variety of chemical bonds, including but not limited to, covalent bonding, dative bonding, ionic bonding, hydrogen bonding, or Van der Waals bonding. In an exemplary embodiment, the conjugation is via covalent bonding.

A variety of materials may be utilized as a solid support in the present invention to the extent the material is capable of being derivatized to allow for conjugation to the epitope binding agents. The solid support also typically does not react with either the epitope binding agents or the sample. Suitable examples of materials that may be utilized as solid supports in the present invention include, agarose, cellulose, methacrylate co-polymers, polystyrene, polypropylene, paper, polyacrylic, polyacrylamide, polyamide, polyacrylonitrile, polyvinylidene, polysulfone, nitrocellulose, polyester, polyethylene, silica, glass, latex, plastic, gold, iron oxide polyacrylamide, solid supports coated with 3-dimensional high capacity dextran coatings, or combinations of the above materials. The solid support may be a variety of sizes and forms depending upon the embodiment of the invention. For example, the solid support may be resins, beads, microarrays, microtiter wells, emulsions, glass supports, silica supports, magnetic supports, powders, polymer supports, copolymer supports, nano-capillaries, or other nano-materials

In one embodiment of the invention, the solid support comprises a resin. Suitable resins may be comprised of dextrans, styrenes, agarose, (e.g. Sepharose), calcium phosphates, acrylics, polyamines, acrylamides, polyacrylamindes, polyacrylics, silicas, cellulose, synthetic polymers, copolymers, or a combination thereof. In a further embodiment, the solid support comprises a silica resin. In a preferred embodiment of the invention, the solid support comprises an agarose resin.

In an additional embodiment of the invention, the solid support is comprised of beads. The beads may vary in size. For example, the beads may be nanobeads or microbeads. Non-limiting examples of suitable beads include magnetic beads, magnetically responsive beads (paramagnetic beads), polystyrene beads, agarose beads, polyacrylamide beads, polyacrylic beads, silica beads, and beads comprised of copolymers, such as a polyacrylamide/azlactone copolymer. In a preferred embodiment, the solid support comprises agarose (Sepharose) beads.

In yet another embodiment, the solid support comprises a reservoir, for instance, a micro-titer well, a cuvet, or a test tube. Non-limiting examples of micro-titer wells include 6, 12, 24, 32, 48, 96, 384 and 1536 well plates. Non-limiting examples of test tubes include polystyrene, polypropylene, borosilicate, and flint glass tubes. Additionally, reservoirs may be arranged or orientated to allow for automation.

In certain embodiments of the invention, the solid support comprises a magnetic support. Suitable magnetic supports (including magnetically responsive supports) include magnetic beads, magnetic stir bars or rods, or other magnetic objects.

In an alternative embodiment of the invention, the solid support comprises a membrane. Non-limiting examples of membranes include nitrocellulose, and polyvinylidene difluoride membranes.

In further embodiments of the invention, the solid support is a microarray support. In yet a further aspect, the solid support is an emulsion. The emulsion may comprise liposomes or micelles. The size of the emulsion particles may vary.

As will be appreciated by a skilled person, the density of epitope binding agents conjugated to the solid support can and will vary depending upon the type of solid support and the particular material from which the solid support is made. Generally speaking, the epitope binding agents are stochastically conjugated to the solid support, either directly or via one or more linkers, at a density of about 3 mg/ml to about 30 mg/ml. In a further embodiment, the epitope binding agents are stochastically conjugated to the solid support at a density of about 4 mg/ml to about 25 mg/ml. In a further embodiment, the epitope binding agents are stochastically conjugated to the solid support at a density of about 5 mg/ml to about 20 mg/ml. Stated another way, the separation composition of the invention typically has a target binding capacity from about 0.1 mg/ml to about 30 mg/ml. In one embodiment, the target binding capacity is from about 0.5 mg/ml to about 25 mg/ml. In another embodiment, the target binding capacity is from about 5 mg/ml to about 20 mg/ml.

### (c) Linkers

As detailed above, in certain embodiments of the invention, the epitope binding agents may be stochastically conjugated to the solid support by one or more linkers, either of the same type or of different types. Typically, one or more linkers is disposed between an epitope binding agent and the solid support, and couples the epitope binding agent to the solid support. The linker will generally be conjugated to the solid support by strong, non charged bonds selected from ether, thioether, amide, and carbon to carbon. Processes for stochastically conjugating the epitope binding agents *en masse* to the solid support are detailed in section (II).

Generally speaking, the chain of atoms defining the linker can and will vary depending upon the embodiment. Typically, the linker will be of a sufficient length to present the epitope binding agents for efficient binding interactions with targets. If the linker is too short, then steric constraints may materialize; however, if the linker is too long, unfavorable interactions between linkers comprising the separation composition may occur. In a typical embodiment the linker is from about 5 to about 50 atoms in length. Alternatively, the linker is from about 5 to about 30 atoms in length. In an exemplary embodiment, the linker is from about 10 to about 20 atoms in length.

The linker may comprise a variety of heteroatoms that may be saturated or unsaturated, substituted or unsubstituted, linear or cyclic, or straight or branched. The chain of atoms defining the linker will typically be selected from the group consisting of carbon, oxygen, nitrogen, sulfur, selenium, silicon and phosphorous. In an alternative embodiment, the chain of atoms is selected from the group consisting of carbon, oxygen, nitrogen, sulfur and selenium. In an exemplary embodiment, the linker will comprise substantially carbon and oxygen atoms. In addition, the chain of atoms defining the linker may be substituted or unsubstituted with atoms other than hydrogen, including, but not limited to, hydroxy, keto (=O), or acyl, such as acetyl. Thus, the chain may optionally include one or more ether, thioether, selenoether, amide, or amine linkages between hydrocarbyl or substituted hydrocarbyl regions.

In an exemplary embodiment, the linker will comprise from about 10 to 20 carbon or oxygen atoms, will be substantially uncharged along its entire length, will be substantially hydrophilic, and will impart stability to the separation composition of the invention. In this context, strong covalent bonds within the linker, and in particular, at the point of conjugation, generally provide stability to the separation composition under a variety of conditions, including but not limited to, loading, washing, elution, and regeneration. In certain embodiments, as detailed in section (II), more than one linker may be used to conjugate each individual epitope binding agent to the solid support. Because of the multiple points of attachment, this feature provides further stability to the separation composition of the invention.

### (II) Process for Preparing Separation Composition

A further aspect of the invention encompasses a process for preparing separation compositions of the invention. In general, the process involves combining a plurality of heterogeneous epitope binding agents to form an epitope binding agent mixture. The epitope binding agent mixture is contacted with a solid support under conditions such that the plurality of epitope binding agents are stochastically conjugated *en masse* to the solid support. In certain embodiments, the epitope binding agents may be conjugated directly to the solid support. In other embodiments, the epitope binding agents may be conjugated to the solid support by one or more linkers. In still another embodiment, some of the epitope binding agents may be conjugated directly to the solid support and some of the epitope binding agents may be conjugated via one or more linkers.

### (a) Conjugation Directly to the Solid Support

The epitope binding agents may be conjugated directly to the solid support by a variety of suitable methods known in the art. In this context, "directly" means that the epitope binding agents are not conjugated to the solid support by one or more linkers, as described below. Methods for direct conjugation are suitable for any particular embodiment to the extent the separation composition has the desired binding capacity for distinct targets.

Generally speaking, the solid support typically is derivatized in a manner that changes its chemical composition to facilitate binding between the epitope binding agent and the solid support. The epitope binding agents may be activated so that they covalently bind to the derivatized solid support. Activation of the epitope binding agents is described in more detail below. As will be appreciated by a skilled person, the manner by which the solid support is derivatized can and will vary depending upon the embodiment and the material from which the solid support is made. The solid support may be, for example, coated with a substance or with a polymer containing a substance that reacts under appropriate conditions to form a covalent bond between the solid support and the epitope binding agents. By way of a non-limiting example, the substance may be a reactive group, such as a photo-reactive group, that when contacted with light may become activated, and capable of covalently attaching to the surface of a solid substrate. Exemplary reactive groups include, but are not limited to, reactive groups typically used in the preparation of chromatography media which include: epoxides, oxiranes, esters of N-hydroxysuccinimide, aldehydes, hydrazines, maleimides, mercaptans, amino groups, alkylhalides, isothiocyanates, carbodiimides, diazo compounds, tresyl chloride, tosyl chloride, and trichloro S-triazine. Exemplary photo-reactive groups include aryl azides, diazarenes, beta-carbonyldiazo, and benzophenones. The reactive species are nitrenes, carbenes, and radicals. These reactive species are generally capable of covalent bond formation.

Alternatively, the surface of the solid support may be coated with a polymer containing a substance that reacts under appropriate conditions to form a covalent bond between the solid support and the epitope binding agents. Suitable reactive substances include any of the reactive groups detailed above. In this embodiment, the reactive groups are activated to covalently bind at least some of the polymer molecules directly to the solid support and to induce cross-linking between polymer molecules to form a polymer matrix attached to the solid support. Suitable polymers may comprise natural polymers (or a derivative thereof), synthetic polymers (or a derivative thereof), a blend of natural polymers (or derivative(s) thereof), a blend of synthetic polymers (or derivative(s) thereof), or a blend of one or more natural polymers (or derivative(s) thereof) and one or more synthetic polymers (or derivative(s) thereof). Exemplary polymers include, but are not limited to, cellulose-based products such as hydroxyethyl cellulose, hydroxypropyl cellulose, carboxymethyl cellulose, cellulose acetate, and cellulose butyrate; acrylics such as those polymerized from hydroxyethyl acrylate, hydroxyethyl methacrylate, glyceryl acrylate, glyceryl methacrylate, acrylic acid, methacrylic acid, acrylamide, and methacrylamide; vinyls such as polyvinyl pyrrolidone and polyvinyl alcohol; nylons such as polycaprolactam, polylauryl lactam, polyhexamethylene adipamide, and polyhexamethylene dodecanediamide; polyurethanes; polylactic acids; linear polysaccharides such as amylose, dextran, chitosan, heparin, and hyaluronic acid; and branched polysaccharides such as amylopectin, hyaluronic acid and hemicelluloses. Whether natural or synthetic, the polymer may be derivatized or modified by oxidation, or by the covalent attachment of photo-reactive groups (as described above), affinity ligands, ion exchange ligands, hydrophobic ligands, or other natural or synthetic polymers.

The solid support may be coated by contacting the polymer composition or the reactive substance with the surface of the solid support. The method used to contact the polymer composition with the solid support depends on the dimensions and shape of the surface to be coated. When coating a multiwell plate, tube or a surface or a portion thereof, larger than 0.1 mm square, the polymer composition may be contacted with the solid support surface by pouring, spraying, micropipeting, or transferring the polymer composition onto the portions of the container or plate, e.g., wells, to be coated. In the alternative, the portion of the plate, tube, container surface, or support larger than 2 mm square to be coated may also be coated by dipping the portion of the surface into a solution of the polymer composition so as to place the solid support in contact with the polymer composition.

Once the polymer composition is placed in contact with the surface of the solid support, the polymer composition may be dried on the surface prior to activating the reactive groups, for example, evaporated to dryness by incubation in the dark at 20-50 °C with air flow. The polymer composition can also be evaporated, lyophilized or any other drying means, including air drying, to remove the solvent. A variety of drying methods may be used, provided there is no premature activation of the reactive groups in response to the drying step. The solid support is considered sufficiently dry when no moisture is visibly detectable.

The dried coated solid surface is then treated to induce the reactive groups to covalently bond to the substrate and to the activated epitope binding agents as well. By way of a non-limiting example, in the case of the photo-reactive groups, they may be activated by irradiation. Activation is the application of an external stimulus that causes reactive groups to bind the epitope binding agents to the solid support. Specifically, a covalent bond is formed between the substrate and the reactive group, e.g., carbon-carbon bond formation. For example, in the case of benzophenone the photo-reactive groups may be activated by exposure to UV light from about 3 Joules/cm² to about 6 Joules/cm² depending on the intensity of light and duration of exposure time. The exposure times may range from as low as 0.5 sec/cm² to approximately 32 min/cm² depending on the intensity of the light source.

### (b) Conjugation to the Solid Support Via One or More Linkers

Alternatively, the epitope binding agents may be conjugated to the solid support via one or more linkers. Suitable linkers are defined by the chain of atoms described in section (I). It is envisioned that the epitope binding agents may be conjugated to the solid support in a variety of suitable manners. In certain embodiments, the linker may be bound directly to the solid support at one end and bound to the epitope binding agents at the other end, thereby conjugating the epitope binding agents to the solid support. In the alternative, the solid support may be coated with a substance, such as a polymer composition, and one end of the linker may be bound to the polymer and the other end of the linker may be bond to the epitope binding agent, thereby conjugating the epitope binding agents to the solid support. Irrespective of the manner of conjugation, the solid support and epitope binding agents may be chemically modified with any of a variety of suitable reactants so as to facilitate stochastic coupling of the epitope binding agents to the solid support via one or more linkers.

### (1) Solid supports derivatized with chemical moieties

The solid support is typically derivatized with a chemical moiety that, when bound to the activated epitope binding agents, forms the linker, or portion thereof. A variety of chemical moieties may be suitable for use in the invention to the extent they stochastically conjugate the epitope binding agents *en masse* to the solid support via a linker that is substantially hydrophilic and that is substantially uncharged along its entire length. Additional suitable properties of the separation composition having a linker are detailed in section (I).

In an exemplary embodiment for derivatizing the solid support with a chemical moiety, as illustrated in the examples, the solid support is reacted with reagents under appropriate conditions to form chains of atoms that are substantially hydrophilic and are attached to the solid support by strong bonds. Non-limiting examples of strong, non-charged bonds include ether, thioether, amide, and carbon-carbon bonds. The number of atoms in the hydrophilic chain is typically from about 5 to about 50 atoms. Any of a variety of reagents or combination of reagents may be utilized to form the substantially hydrophilic arm. In one embodiment, the solid support is reacted with a reagent that forms a strong, non-charged ether linkage with hydroxyl groups. In another embodiment, the reagent is an epoxy activating reagent. In yet another embodiment, the reagent is a halogen containing reagent. A suitable example of a halogen containing reagent is epichlorohydrin. In an exemplary embodiment, the reagent is 1,4-butanediol diglycidyl ether. The reaction may be performed in accordance with the reaction parameters detailed in the examples. The successful addition of the substantially hydrophilic arm can be determined by assays known in the art.

After formation of the substantially hydrophilic arm on the solid support, the solid support is contacted with one or more reagents that add a nucleophilic group to the free end of the substantially hydrophilic arm. In one embodiment, the nucleophilic group is an amine group. In an exemplary embodiment, the amine is a primary amine. In an exemplary embodiment, the primary amine is added to the free end of the substantially hydrophilic arm by mixing the solid support with ammonium hydroxide. The reaction may be performed in accordance with the reaction parameters detailed in the examples. The successful addition of the nucleophilic group to the end of the substantially hydrophilic arm, such as an amine group, may be qualitatively evaluated by assays known in the art, for example, the Tri Nitro Benzene Sulfonic (TNBS) assay or more quantitatively by ninhydrin assay (see the examples).

The solid support having a substantially hydrophilic arm disposed with a nucleophilic group at its end, may be contacted with one or more additional reagents to form a chemical moiety on the solid support that is capable of binding to the activated epitope binding agents. It will be appreciated by the skilled person, that the selection of particular reagents to form the chemical moiety will greatly depend upon the manner in which the epitope binding agents will be attached. In one exemplary embodiment, the solid support is reacted with one or more reagents to form a chemical moiety capable of binding sulfhydryl groups. In another exemplary embodiment, the solid support is reacted with one or more agents that form an alkyl chain thioether upon reaction with sulfhydryl. In a further exemplary embodiment, the solid support is reacted with one or more reagents that forms a maleimide-bonded thioether upon reaction with a sulfhydryl. A variety of reagents are suitable to form a chemical moiety capable of binding sulfhydryl groups on epitope binding agents. In one embodiment, the reagent is bromoacetic acid NHS ester. In another embodiment, the reagent is maleimidobutyric acid NHS ester. In yet another embodiment, the reagent is iodoacetic acid NHS ester. In yet a further embodiment, the reagent is maleimidocaproic acid NHS ester. The reaction may be performed in accordance with the reaction parameters detailed in the examples.

In an exemplary embodiment, the chemical moiety is added to the solid support by a three-step process. In the first step, the solid support is contacted with a first reagent under conditions such that about 5 to about 50 atoms are added to the solid support to form a solid support having a substantially hydrophilic arm. In the second step, the solid support having a substantially hydrophilic arm is contacted with a second reagent that adds an amine group to the end of the arm. Finally, the product of the second step is contacted with a third reagent that forms via amide bond the chemical moiety on the solid support that is capable of binding to sulfhydryl groups. In this particular embodiment, the first reagent is 1,4-butanediol diglycidyl ether, the second reagent is ammonium hydroxide, and the third reagent is selected from bromoacetic acid NHS ester or maleimide spacer NHS ester.

Following the addition of the chemical moiety to the solid support, the solid support is washed and is ready to be contacted with the activated epitope binding agents.

### (2) Activating the Epitope Binding Agents

The epitope binding agents may be activated by a variety of suitable methods so that they will be bound by one or more chemical moieties derivatized on the solid support.

In an exemplary embodiment, the epitope binding agents comprise a heterogeneous mixture of antibodies that have been reacted with one or more reagents that stochastically adds multiple, extrinsic sulfur-containing groups to the antibodies. In one embodiment, the sulfur-containing groups are sulfhydryl groups. In a further embodiment, the added extrinsic sulfhydryl groups are protected, (and remain protected by the inclusion of EDTA and / or utilizing low pH) such that they are not readily available for disulfide bond formation.

In one embodiment, the sulfhydryl groups are added to the antibodies by reaction with S acetyl mercapto succinic anhydride (SAMSA). In yet another embodiment, the sulfhydryl groups are added to the antibodies by reaction with iminothiolane. In an exemplary embodiment, the random extrinsic sulfhydryl groups are added to the antibody by reaction with S-acetylthioacetic acid succinimide (SATA). Generally speaking, SATA reacts with amine groups on a protein. In one embodiment, the SATA is reacted with the antibodies such that between 0 and about 75 percent of the amines remain unreacted. In another embodiment, the SATA is reacted with the antibodies such that between about 15 and about 70 percent of the amines remain unreacted. In yet another embodiment, the SATA is reacted with the antibodies such that between about 25 and about 70 percent of the amines remain unreacted.

One skilled in the art will appreciate that the ideal reaction conditions for adding sulfur-containing groups to antibodies will depend on the type of antibody used. In general, between about 1.0 mM and about 8.0 mM SATA is used to add sulfhydryl groups to antibodies. For more detailed methods, see the examples.

### (3) Coniugation of the Activated Antibodies to the Solid Support

The activated antibodies, as described above, are typically coupled to the chemically reactive moiety on the solid support, through a linker. This coupling provides stochastic conjugation of the antibodies *en masse* to the solid support by formation of one or more linkers. In one embodiment, the chemical moiety of the solid support and a sulfur-containing group on the antibodies form a covalent bond. In another embodiment, the bond between the chemical moiety and the sulfur-containing group is strong, and non-charged. In yet another embodiment, the sulfur-containing group forms a thioether bond with the chemical moiety. In yet still another embodiment, the sulfur-containing group is a protected sulfhydryl. One skilled in the art will appreciate that the method of coupling the antibodies to the solid support via the chemical moiety will depend in part on the constitution of the chemical moiety. More detailed methods of coupling antibodies to the chemical moiety of the solid support are illustrated in the examples.

During preparation of the affinity matrix not all of the functional groups introduced onto the linker and the epitope binding ligands will have reacted. Specifically as described in the exemplary embodiment, following the incorporation of the sulfhydryl reactive moieties onto the amine terminated linker, there may be unreacted free amines. As will be appreciated by a skilled person, depending upon the embodiment, it may be desirable to neutralize these unreacted groups. Neutralization of unreacted groups may minimize the possibility of non-specific interactions between the targets or non-targets and the separation composition of the invention. In one embodiment, the unreacted groups may be chemically neutralized. In another embodiment, the unreacted groups may be neutralized by acetylation. In still a further embodiment, the unreacted groups may be neutralized with acetic anhydride. In addition, following the conjugation reaction between the activated antibodies and the chemical moieties on the solid support not all of the active groups disposed on the end of the chemical moieties will have reacted. Stated another way, after the conjugation reaction some chemical moieties will have unreacted groups such as sulfhydryl and bromoacetyl. Again as will be appreciated by the skilled person it may be desirable to block these unreacted groups. Blocking of these unreacted groups will prevent undesirable bonding reactions. In one embodiment, the unreacted groups may be chemically blocked. In a further embodiment the unreacted sulfhydryl groups may be blocked by reaction with iodoacetamide. In another embodiment the unreacted sulfydryl reactive moieties, (e.g. bromoacetyl) may be blocked by reaction with a strong nucleophile such as hydroxylamine.

### (c) Iterative Selection

A further feature of the invention provides methods for iteratively selecting the relative concentration of each type of epitope binding agent present in the separation composition. The concentration of each epitope binding agent conjugated to the solid support may be optimized based on the relative molar ratio of each respective target to be separated from the sample. The concentration is typically determined such that the desired level of separation is achieved for each target. Instead of performing experiments to determine the desired concentration of epitope binding agent for every target individually, the process of the present invention allows for determining the optimal concentration of each epitope binding agent simultaneously. This simultaneous iterative determination greatly reduces the time necessary to develop an ideal concentration for each epitope binding agent.

The concentration of an epitope binding agent may be iteratively determined by depleting a fixed target volume of plasma (e.g., 25 µl) utilizing various volumes of resin. The resin volumes chosen typically involve a target resin volume (e.g., 500 µl) and at least one larger (e.g., 750 µl) and one smaller volume (e.g. 250 µl) of resin. If sufficient depletion with the larger volume (e.g., 750 µl) of resin is observed but not with the target resin volume (e.g., 500 µl), the amount of that particular antibody would be increased in the subsequent batch of resin by the percent difference between the resin volumes (e.g., in this case 50%). If sufficient depletion with the larger volume (e.g., 750 µl) and target resin volume (e.g., 500 µl) is observed but not with the smaller volume (e.g., 250 µl), the amount of that particular antibody would not need to be adjusted. If sufficient depletion with all 3 volumes of resin including the smaller volume (e.g., 250 µl) is observed, the amount of that particular antibody could be decreased in the subsequent batch of resin by the percent difference between the resin volumes (e.g., 50%). It must be noted that without a resin volume lower than the target, it would be impossible to know if the resin contains more antibody than is necessary. It is important that each antibody be optimized. Adding more of one antibody than is necessary may take up valuable sites on the resin, which may be needed by other antibodies. The initial selection of a concentration may be based on the typical relative molar ratio of a respective target in the sample mixture. Methods of performing separations of targets and non-targets are discussed in detail below. The level of separation between targets and non-targets may be determined by assays known in the art, for instance, ELISA analysis (see Examples 1 through 5).

Alternatively, if after a separation of a fixed volume of plasma with only one volume of resin it is determined that the level of target separation was ideal, the concentration of the corresponding epitope binding agent may be decreased. If, after decreasing the concentration, the level of target separation is still ideal, the iterative selection has enabled a more efficient usage of the epitope binding agent. If, after decreasing the concentration, the level of target separation is no longer ideal, the concentration can be subsequently increased. It is clear to one skilled in the art that this process of optimization of the epitope binding agent would be much more time consuming than the one described above.

Additional detailed examples of separating targets from non-targets, determining the level of separation, and adjusting the concentration of each epitope binding agent can be found in the examples.

### (III) Methods for Separating Targets from Non-targets

The present invention encompasses a method for separating at least two distinct targets from a sample comprising a mixture of targets and non-targets. In an exemplary embodiment, as detailed below, the method may be utilized to separate from about 5 to about 100 distinct targets. The distinct targets are typically molecules present at the highest concentration within the sample. Generally speaking, the method comprises contacting the sample with a separation composition of the invention comprising a plurality of heterogeneous epitope binding agents stochastically conjugated *en masse* to a solid support. The epitope binding agents have affinity for the targets, such that when they contact the targets, they bind the targets for a sufficient length of time to allow separation of the targets from the non-targets. The method may further comprise fractionating the non-targets that do not bind the epitope binding agents. The separation composition and the process for preparing the separation composition are described in sections (I) and (II), respectively.

### (a) Samples

One aspect of the method of the invention is collection of a sample derived from a suitable source. Targets from samples derived from a variety of different sources may be separated by the methods of the invention. For example, the sample may be from an animal, plant, microorganism, or from the environment. In an embodiment, the sample is from an animal. Non-limiting examples of animals include mammals, amphibians, reptiles, birds, insects, and fish. In a further embodiment the sample is from a mammal. In yet a further embodiment, the sample is derived from a source selected from the group comprising a human, mouse, rat, hamster, primate, bovine, Drosophila, zebrafish, chicken, Arabidopsis, rice, Dictyostelium, yeast, feline, and canine sample. Examples of primates include *Homo sapiens,* apes and monkeys. Examples of canines include domestic dogs, foxes, and wolves. In an exemplary embodiment, the sample is derived from a human. In an alternative embodiment, the sample is from the environment. Non-limiting examples of environmental samples include water samples, soil samples, and air samples.

As will be appreciated by the skilled person, the type of sample collected from the source can and will vary depending upon the embodiment. For example, suitable types of samples include organ samples, tissue samples, cell samples, microorganism samples, (i.e., bacteria samples, yeast samples), and derivatives thereof, such as tissue or cell lysates, cell extracts, and microorganism lysates. Suitable types of samples also include subcellular fractions, cytoplasm, cell culture supernatants, environmental samples, and bodily fluid. A variety of bodily fluids may be separated according to the present invention, including; plasma; serum; whole blood; cerebrospinal fluid (CSF); synovial fluid; tissue fluid; organ fluid, such as bile, semen and the like; humors, such as vitreous humor; lavage fluids, such as nasal lavage fluid and bronchoalveolar lavage fluid; secretions, such as mucinous fluids, nipple aspirates, exudates, saliva, perspiration and tears; waste products, such as urine and feces; and other biological fluids in the case of non-human animals. Methods for collecting lysates, extracts, or fluid samples are well known in the art. In one embodiment, the sample is selected from the group comprising whole blood, serum, and plasma. In an additional embodiment, the sample is whole blood. In another embodiment, the sample is serum. In yet another embodiment, the sample is plasma. In a further embodiment, the sample is *human* serum. In an exemplary embodiment, the sample is *human* plasma.

In another embodiment, the sample is from a plant. With regard specifically to plant and microorganism samples, various fluids, tissue extracts, and cell extracts may be sampled. For example, plant tissue, such as leaf tissue, can be macerated in a suitable buffer to yield a suitable extract, or a natural plant fluid, such as sap, may be used. Note that samples may be prefractionated or otherwise treated before application to the solid support. Such upstream treatment prior to separation / depletion on the solid support may include but is not necessarily limited to ion exchange or reverse phase liquid chromatography, size exclusion chromatography, isolectric focusing, 1 or 2 dimensional electrophoresis, etc. Procedures generally known in the art or detailed herein may be utilized to collect samples in accordance with the method of the invention.

### (b) Targets

After the sample of interest has been collected from the selected source, the method of the present invention may be used to separate a variety of targets from non-targets in the sample. Typically, at least two distinct targets are separated. In one embodiment, from about 2 to about 1000 different targets are separated. In an alternative embodiment, from about 5 to about 100 distinct targets are separated. The targets may be the same type of molecule or different types of molecules. For example, the targets may include, but are not limited to, analytes, macromolecular complexes, microorganisms, prions, organelles or subcellular components, cells, contaminants, and biomolecules. It is envisioned that the targets will depend, in part, on the source of the sample. In one embodiment, at least one target is an analyte, including interferents. In another embodiment, at least one target is a macromolecular complex. In a further embodiment, the macromolecular complex comprises at least one protein. In yet a further embodiment, at least one target is a macromolecular complex that comprises at least one carbohydrate. In still a further embodiment, at least one target is a macromolecular complex that comprises at least one lipid. In yet a further embodiment, at least one target is a macromolecular complex that comprises at least one nucleic acid.

In an additional embodiment, at least one target is a microorganism. Non-limiting examples of microorganisms include archaebacteria, bacteria, viruses, protists, and fungi. In one embodiment, at least one target is an archaebacteria. In another embodiment, at least one target is a bacterium. In yet another embodiment, at least one target is a virus. In still another embodiment, at least one target is a protist. In still yet another embodiment, at least one target is a fungi. Alternatively, at least one target is a prion.

In another embodiment, at least one target is an organelle or subcellular component. Non-limiting examples of organelles or subcellular components include chloroplasts, mitochondria, the acrosome, centrioles, cilium/flagellum, the endoplasmic reticulum (rough and smooth), glyoxysomes, the golgi apparatus, lysosomes, melanosomes, myofibrils, the nucleus, the nucleolus, parenthesomes, peroxisomes, ribosomes, vacuoles, vesicles, microtubules, the cell membrane or fragments thereof, the nuclear membrane or fragments thereof, and the cytoskeleton or fragments thereof. In an additional embodiment, at least one target is a cell. In another embodiment, at least one target is a blood cell. Non-limiting examples of blood cells include red blood cells, white blood cells, and platelets. In a further embodiment, at least one target is a contaminant. Non-limiting examples of contaminants include environmental contaminants and interferents.

In another embodiment, at least one target is a nucleic acid. Nucleic acids may vary in length, i.e., polynucleotides, oligonucleotides, and include, but are not limited to, DNA, mtDNA (mitochondrial DNA), RNA, PNA (peptide nucleic acid), LNA (locked nucleic acid), mRNA, ncRNA (non-coding RNA, i.e., tRNA, rRNA), miRNA (micro-RNA), rRNA, siRNA (small interfering RNA), shRNA (short hairpin RNA), dsRNA. Also, nucleic acids may be double-stranded or single-stranded, and may include modified nucleotides.

In an alternative embodiment, at least one target is a biomolecule. Non-limiting examples of a biomolecule include lipids, proteins, carbohydrates and nucleic acids, or combinations thereof. In one embodiment, at least one target is a lipid. In another embodiment, at least one target is a protein. Non-limiting examples of proteins include peptides, polypeptides, protein fragments, protein complexes, recombinant proteins, phosphoproteins, glycoproteins and lipoproteins. In certain embodiments of the invention, at least one target is a protein complex, for instance, a protein-nucleic acid complex. In other embodiments, at least one target is a phosphoprotein or glycoprotein. In particular embodiments of the invention, at least one target is a lipoprotein.

In certain embodiments of the invention, at least one target is a serum / plasma component. In one embodiment of the invention, at least the two, five, or eight highest concentration serum / plasma components are targets. In another embodiment, at least the ten, twelve, or fifteen highest concentration serum / plasma components are targets. In yet another embodiment, at least the eighteen, twenty, or twenty-five highest concentration serum / plasma components are targets. In still another embodiment, at least the thirty, thirty-five, or forty highest concentration serum / plasma components are targets. In still yet another embodiment, at least the fifty, sixty, or seventy highest concentration serum / plasma components are targets. In an additional embodiment, at least the eighty, ninety, or one hundred highest concentration serum / plasma components are targets. In a further embodiment, at least the hundred-fifty highest concentration serum / plasma components are targets. Examples of the target serum / plasma components include globulins, complement proteins, apolipoproteins, coagulation factors, albumins, proteins that bind or transport ions, protease inhibitors, and acute phase reactants. In one embodiment, the target serum / plasma components are globulins. Non-limiting examples of globulins include alpha globulins, beta globulins, and gamma globulins. In another embodiment, the target serum / plasma components are gamma globulins, including IgG, IgM, IgA, IgE, IgD, and IgY. In yet another embodiment, the target serum / plasma components are complement proteins. Non-limiting examples of complement proteins include C1q, C1r, C1s, C2, C3, C4, C5, C6, C7, C8, and C9. In still another embodiment, the target serum / plasma components are apolipoproteins, including apolipoprotein A1, A2, B, C, D, and E. In still yet another embodiment, the target serum /plasma components are proteins involved in the clotting cascade. Non-limiting examples of proteins involved in the clotting cascade include fibrinogen, fibronectin, prothrombin, thrombin, antithrombin, plasminogen, factors V, VI, VII, VIII, IX, X, XI, XII, XIII, von Willebrand factor, prekallikrein, and urokinase. In a further embodiment, the target serum / plasma components are albumin and prealbumin. In yet a further embodiment, the target serum /plasma components are proteins that bind or transport ions, including transferrin, haptoglobin, and ceruloplasmin. In a still further embodiment, the target serum /plasma components are protease inhibitors, including serine protease inhibitors. In still yet a further embodiment, the target serum /plasma components are acute phase reactants. Non-limiting examples of acute phase reactants include C-reactive protein, Alpha 1-antitrypsin, Alpha 1-antichymotrypsin, Alpha 2-macroglobulin, complement proteins, and serum amyloid protein. In an exemplary embodiment, the target serum / plasma components are immunoglobulins, plasminogen, albumin, transferrin, haptoglobin, C3, Alpha-1-antitrypsin, alpha 1 acid glycoprotein, apolipoprotein A1, apolipoprotein A2, apolipoprotein B, ceruloplasmin, C4, C1q, Alpha-2-macroglobulin, fibrinogen, prealbumin, or some combination thereof. Alternatively, a target serum / plasma component may be a microorganism.

In some embodiments, the invention further comprises fractionating the non-targets of the sample at the same time that targets are separated from the sample. Similar to targets, non-targets may include, but are not limited to, analytes, macromolecular complexes, microorganisms, prions, organelles or subcellular components, cells, contaminants, and biomolecules, detailed above. Targets and non-targets from the sample may comprise different types of entities. As an example, the targets may be biomolecules and the non-targets may be small metabolites. In a preferred embodiment, the targets and the non-targets comprise serum / plasma components. In an exemplary embodiment, the non-targets comprise low abundance proteins, glycoproteins, or lipoproteins. The number of non-targets that may be fractionated or separated into different factions can and will vary depending on the fractionation technology and detection methods. In one embodiment, the number of non-targets fractionated ranges from about 1,000 to about 10,000. In another embodiment, the number of non-targets fractionated ranges from about two to about 1,000. In still another embodiment, the number of non-targets fractionated ranges from about 10 to about 500. In a further embodiment, the number of non-targets fractionated ranges from 50 to about 100.

### (c) Reaction Conditions

The sample is generally contacted with a separation composition of the invention under reaction conditions (e.g., temperature, buffer, and incubation time) that facilitate the binding of the epitope binding agents to the targets. As will be appreciated by a skilled person, buffer selection, pH, temperature and incubation time, needed to facilitate optimum binding conditions, can and will vary. The sample is generally mixed with a buffer before being contacted with the separation composition. In certain embodiments, the buffer is isotonic. In other embodiments, the buffer is comprised of salt and phosphate. The buffer may optionally include protease inhibitors. In one embodiment, the buffer has a physiological pH. In another embodiment, the buffer has a neutral pH. In yet another embodiment, the buffer has a pH from about 4.0 to 9.0. In still yet another embodiment, the buffer has a pH from about 5.5 to 8.5. In a further embodiment, the buffer has a pH from between about 6.0 to 8.0. In certain embodiments, the buffer has a pH of between about 6.9 to 7.5.

The temperature at which the separation is conducted is typically selected to optimally balance the binding affinity of the epitope binding agents against the possible degradation of the sample. In general, the sample may be contacted with the separation composition at a temperature between about 2°C and 45°C. In one embodiment, the sample is contacted with the separation composition at a temperature between about 2°C and 10°C. In another embodiment, the sample is contacted with the separation composition at a temperature between about 3°C and 8°C. In yet another embodiment, the sample is contacted with the separation composition at a temperature between about 10°C and 30°C. In still another embodiment, the sample is contacted with the separation composition at a temperature between about 15°C and 28°C. In a further embodiment, the sample is contacted with the separation composition at a temperature between about 20°C and 28°C. In yet a further embodiment, the sample is contacted with the separation composition at a temperature between about 28°C and 45°C. In yet still a further embodiment, the sample is contacted with the separation composition at a temperature between 30°C and 40°C. In certain embodiments, the sample is contacted with the separation composition at a temperature about 37°C. Of course, as will be appreciated by a skilled person, the incubation temperature will depend greatly on the length of the incubation.

Generally speaking, the sample and the separation composition of the invention are incubated together for a duration of time sufficient to allow for adequate binding of the epitope binding agents to targets in the sample. In one embodiment, the sample and the separation composition may be incubated together using a batch treatment. The incubation time may be between about 1 second and 18 hours. In one embodiment, the sample and the separation composition of the invention are incubated together overnight. In another embodiment, the sample and the separation composition are incubated together for between 5 min and 1 hr. In yet another embodiment, the sample and the separation composition are incubated together for between about 5 min and 20 min. In a further embodiment, the sample and the separation composition are incubated together for between about 8 min and 15 min. In yet a further embodiment, the sample and the separation composition are incubated together for about 10 min.

After the sample and the separation composition have been incubated together for a sufficient time under suitable reaction conditions, and the epitope binding agents generally bind to the targets in the sample, the non-targets may be separated from the targets by removing the un-bound sample from the separation composition. Non-limiting ways of removing the un-bound sample include washing the separation composition with sample-free buffer, spinning the separation composition to remove un-bound sample. In one embodiment the separation composition is washed with buffer, and then centrifuged to remove the un-bound sample and buffer. In certain embodiments, the separation composition may be washed 1, 2, 3, 4, 5, 6, 7, or more times. For a detailed example of removing the non-target (un-bound) sample from the separation composition, see the Examples. Alternatively, the un-bound sample and the separation composition may remain together. This mixture may be used for further processes, such as an ELISA (Enzyme Linked Immunosorbent Assay), FACS (Fluorescent Activated Cell Sorting) analysis, or other analysis.

In another embodiment of this method, binding between the targets and the separation composition of the invention may be achieved by column chromatography. The type of chromatography may be spin-column chromatography, fast protein liquid chromatography (FPLC), low pressure liquid chromatography, and high performance liquid chromatography (HPLC). In one embodiment, the chromatography comprises FPLC. For chromatographic separation, the solid support comprising the separation composition is generally packed into a column. The solid support will generally comprise a chromatography matrix, such as resin or beads. Non-limiting examples of chromatographic matrices include agarose beads, polyacrylamide beads, polyacrylic beads, copolymer beads, silica beads, and magnetic beads. In a preferred embodiment, the solid support comprises agarose beads. The size of the column can and will vary depending upon the sample size and the application. In one embodiment, the column may comprise 0.3 ml of chromatography matrix. In another embodiment, the column may comprise 10 ml of chromatography matrix. Typically, the sample is applied to the column, and then the column is rinsed with a buffer to remove the unbound non-targets. The rates of flow of the sample and the buffer through the column can and will vary depending upon the type of chromatography. Fractions of the column flow through, which contain non-targets, may be collected. Non-targets may be separated on the basis of size, charge, shape, hydrophobicity, polarity, or interactions with targets bound to the solid support. In a preferred embodiment, non-targets are fractionated over agarose beads that have size exclusion properties. Once the column is rinsed free of non-targets, the targets may be eluted, as described below.

Under certain circumstances, it may be desirable to contact the sample with the separation composition more than once in order to more completely separate additional targets from the sample. For instance, the separation composition may be contacted with the sample and the non-target (un-bound) sample collected. That non-target (un-bound) sample may then be contacted with the separation composition a second time. The sample and the separation composition can be contacted for a total of 1, 2, 3, 4, or 5 times. In one embodiment, the separation composition and the sample are contacted for a total of 1, 2, or 3 times. In another embodiment, the separation composition and the sample are contacted for a total of 2 times.

The non-target (un-bound) sample or fractions, once separated from the separation composition, can be stored, or subjected to further analysis by methods generally known in the art. Non-limiting examples of analysis techniques include blotting (i.e. Western blotting, Northern blotting, Southern blotting, or a combination), FACS analysis, electrophoresis (one and two dimensional), liquid chromatography, high performance liquid chromatography, mass spectrometry, capillary electrophoresis (CE), cell culture, and ELISA analysis. Alternatively, the non-target (un-bound) sample may be used therapeutically. In one embodiment, the non-target (un-bound) samples are stored at less than 0°C. In another embodiment, the non-target (un-bound) samples are stored at less than cooler temperatures (i.e., 2 °C - 8 °C).

The bound targets may be eluted from the separation composition. Elution can be accomplished by any means that disrupts the binding between the epitope binding agent and the targets. For instance, low pH, high salt concentration, or high temperature can be used to elute the epitope binding agents from the targets. Alternatively, a competitor can be used to disrupt the binding between the epitope binding agent and the target. In one embodiment, a solution of acidic glycine is used to elute the bound targets. In another embodiment, a solution with a pH between about 1.0 and 4.0 is used to elute the bound targets. In yet another embodiment, a solution with a pH between about 2.0 and 3.0 is used to elute the bound target. In still yet another embodiment, a solution with a pH about 2.5 is used to elute the bound target. In a further embodiment, a solution of acid glycine is used to elute the bound target. I n a still further embodiment, a solution of 0.1 M glycine at pH 2.5 is used to elute the bound target. In a preferred embodiment a solution of 0.1 M glycine at pH 2.5 containing detergent is used to elute the bound target. In an exemplary embodiment a solution of 0.1 M glycine at pH 2.5 containing 0.8 % (v/v) Tween 20 detergent is used to elute the bound target. Alternatively in another embodiment, for some applications a basic or high pH elution (viz. ph = 7 to 14) may be chosen or preferred due to the nature of the interaction between the bound molecules and the resin. Once eluted, the pH of the eluate may be neutralized. The eluate may either be used, such as for Western blots, FACS analysis, electrophoresis, or ELISA analysis, stored for later use, or disposed of. Alternatively, the target(s) may be stored bound to the separation composition for later use.

Once the bound target is removed from the epitope binding agents of the separation composition, the separation composition may be regenerated in accordance with methods generally known in the art, as illustrated in the examples, and used for additional separations.

### (IV) Kit for Separating Targets from Non-targets

The separation composition and the method of the present invention may be combined to create a kit for separating targets from non-targets. In one embodiment, the basic kit comprises the separation composition of the invention and instructions for separating at least two distinct targets from a sample comprised of a mixture of targets and non-targets. The kit may further comprise a chromatographic column comprising the separation composition of the invention. In a preferred embodiment the invention encompasses an enhanced kit, including all concomitant equilibration and elution buffers, etc., for the separation of at least two distinct targets from a sample comprising a mixture of targets and non-targets. In an exemplary embodiment the *complete* kit comprises a separation composition of the invention, all purpose designed and built companion solutions, (including preservatives), equilibration and elution buffers, necessary "techware" and detailed instructions for use.

### DEFINITIONS

The term "contaminant" refers to a substance that is not ordinarily found in a sample.

The term "*en masse*" as used herein, refers to the manner in which the epitope binding agents are conjugated to the solid support. In this context, it means that substantially all the heterogeneous epitope binding agents are conjugated to the solid support in substantially one set of reactions. This is in lieu of individually conjugating each epitope binding agent to the solid support in a series of separate reactions.

The term "epitope binding agent" refers to a substance that is capable of binding to a specific epitope of an antigen, a peptide, a polypeptide, a protein or a macromolecular complex. Non-limiting examples of epitope binding agents include aptamers, double-stranded DNA sequence, ligands and fragments of ligands, receptors and fragments of receptors, antibodies and fragments of antibodies, potynucleotides, coenzymes, coregulators, allosteric molecules, and ions.

The term "hydrocarbyl" as used herein describe organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The term "interferent" refers to a substance that interferes with a desired reaction, process, or method.

The term "macromolecular complex" refers to a composition of matter comprising a macromolecule. Preferably, these are complexes of one or more macromolecules, such as polypeptides, proteins, lipids, carbohydrates, nucleic acids, natural or artificial polymers and the like, in association with each other. The association may involve covalent or non-covalent interactions between components of the macromolecular complex. Macromolecular complexes may be relatively simple, such as a ligand bound polypeptide, relatively complex, such as a lipid raft, or very complex, such as a cell surface, virus, bacteria, spore and the like. Macromolecular complexes may be biological or non-biological in nature.

The term "non-target" is used in its broadest interpretation to mean any substance, atom, molecule, component or cell, as detailed herein, that does not bind the separation composition of the invention. In some embodiments, non-targets may be fractionated from other non-targets.

The term "plurality" as used herein means at least two.

The term "small molecule" as used herein refers generally to a ligand, chemical moiety, compound, ion, salt, metal, enzyme, secondary messenger of a cellular signal transduction pathway, drug, nanoparticle, environmental contaminant, toxin, fatty acid, steroid, hormone, carbohydrate, amino acid, peptide, polypeptide, or other amino acid polymer or any other agent which is capable of being an epitope binding agent.

The term "stochastically" as used herein, refers to the manner in which the epitope binding agent is conjugated to the solid support. In this context, it means that the epitope binding agents are randomly conjugated to the solid support. Stated another way, the epitope binding agents are conjugated to the solid support in a non-oriented fashion in lieu of being conjugated to the solid support in an oriented manner.

The term "substituted hydrocarby" moieties described herein are hydrocarbyl moieties which are substituted with at least one atom other than carbon, including moieties in which a carbon chain atom is substituted with a hetero atom such as nitrogen, oxygen, silicon, phosphorous, boron, sulfur, or a halogen atom. These substituents include halogen, carbocycle, aryl, heterocyclo, alkoxy, alkenoxy, alkynoxy, aryloxy, hydroxy, protected hydroxy, keto, acyl, acyloxy, nitro, amino, amido, nitro, cyano, thiol, ketals, acetals, esters and ethers.

The term "target" is used in its broadest interpretation to mean any substance, atom, molecule, or cell, as detailed herein, that is to be detected, separated, or separated and purified from the sample. A single target is deemed to (in the case of proteins, peptides, and polypeptides) include all variations conferred by Post-Translational Modifications (PTMS).

As various changes could be made in the above compounds, products and methods without departing from the scope of the invention, it is intended that all matter contained in the above description and in the examples given below, shall be interpreted as illustrative and not in a limiting sense.

### EXAMPLES

The following examples illustrate various embodiments of the invention.

### Example 1: En masse conjugation of antibodies to the top sixteen human serum proteins based on concentration.

This example demonstrates an attempt at the resin optimization process.

### SATA Activation of the Antibodies

The absorbance at 280 nm (A₂₈₀) of the different antibodies were read to determine concentration. An extinction coefficient of 1.4 was used. The amount of each antibody needed for coupling to 1 ml of resin is presented in Table 1-1 below. Since 3 ml of resin was coupled, the amount of antibody needed for SATA activation is also presented in Table 1-1.

The 14 IgG antibodies were pooled and concentrated in four YM-50 Centricons for 7 hrs at 5,000 x g to obtain a final volume of 2 ml. The A₂₈₀ of these IgG antibodies was measured again on duplicate samples to determine the final concentration of the pooled IgG Abs (Table 1-2). The mixture of concentrated antibodies was adjusted to a final buffer concentration of 50 mM sodium phosphate, 0.5 M NaCl, and 1mM EDTA.

The single chained recombinant antibody fragments were combined in a separate tube, using the volumes calculated in the upper part of Table 1-1.

**Table 1-1: Details of antibodies (including concentrations) and activation reagents used to generate a 16-plex resin.**

| **Antibody** | **Conc mg/ml** | **mg needed/ml resin** | **50% increase to account for loss** | **mg for SATA activation of 3 ml** | **Volume used for SATA activation (ml)** |
|---|---|---|---|---|---|
| **Albumin** | 9.9 | 2.8 | 4.2 | 12.6 | 1.27 |
| **lgG** | 9.7 | 0.5 | 0.75 | 2.25 | 0.23 |
| **Total recombinant antibody fragments** | | 3.3 | | 14.85 | 1.50 |
| | | | | | |
| **Transferrin** | 1 | 0.5 | 0.75 | 2.25 | 2.25 |
| **Fibrinogen** | 2 | 0.32 | 0.48 | 1.44 | 0.72 |
| **IgA** | 3 | 0.35 | 0.525 | 1.58 | 0.53 |
| **alpha-2-Mac** | 1.2 | 0.39 | 0.585 | 1.76 | 1.46 |
| **IgM** | 3.2 | 0.13 | 0.195 | 0.59 | 0.18 |
| **alpha-1-Antitrypsin** | 1.5 | 0.75 | 1.125 | 3.38 | 2.25 |
| **Haptoglobin** | 0.5 | 0.25 | 0.375 | 1.125 | 2.25 |
| **Orosomucoid (Acid-1-Glycoprotein)** | 1 | 0.25 | 0.375 | 1.125 | 1.13 |
| **Ceruloplasmin** | 1 | 0.2 | 0.3 | 0.9 | 0.90 |
| **IgD** | 1 | 0.15 | 0.225 | 0.675 | 0.68 |
| **Apo A1** | 0.26 | 0.25 | 0.375 | 1.125 | 4.33 |
| **C1q** | 1.15 | 0.1 | 0.15 | 0.45 | 0.39 |
| **C3** | 1.5 | 0.17 | 0.255 | 0.765 | 0.51 |
| **HDL** | 0.39 | 0.17 | 0.255 | 0.765 | 1.96 |
| **Total IgG's** | | **3.98** | | **17.91** | **19.53** |

**Table 1-2: Calculations showing total concentrations of antibody prior to and after concentration.**

| | A₂₈₀ | A₂₈₀ | Ave | dil | A₂₈₀ *dil | Extinction Coefficient | mg/ml | Vol (ml) | mg |
|---|---|---|---|---|---|---|---|---|---|
| Before conc | 0.0238 | 0.0258 | 0.0248 | 50 | 1.24 | 1.4 | 0.89 | 19.53 | 17.3 |
| After conc | 0.1701 | 0.1658 | 0.16795 | 50 | 8.3975 | 1.4 | 6.00 | 2.15 | 12.9 |

The amount of SATA required for the activation of the antibody was calculated with a 10 (for the recombinant antibody fragments) or 40 (for the IgG antibodies) molar excess of SATA:
recombinant antibody fragments:
   ➢ 12,000 mg/mmole = 0.0000833 mmole/mg
   ➢ 0.0000833 mmole/mg x 10 = 0.00083 mmoles SATA/mg Ab
   ➢ 0.00083 mmole/mg Ab / 0.25 mmoles/ml SATA = 3.3 µl of 0.25 M SATA/mg Ab
IgG antibodies:
   ➢ 150, 000 mg/mmole Ab = 0.0000067 mmole/mg Ab
   ➢ 0.0000067 mmole/mg Ab x 40 = 0.000267 mmole SATA/mg Ab
   ➢ 0.000267 mmole/mg Ab / 0.25 mmoles/ml SATA = 1.07 µl of 0.25 M SATA/mg Ab

Fresh SATA (Sigma Product Code A 9043) was prepared in dimethylformamide (DMF) to obtain a 0.25 M solution (58 mg / ml;15.3 mg / 265 µl DMF). A sample (25 µL) of the antibody mixtures was removed, prior to conjugation, for later assays. Then a 10 molar excess of the SATA (49 µl of the 0.25M solution) was added to the recombinant antibody fragments and 40 molar excess of SATA (13.8 µl of the 0.25M solution) was added to the other antibodies. The solutions were mixed and allowed to react for approximately 1 hour at room temperature.

Afterwards, the percent of remaining amines on the antibodies was measured by a fluorescamine assay. A 2 mg / ml fluorescamine solution was prepared in DMSO. The reagents were combined, as per Table 1-3 below, into individual 2 ml amber tubes and gently mixed. The plate reader was recalibrated, and two 0.2 ml aliquots from each tube were placed into duplicate wells of a multiwell fluorescence plate (Corning plate, Product Code 3916). The plate was read on a fluorescence plate reader at an excitation wavelength of 390 nm and an emission wavelength of 480 nm. The percent amines remaining was calculated using the following equation: (fluorescence of SATA activated Ab - blank fluorescence) / (fluorescence of Ab solution - blank fluorescence) x 100 = % amines (Table 1-3). Because the level of remaining amines on the lgG antibodies was higher than desired, 12 µl of additional SATA was added to the lgG reaction, and the mixture was incubated for a second time.

**Table 1-3: Estimations of activation level based on fluorescamine assay.**

| **Sample** | **0.05 M Na phosphate, pH 8.2** | **Fluoresc 2 mg/ ml** | **A₄₈₀** | **A₄₈₀** | **A₄₈₀** | **A₄₈₀** | **Avg** | **% amines remaining** | **% amines coupled** |
|---|---|---|---|---|---|---|---|---|---|
| (Blank) | 1 ml | 10 ml | 0 | | | | | | |
| 5 µl recombinant antibody fragments | 1 ml | 10 ml | 899 | 831 | 890 | 848 | 867 | 100 | 0 |
| 5 µl recombinant antibody - SATA | 1 ml | 10 ml | 130 | 110 | 111 | 109 | 115 | 13 | 87 |
| (Blank) | 1 ml | 10 ml | 0 | | | | | | |
| 5 µl IgG antibodies | 1 ml | 10 ml | 495 | 454 | 537 | 548 | 509 | 100 | 0 |
| 5 µl IgG antibodies-SATA | 1 ml | 10 ml | 344 | 306 | 385 | 374 | 352 | 69 | 31 |

After SATA activation, the recombinant antibody fragment mix was diluted 10 fold (15 ml) with PBS-EDTA, pH 6.7, and concentrated to the original volume on a stir cell with a 3,000 MW cut off membrane. The recombinant antibody fragment mix was again diluted 10 fold with PBS-EDTA, pH 6.7 (15 ml) and concentrated to approximately 60% of the original volume. The A₂₈₀ of the Ab mix prior to SATA activation and the concentrate after SATA activation was measured in duplicate by diluting 20 fold in PBS-EDTA, pH 6.7 (Table 1-4).

**Table 1-4: Estimation of total antibody concentration before and after SATA activation of the small recombinant antibody ligands.**

| **Sample** | **Volume (ml)** | **A₂₈₀** | **A₂₈₀** | **A₂₈₀** avg with dilution | **mg/ml** | **Total mg** |
|---|---|---|---|---|---|---|
| recombinant antibody fragments before SATA | 1.5 | 0.172 | 0.1689 | 8.52 | 9.9 | 14.85 |
| recombinant antibody fragments after stir cell | 1.5 | 0.1443 | 0.1423 | 7.17 | 8.3 | 12.48 |
| First flow thru | 15 | 2.15 | | | | |
| 2^{nd} flow thru | 15 | 0.4112 | | | | |

After SATA activation, the IgG antibody mixture was gel filtered. A 20 ml column of Sephadex G25-80 was poured and equilibrated with 3 column volumes of PBS-EDTA, pH 6.7. The SATA activated lgG antibody mixture was added to the top of the column and the column was eluted with PBS-EDTA; 2 ml fractions were collected. The A₂₈₀ value of the fractions was read after diluting a 20 µl sample to 1 ml with PBS-EDTA (Figure 1). Fractions 5-6 were pooled. Fraction 7 was concentrated to 0.2 ml and added to the pool. The A₂₈₀ value of the pooled fractions was measured in duplicate (Table 1-5). (Samples were diluted 1:50 in PBS-EDTA; an extinction coefficient of 1.4 was used.)

**Table 1-5: Estimation of total IgG antibody concentration after SATA activation.**

| Sample # | A₂₈₀ | A₂₈₀ | mg/ml | Vol | mg |
|---|---|---|---|---|---|
| 5-7 | 0.0733 | 0.0742 | 2.63 | 3.9 | 10.3 |

### B. Activation of the Solid Support

Four mls of epoxy activated resin reacted with ammonium hydroxide (see Example 4) were washed with 5 volumes of 0.05 M NaH₂PO₄, pH 7.0. In a poly container, a 50% (1:1) gel suspension was prepared in 0.05 M NaH₂PO₄, pH 7.0. The total volume of buffer used was 4 ml. Three µmoles of maleimidocaproic acid (MA) NHS ester were dissolved per ml of gel in DMF to generate a 25 mg / ml MA NHS ester solution.
Volume of gel = 4 ml
Amount of MA NHS ester needed =
   (3µmol / ml gel) x 4 ml x 1/1000 mmol / µmole x 308.3 mg MA NHS ester / mmol = 3.7 mg MA NHS ester
Amount of MA NHS ester weighed out = 5.7 mg
   5.7 mg MA NHS ester / 25 = 0.228 (ml)
Amount of DMF to add to the weighed out MA NHS ester
   3.7 mg MA NHS ester needed / 25 mg MA NHS ester / ml = 0.148 ml of 25 mg/ml MA NHS ester in DMF to add to the resin suspension.

The MA NHS ester/DMF solution was slowly added to the mixing gel suspension. The suspension was mixed at room temperature for 30 minutes. The unreacted amines on the resin were acetylated by slowly adding acetic anhydride (0.028 x 4 ml resin = 0.112 ml acetic anhydride) and mixing for 15 minutes. The resin slurry was transferred to a Buchner filter funnel and washed with 4 volumes of deionized H₂O. The resin was filtered to a damp cake, which was stored in the cold room until needed.

### C. Coupling of the Antibody to the Solid Support

Fresh 1 M hydroxylamine pH 6.7 (10 ml) was made by dissolving 695 mg of hydroxylamine and adjusting the pH with 1 M NaOH (approximately 8 ml was required). The reagents listed in Table 1-6 were added to 15 ml Corning tubes. The maleimide-activated resin was added first, then the antibodies were added and mixed, followed by the hydroxylamine. The tubes were mixed overnight in the cooler. The resin was washed twice by resuspending in 3 column volumes of 3x PBS and centrifuging for 1 min at 2,000 rpm in a clinical centrifuge. The resin was then washed twice with 3 column volumes of 0.1 M Bis-Tris Propane, pH 8.5. One column volume of 0.1 M Bis-Tris propane, pH 8.5, 30 mM lodoacetamide was added to all tubes to block excess sulfhydryl moieties. This blocking step was carried out at room temperature on an orbital mixer for 30 minutes. The resin was washed three times with three column volumes of water. The resin was then washed three times with three column volumes of 2x PBS, pH 7.5. Sufficient glycerol was added to generate a final solution containing 50% glycerol / 50% PBS, and the resin was stored at 4 °C.

**Table 1-6: Reagents and quantities employed to couple antibodies to solid support using maleimide chemistry.**

| **Ab type** | | **Ab conc (mg/ml)** | | **mg Ab/ml resin** | **ml of resin** | **Vol of Ab (ml)** | | **Buffer (0.5M Na P pH 5.6)** | | **Hydroxyamine (ml)** | **Total Vol (ml)** |
|---|---|---|---|---|---|---|---|---|---|---|---|
| recombinant antibody fragments | | 8.3 | | 3.3 | 2.5 | 0.99 | | | | | |
| IgG/IgY | | 2.63 | | 3.98 | 2.5 | 3.78 | | | | | |
| Total | | | | 7.28 | 2.5 | 4.78 | | 0 | | 0.187 | 7.5 |

| **Ab type** | **ml of resin** | | **Vol of PBS- EDTA pH 6.7** | | **1M Hydroxyamine, pH 6.7 (ml)** | | **Total vol (ml)** | | | | |
|---|---|---|---|---|---|---|---|---|---|---|---|
| Blank | 0.3 | | 0.575 | | 0.023 | | 0.9 | | | | |

Duplicate 10 µl aliquots of each of the resins (blank and 2a), and 5 µL and 10 µl aliquots of the activated Abs, were assayed by the BCA-1 assay (reagents: 25 ml Reagent A (Sigma Product Code B 9643) and 0.5 ml Reagent B (Sigma Product Code C 2284)). The BCA assay was carried out overnight at room temp on a rotating wheel. The tubes were microcentrifuged at 8,000 rpm for 1 min and 1 ml was transferred to disposable cuvets and read at A₅₆₂ (Table 1-7). The concentration of SATA-activated antibody coupled to the resin was determined by comparing it to the unconjugated SATA-activated antibody with a known concentration.

**Table 1-7: Protein Concentration bound to resin as determined by BCA Assay.**

| **Sample** | **mg Ab/ml resin** | **Buffer** (µl) | **Sample** (µl) | **BCA reagent** | **A₅₆₂** | **µg** | **A₅₆₂** | **µg** | **mg/ml (BSA)** | **BSA to ligand mult fact.** | **mg protein/ml resin** | **avg mg/ml - bland** | **avg % coupling - blank** |
|---|---|---|---|---|---|---|---|---|---|---|---|---|---|
| 0 std | | 750 | 0 | 750 | 0 | | | | | | | | |
| 10 std | | 740 | 10 | 750 | 0.3295 | | | | | | | | |
| 20 std | | 730 | 20 | 750 | 0.5827 | | | | | | | | |
| 40 std | | 710 | 40 | 750 | 1.0093 | | | | | | | | |
| 60 std | | 690 | 60 | 750 | 1.385 | | | | | | | | |
| 100 std | | 650 | 100 | 750 | 2.1053 | | | | | | | | |
| blank | 0 | 740 | 10 | 750 | 0.3084 | 10.3 | 0.2784 | 9.1 | 1.9 | 0.78 | 1.5 | 0.0 | |
| 2a | 7.28 | 740 | 10 | 750 | 1.1373 | 46.0 | 1.1411 | 46.2 | 9.2 | 0.78 | 7.2 | 5.7 | 78 |
| antibody- SATA | 3.8 | 740 | 5 | 750 | 0.649 | 24.2 | 0.6532 | 24.4 | 4.9 | 0.78 | | | |
| antibody- SATA | 3.8 | 740 | 10 | 750 | 1.2286 | 50.4 | 1.1548 | 46.9 | 4.9 | 0.78 | | | |

### D. Plasma Separation

Aliquots of the resin were added to microcolumns as indicated in Table 1-8 below. The volume of 50% slurry is 2x the final volume of resin.

**Table 1-8: Plasma and wash volumes for each specific resin volume.**

| **Sample #** | **Resin vol** (µl) | **Final vol of 25** µl **Plasma** | **Wash vol (2X)** (µl) | **Final vol** (µl) | **Final dil** |
|---|---|---|---|---|---|
| 1 | 200 of blank | 57 | 57 | 171 | 6.9 |
| 2 | 200 | 57 | 57 | 171 | 6.9 |
| 3 | 350 | 100 | 100 | 300 | 12.0 |
| 4 | 500 | 143 | 143 | 429 | 17.1 |
| 5 | 750 | 214 | 214 | 643 | 25.7 |
| Total | 1800 | | | | |

The snap off bottoms of the columns were removed, the caps loosened and the columns placed into 2 ml collection tubes. The columns were microcentrifuged at 8,000 rpm for 5 sec. The columns were equilibrated with three washes of 1 column volume of PBS at 8,000 rpm for 5 sec. The final spin was carried out for 30 sec. Plasma (citrated) was diluted with PBS as indicated in the table below (Table 1-9). The plasma was diluted so that the final volume of the plasma sample was 28.5% of the volume of the resin to be absorbed.

**Table 1-9: Plasma dilution conditions for each specific resin volume.**

| **Sample #** | **Resin vol** (µl) | **Final vol of 25** µl **Plasma** | **2x vol of plasma** (µl) | **2x vol of PBS** (µl) |
|---|---|---|---|---|
| 1 | 200 of blank | 57 | 50 | 64 |
| 2 | 200 | 57 | 50 | 64 |
| 3 | 350 | 100 | 50 | 150 |
| 4 | 500 | 143 | 50 | 236 |
| 5 | 750 | 214 | 50 | 379 |

An aliquot of the diluted plasma was added to each column and incubated for 10 min. The columns were then centrifuged at 10,000 rpm for 1 min. The depleted plasma was reapplied to the column and incubated for 10 min. Again, the columns were subsequently centrifuged at 10,000 rpm for 1 min. An aliquot of PBS equal to the diluted plasma volume was added to each column and then centrifuged at 10,000 rpm for 1 min. This wash step was repeated. The pooled, depleted plasma was stored at -20 °C.

The columns were stripped of bound protein three times with a wash of one diluted plasma volume of 20 mM HEPES, 2M MgCl₂, pH 7.4, followed by centrifugation at 10,000 rpm for 1 min. The pooled elution was stored at -20 °C. After elution the columns were washed twice with one column volume of 3x PBS. Then one column volume of PBS was added to the columns, and they were stored at 4 °C.

### E. Analysis

### ELISA Assay (direct) of all 16 proteins

The separated plasma samples were diluted with serial 10 fold dilutions of coating buffer (100, 1,000, and 10,000 fold; see Table 1-10). The whole plasma samples were diluted with serial 10 fold dilutions of coating buffer (100, 1,000, 10,000, 100,000, and 1,000,000 fold; see Table 1-10). Nine plates were coated by adding 0.1 ml of diluted samples to each well. Plates were incubated at 4 °C overnight.

**Table 1-10: Antibody Dilution Table mapped to a 96 well plate format.**

| | 1 | 2 | 3 | 4 | 5 | 6(P) | 7(1) | 8(2) | 9(3) | 10(4) | 11(5) | 12 (P) |
|---|---|---|---|---|---|---|---|---|---|---|---|---|
| A | E6 | 10000 | 10000 | 10000 | 10000 | E6 | E6 | 10000 | 10000 | 10000 | 10000 | E6 |
| B | E6 | 10000 | 10000 | 10000 | 10000 | E6 | E6 | 10000 | 10000 | 10000 | 10000 | E6 |
| C | E5 | 1000 | 1000 | 1000 | 1000 | E5 | E5 | 1000 | 1000 | 1000 | 1000 | E5 |
| D | E5 | 1000 | 1000 | 1000 | 1000 | E5 | E5 | 1000 | 1000 | 1000 | 1000 | E5 |
| E | E4 | 100 | 100 | 100 | 100 | E4 | E4 | 100 | 100 | 100 | 100 | E4 |
| F | E4 | 100 | 100 | 100 | 100 | E4 | E4 | 100 | 100 | 100 | 100 | E4 |
| G | 1000 | Bl | | | | 1000 | 1000 | Bl | | | | 1000 |
| H | 1000 | Bl | | | | 1000 | 1000 | Bl | | | | 1000 |

After incubation, the plates were washed 3 times with TBS-T using a platewasher. Next, the plates were blocked with a 0.3 ml of TBS, 1% BSA solution. The blocking solution was incubated with the plates for a minimum of 30 minutes at room temp, and was then aspirated from the wells.

### For the anti-human plasma protein primary antibodies:

Each anti-human plasma protein antibody was diluted (1-20 mg / ml) 1:1,000 in TBS, 1% BSA, and 0.1 ml of the diluted antibody was added to each well. The anti-human antibody was incubated at 37 °C for 90 minutes. The plates were then washed 3 times with TBS-T using a platewasher.

### For the HRP-conjugated secondary antibodies:

The anti chicken and goat HRP conjugates were diluted 1:40,000 in TBS, 1% BSA. Specifically 5 µl of the anti Chicken HRP conjugate was added to 195 µl of TBS-BSA and then 75 µl was diluted to 75 ml. Then 5 µl of Goat HRP conjugate was added to 195 µl of TBS-BSA; next 30 µl of this solution was added to 30 ml of TBS-BSA. The anti mouse HRP conjugate was diluted 1:50,000. Specifically 5 µl of the anti mouse HRP conjugate was added to 245 µl of TBS-BSA and then 12 µl of this solution was added to 12 ml of TBS-BSA. Next 0.1 ml of each HRP conjugate was added to each well and incubated at 37 °C for 90 minutes. The plates were washed 3 times with TBS-T using a platewasher. Then 0.1 ml of TMB was added (cold, directly out of the refrigerator) to each well and incubated at room temperature for the amount of time indicated in Table 1-12, below. The reactions were stopped by the addition of 0.1 ml 1M HCl. All of the plates were read at 450 nm. Results are detailed in Table 1-12 and Figure 2.

A depletion level of greater than 95% of each target protein from 25 µl of plasma using 500 µl of resin was the goal for this experiment. Four of the antibodies (HDL, C3, Apo A1 and Acid1 glycoprotein) depleted at less than 85% using 750 µl of resin. The C1q antibody depleted at 94% using 750 µl of resin. The level of the Haptoglobin, anti-lgD and anti-lgM antibodies gave good depletion using 350 µl of resin. The levels of the remaining antibodies (viz. alpha-1-antitrypsin, Ceruloplasmin, alpha-2-mac, and lgA) depleted at greater than 95% using 500 µl of resin. The depletion levels of albumin and lgG were essentially unchanged as compared to a previous experiment in which a 12 fold level of SATA was used. The MgCl₂ solution did not strip the bound proteins from the resin. The bound proteins were removed by following up with a low pH glycine buffer.

From this example, the following modifications were made for the next experiment. The level of the anti-Haptoglobin antibody was reduced to 0.18 mg / ml of resin. The level of the anti-lgM antibody was reduced to 0.1 mg / ml of resin. The level of the anti-lgD antibody was reduced to 0.1 mg / ml of resin. The level of the C1q antibody was increased to 0.2 mg / ml of resin. The level of the HDL antibody was increased to 0.35 mg/ml. The level of the C3 antibody was increased to 0.35 mg / ml. The level of the Acid-1-glycoprotein antibody was increased to 0.35 mg / ml. The levels of the remaining antibodies remained the same.

**Table 1-11: TMB development times (in minutes) for discrete antibodies.**

| | **AntiTry** | **Cerul** | **A2M** | **Fibrin** | **Hapto** | **Acid 1glyco** | **ApoA1** | **C1q** | **C3** | **HDL** |
|---|---|---|---|---|---|---|---|---|---|---|
| **HRP** | Chick | Chick | Chick | Chick | Chick | Chick | Chick | Chick | Chick | Chick |
| **time** | 2.5 | 3.5 | 4 | 2 | 2.5 | 3.5 | 6 | 9 | 1 | 1 |
| | **IgM** | **Trans** | **IgA** | **IgD** | | **HAS** | **IgG** | | | |
| **HRP** | Goat | Goat | Goat | Goat | | A 9044 | N/A | | | |
| **time** | 6.5 | 6.5 | 2 | 9 | | 5 | 3 | | | |

**Table 1-12: ELISA data for discrete antibodies.**

| **IgG** | | | | | | | | **IgA** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dllut** | **A₄₀₅** x dil | **% rem** | **% depl** |
| **Plasma** | 0.07 | 1 | 1.00E+06 | 1.00E+06 | 0.07 | | | **Plasma** | 0.05 | 1 | 1.00E+06 | 1.00E+06 | 0.05 | | |
| **1** | 0.1 | 6.9 | 1.00E+05 | 6.90E+05 | 0.07 | 100.0 | 0.0 | **1** | 0.07 | 6.9 | 1.00E+05 | 6.90E+05 | 0.05 | 100.0 | 0.0 |
| **2** | 0.09 | 6.9 | 1.00E+04 | 6.90E+04 | 0.01 | 8.7 | 91.3 | **2** | 0.19 | 6.9 | 1.00E+04 | 6.90E+04 | 0.01 | 27.7 | 72.3 |
| **3** | 0.14 | 12 | 1.00E+03 | 1.20E+04 | 0.00 | 2.4 | 97.6 | **3** | 0.2 | 12 | 1.00E+03 | 1.20E+04 | 0.00 | 5.0 | 95.0 |
| **4** | 0.06 | 17.1 | 1.00E+03 | 1.71E+04 | 0.00 | 1.4 | 98.6 | **4** | 0.22 | 17.1 | 1.00E+02 | 1.71E+03 | 0.00 | 0.8 | 99.2 |
| **5** | 0.16 | 25.7 | 1.00E+02 | 2.57E+03 | 0.00 | 0.6 | 99.4 | **5** | 0.17 | 25.7 | 1.00E+02 | 2.57E+03 | 0.00 | 0.9 | 99.1 |

| **HAS** | | | | | | | | **Fibrinogen** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.1 | 1 | 1.00E+06 | 1.00E+06 | 0.10 | | | **Plasma** | 0.19 | 1 | 1.00E+06 | 1.00E+06 | 0.19 | | |
| **1** | 0.16 | 6.9 | 1.00E+05 | 6.90E+05 | 0.11 | 100.0 | 0.0 | **1** | 0.04 | 6.9 | 1.00E+06 | 6.90E+06 | 0.27 | 100.0 | 0.0 |
| **2** | 0.27 | 6.9 | 1.00E+04 | 6.90E+04 | 0.02 | 16.5 | 83.5 | **2** | 0.29 | 6.9 | 1.00E+04 | 6.90E+04 | 0.02 | 7.4 | 92.6 |
| **3** | 0.06 | 12 | 1.00E+04 | 1.20E+05 | 0.01 | 6.2 | 93.8 | **3** | 0.06 | 12 | 1.00E+04 | 1.20E+05 | 0.01 | 2.8 | 97.2 |
| **4** | 0.08 | 17.1 | 1.00E+03 | 1.71E+04 | 0.00 | 1.2 | 98.8 | **4** | 0.24 | 17.1 | 1.00E+03 | 1.71E+04 | 0.00 | 1.5 | 98.5 |
| **5** | 0.12 | 25.7 | 1.00E+02 | 2.57E+03 | 0.00 | 0.3 | 99.7 | **5** | 0.13 | 25.7 | 1.00E+03 | 2.57E+04 | 0.00 | 1.3 | 98.7 |

| **a2M** | | | | | | | | **antitrypsin** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.15 | 1 | 1.00E+06 | 1.00E+06 | 0.15 | | | **Plasma** | 0.07 | 1 | 1.00E+06 | 1.00E+06 | 0.07 | | |
| **1** | 0.15 | 6.9 | 1.00E+05 | 6.90E+05 | 0.10 | 100.0 | 0.0 | **1** | 0.1 | 6.9 | 1.00E+05 | 6.90E+05 | 0.07 | 100.0 | 0.0 |
| **2** | 0.28 | 6.9 | 1.00E+04 | 6.90E+04 | 0.02 | 18.4 | 81.6 | **2** | 0.19 | 6.9 | 1.00E+04 | 6.90E+04 | 0.01 | 19.8 | 80.2 |
| **3** | 0.06 | 12 | 1.00E+04 | 1.20E+05 | 0.01 | 6.6 | 93.4 | **3** | 0.21 | 12 | 1.00E+03 | 1.20E+04 | 0.00 | 3.7 | 96.3 |
| **4** | 0.22 | 17.1 | 1.00E+03 | 1.71E+04 | 0.00 | 3.6 | 96.4 | **4** | 0.05 | 17.1 | 1.00E+03 | 1.71E+04 | 0.00 | 1.3 | 98.7 |
| **5** | 0.15 | 25.7 | 1.00E+03 | 2.57E+04 | 0.00 | 3.7 | 96.3 | **5** | 0.12 | 25.7 | 1.00E+02 | 2.57E+03 | 0.00 | 0.5 | 99.5 |
| **IgM** | | | | | | | | **transferrin** | | | | | | | |
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.12 | 1 | 1.00E+05 | 1.00E+05 | 0.01 | | | **Plasma** | 0.09 | 1 | 1.00E+06 | 1.00E+06 | 0.09 | | |
| **1** | 0.21 | 6.9 | 1.00E+04 | 6.90E+04 | 0.01 | 100.0 | 0.0 | **1** | 0.12 | 6.9 | 1.00E+05 | 6.90E+05 | 0.08 | 100.0 | 0.0 |
| **2** | 0.14 | 6.9 | 1.00E+03 | 6.90E+03 | 0.00 | 6.9 | 93.1 | **2** | 0.12 | 6.9 | 1.00E+04 | 6.90E+04 | 0.01 | 9.7 | 90.3 |
| **3** | 0.09 | 12 | 1.00E+02 | 1.20E+03 | 0.00 | 0.8 | 99.2 | **3** | 0.09 | 12 | 1.00E+03 | 1.20E+04 | 0.00 | 1.3 | 98.7 |
| **4** | 0.07 | 17.1 | 1.00E+02 | 1.71E+03 | 0.00 | 0.9 | 99.1 | **4** | 0.08 | 17.1 | 1.00E+02 | 1.71E+03 | 0.00 | 0.2 | 99.8 |
| **5** | 0.06 | 25.7 | 1.00E+02 | 2.57E+03 | 0.00 | 1.0 | 99.0 | **5** | 0.06 | 25.7 | 1.00E+02 | 2.57E+03 | 0.00 | 0.2 | 99.8 |

| **Hapto** | | | | | | | | **Acid1glyco** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.05 | 1 | 1.00E+06 | 1.00E+06 | 0.05 | | | **Plasma** | 0.15 | 1 | 1.00E+05 | 1.00E+05 | 0.015 | | |
| **1** | 0.07 | 6.9 | 1.00E+05 | 6.90E+05 | 0.05 | 100.0 | 0.0 | **1** | 0.18 | 6.9 | 1.00E+04 | 6.90E+04 | 0.013 | 100.0 | 0.0 |
| **2** | 0.17 | 6.9 | 1.00E+03 | 6.90E+03 | 0.00 | 2.4 | 97.6 | **2** | 0.11 | 6.9 | 1.00E+04 | 6.90E+04 | 0.007 | 58.5 | 41.5 |
| **3** | 0.04 | 12 | 1.00E+03 | 1.20E+04 | 0.00 | 1.0 | 99.0 | **3** | 0.43 | 12 | 1.00E+03 | 1.20E+04 | 0.005 | 41.2 | 58.8 |
| **4** | 0.12 | 17.1 | 1.00E+02 | 1.71E+03 | 0.00 | 0.4 | 99.6 | **4** | 0.28 | 17.1 | 1.00E+03 | 1.71E+04 | 0.005 | 38.2 | 61.8 |
| **5** | 0.1 | 25.7 | 1.00E+02 | 2.57E+03 | 0.00 | 0.6 | 99.4 | **5** | 0.18 | 25.7 | 1.00E+03 | 2.57E+04 | 0.005 | 36.4 | 63.6 |

| **Ceruloplasmin** | | | | | | | | **IgD** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dll** | **total dilut** | **A₄₀₅** x dil | **% rem** | **% depl** |
| **Plasma** | 0.4 | 1 | 1.00E+05 | 1.00E+05 | 0.04 | | | **Plasma** | 0.22 | 1 | 1.00E+05 | 1.00E+05 | 0.02 | | |
| **1** | 0.06 | 6.9 | 1.00E+05 | 6.90E+05 | 0.04 | 100.0 | 0.0 | **1** | 0.28 | 6.9 | 1.00E+04 | 6.90E+04 | 0.02 | 100.0 | 0.0 |
| **2** | 0.08 | 6.9 | 1.00E+04 | 6.90E+04 | 0.01 | 14.3 | 85.7 | **2** | 0.26 | 6.9 | 1.00E+03 | 6.90E+03 | 0.00 | 9.4 | 90.6 |
| **3** | 0.18 | 12 | 1.00E+03 | 1.20E+04 | 0.00 | 5.7 | 94.3 | **3** | 0.15 | 12 | 1.00E+02 | 1.20E+03 | 0.00 | 0.9 | 99.1 |
| **4** | 0.09 | 17.1 | 1.00E+03 | 1.71E+04 | 0.00 | 3.9 | 96.1 | **4** | 0.11 | 17.1 | 1.00E+02 | 1.71E+03 | 0.00 | 0.9 | 99.1 |
| **5** | 0.05 | 25.7 | 1.00E+03 | 2.57E+04 | 0.00 | 3.4 | 96.6 | **5** | 0.06 | 25.7 | 1.00E+02 | 2.57E+03 | 0.00 | 0.8 | 99.2 |

| **ApoA1** | | | | | | | | **C3** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dllut** | **A₄₀₅** x dil | **% rem** | **% depl** |
| **Plasma** | 0.05 | 1 | 1.00E+06 | 1.00E+06 | 0.05 | | | **Plasma** | 0.08 | 1 | 1.00E+06 | 1.00E+06 | 0.08 | | |
| **1** | 0.08 | 6.9 | 1.00E+05 | 6.90E+05 | 0.06 | 100.0 | 0.0 | **1** | 0.11 | 6.9 | 1.00E+05 | 6.90E+05 | 0.07 | 100.0 | 0.0 |
| **2** | 0.29 | 6.9 | 1.00E+04 | 6.90E+04 | 0.02 | 35.6 | 64.4 | **2** | 0.33 | 6.9 | 1.00E+04 | 6.90E+04 | 0.02 | 31.4 | 68.6 |
| **3** | 0.14 | 12 | 1.00E+04 | 1.20E+05 | 0.02 | 28.8 | 71.2 | **3** | 0.16 | 12 | 1.00E+04 | 1.20E+05 | 0.02 | 26.4 | 73.6 |
| **4** | 0.08 | 17.1 | 1.00E+04 | 1.71E+05 | 0.01 | 23.0 | 77.0 | **4** | 0.08 | 17.1 | 1.00E+04 | 1.71E+05 | 0.01 | 19.4 | 80.6 |
| **5** | 0.05 | 25.7 | 1.00E+04 | 2.57E+05 | 0.01 | 20.4 | 79.6 | **5** | 0.04 | 25.7 | 1.00E+04 | 2.57E+05 | 0.01 | 14.8 | 85.2 |

| **C1q** | | | | | | | | **HDL** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.18 | 1 | 1.00E+05 | 1.00E+05 | 0.02 | | | **Plasma** | 0.05 | 1 | 1.00E+06 | 1.00E+06 | 0.05 | | |
| **1** | 0.21 | 6.9 | 1.00E+04 | 6.90E+04 | 0.01 | 100.0 | 0.0 | **1** | 0.12 | 6.9 | 1.00E+05 | 6.90E+05 | 0.08 | 100.0 | 0.0 |
| **2** | 0.23 | 6.9 | 1.00E+03 | 6.90E+03 | 0.00 | 10.8 | 89.2 | **2** | 0.21 | 6.9 | 1.00E+04 | 6.90E+04 | 0.01 | 17.7 | 82.3 |
| **3** | 0.1 | 12 | 1.00E+03 | 1.20E+04 | 0.00 | 8.3 | 91.7 | **3** | 0.14 | 12 | 1.00E+04 | 1.20E+05 | 0.02 | 20.7 | 79.3 |
| **4** | 0.07 | 17.1 | 1.00E+03 | 1.71E+04 | 0.00 | 7.7 | 92.3 | **4** | 0.08 | 17.1 | 1.00E+04 | 1.71E+05 | 0.01 | 16.9 | 83.1 |
| **5** | 0.34 | 25.7 | 1.00E+02 | 2.57E+03 | 0.00 | 6.1 | 93.9 | **5** | 0.05 | 25.7 | 1.00E+04 | 2.57E+05 | 0.01 | 17.2 | 82.8 |

### Example 2: Modified en masse conjugation of antibodies to the top sixteen proteins based on concentration.

This example demonstrates another attempt at the resin optimization process.

Note that the resin(s) generated in this example were made similarly to that described in Example 1. Specifically, activation of the antibodies and solid support was carried out quite similarly to that detailed in Example # 1.

### A. Activation of the Antibodies

Data germane to the activation of the 16 antibodies is shown below in Tables 2-1 through 2-5.

**Table 2-1: Details of antibodies (including concentrations) and activation reagents used to generate the 16-plex resin.**

| **Antibody** | **Conc mg/ml** | **mg needed/ml resin** | **50% increase to account for loss** | **mg for SATA activation per 3 ml** | **Volume used for SATA activation (ml)** |
|---|---|---|---|---|---|
| **Albumin** | 9.9 | 2.8 | 4.2 | 12.6 | 1.27 |
| **IgG** | 9.7 | 0.5 | 0.75 | 2.25 | 0.23 |
| **Total recombinant antibody fragments** | | 3.3 | | 14.85 | 1.50 |
| | | | | | |
| **Transferrin** | 1 | 0.5 | 0.75 | 2.25 | 2.25 |
| **Fibrinogen** | 1.77 | 0.32 | 0.48 | 1.44 | 0.81 |
| **IgA** | 3.4 | 0.35 | 0.525 | 1.58 | 0.46 |
| **alpha-2-Mac** | 1.09 | 0.39 | 0.585 | 1.76 | 1.61 |
| **IgM** | 2.9 | 0.1 | 0.15 | 0.45 | 0.16 |
| **alpha-1-Antitrypsin** | 1.41 | 0.75 | 1.125 | 3.38 | 2.39 |
| **Haptoglobin** | 1.76 | 0.18 | 0.27 | 0.81 | 0.46 |
| **Orosomucoid (Acid-1-Glycoprotein)** | 1 | 0.35 | 0.525 | 1.575 | 1.58 |
| **Ceruloplasmin** | 1 | 0.2 | 0.3 | 0.9 | 0.90 |
| **IgD** | 1 | 0.1 | 0.15 | 0.45 | 0.45 |
| **Apo A1** | 1.55 | 0.25 | 0.375 | 1.125 | 0.73 |
| **C1q** | 1.15 | 0.2 | 0.3 | 0.9 | 0.78 |
| **C3** | 1.5 | 0.35 | 0.525 | 1.575 | 1.05 |
| **HDL** | 2.68 | 0.35 | 0.525 | 1.575 | 0.59 |
| **Total IgG's** | | **4.39** | | **19.76** | **14.22** |

**Table 2-2: Calculations showing total concentrations of antibody prior to and after concentration.**

| | **A₂₈₀** | **A₂₈₀** | **Ave** | **dil** | **A₂₈₀** * dil | **Extinc** | **mg/ml** | **Vol (ml)** | **mg** |
|---|---|---|---|---|---|---|---|---|---|
| **Before conc** | 0.0352 | 0.0368 | 0.036 | 50 | 1.8 | 1.35 | 1.29 | 14.2 | 18.3 |
| **After conc** | 0.2368 | 0.2369 | 0.237 | 50 | 11.8 | 1.35 | 8.46 | 2 | 16.9 |

**Table 2-3: Estimations of activation level based on fluorescamine assay.**

| **Sample** | **0.05 M sodium phosphate, pH 8.2** | **2 mg/ml Fluoresc** | **A₄₈₀** | **A₄₈₀** | **A₄₈₀** | **A₄₈₀** | **Ave** | **% amines remaining** | **% amines coupled** |
|---|---|---|---|---|---|---|---|---|---|
| **Blank** | 1 ml | 10 ml | 2.83 | 3.038 | 4.951 | 4.335 | 3.8 | | |
| **5 µl recombinant antibody fragments** | 1 ml | 10 ml | 10.04 | 8.995 | 25.82 | 24.29 | 17.29 | 100 | 0 |
| **5 µl recombinant antibody fragments - SATA** | 1 ml | 10 ml | 6.335 | 6.96 | 7.547 | 6.904 | 7 | 23 | 77 |
| **Blank** | 1 ml | 10 ml | 0 | | | | 0.0 | | |
| **5 µl IgG antibodies** | 1 ml | 10 ml | 34.71 | 30.2 | 36.41 | 35.94 | 34 | 100 | 0 |
| **5 µl IgG antibodies - SATA** | 1 ml | 10 ml | 2.413 | 2.461 | 25.92 | 27.87 | 15 | 43 | 57 |

**Table 2-4: Estimation of total antibody concentration before and after SATA activation of the small recombinant antibody ligands.**

| **Sample** | **Volume (ml)** | **A₂₈₀** | **A₂₈₀** | **Dilution** | **A₂₈₀** avg with dil | **mg/ml** | **Total mg** |
|---|---|---|---|---|---|---|---|
| **recombinant antibody fragments before SATA** | 1.5 | 0.1647 | 0.1583 | 50 | 8.08 | 9.9 | 14.85 |
| **recombinant antibody fragments after stir cell** | 2 | 0.1336 | 0.1309 | 50 | 6.61 | 8.1 | 16.21 |
| **First flow thru** | 15 | | | 10 | | | |
| **2^{nd} flow thru** | 15 | | | 1 | | | |

**Table 2-5: Estimation of total IgG antibody concentration after SATA activation.**

| | **A₂₈₀** | **A₂₈₀** | **Ave** | **dil** | **A₂₈₀*** dil | **Extinc** | **mg/ml** | **Vol (ml)** | **mg** |
|---|---|---|---|---|---|---|---|---|---|
| **Pool** | 0.0943 | 0.0939 | 0.094 | 50 | 4.7 | 1.4 | 3.36 | 4 | 13.4 |

### B. Coupling of the Antibody to the Solid Support

Data germane to the coupling of the 16 antibodies to the solid support is shown below in Tables 2-6 and 2-7.

**Table 2-6: Reagents and quantities employed to couple antibodies to solid support using maleimide chemistry.**

| **Ab type** | **Ab conc (mg/ml)** | **mg Ab/ml resin** | **ml of resin** | **Vol of Ab (ml)** | **Buffer (0.5M Na P** pH 5.6 (ml) | **Hydroxyamine (ml)** | **Total vol (ml)** |
|---|---|---|---|---|---|---|---|
| **recombinant antibody fragments** | 8.1 | 3.3 | 2.7 | 1.10 | | | |
| **IgG/IgY** | 3.36 | 4.74 | 2.7 | 3.81 | | | |
| **Total** | | 8.04 | 2.7 | 4.91 | 0 | 0.196 | 7.8 |

| **Ab type** | **ml of resin** | **Vol of PBS-EDTA pH 6.7** | **1M Hydroxyamine, pH 6.7 (ml)** | **Total vol (ml)** |
|---|---|---|---|---|
| **Blank** | 0.6 | 1.15 | 0.046 | 1.80 |

**Table 2-7: Protein Concentration bound to resin as determined by BCA Assay.**

| **Sample** | **mg Ab/ml resin** | **Buffer (µl)** | **Sample (µl)** | **BCA reagent (µl)** | **A₅₆₂** | **µg** | **A₅₆₂** | **µg** | **mg/ml (BSA)** | **BSA to ligand mult fact** | **mg protein/ml resin** | **avg mg/ml - bl** | **avg % coupling - bl** |
|---|---|---|---|---|---|---|---|---|---|---|---|---|---|
| **0 std** | | 750 | 0 | 750 | 0 | | | | | | | | |
| 10 std | | 740 | 10 | 750 | 0.3302 | | | | | | | | |
| **20 std** | | 730 | 20 | 750 | 0.6114 | | | | | | | | |
| **40 std** | | 710 | 40 | 750 | 1.0448 | | | | | | | | |
| **60 std** | | 690 | 60 | 750 | 1.5231 | | | | | | | | |
| 100 std | | 650 | 100 | 750 | 2.2978 | | | | | | | | |
| Blank | 0 | 740 | 10 | 750 | 0.2807 | 9.0 | 0.3231 | 10.5 | 2.0 | 0.87 | 1.7 | 0.0 | |
| 2a | 7.28 | 740 | 10 | 750 | 1.4163 | 54.9 | 1.3415 | 51.6 | 10.7 | 0.87 | 9.3 | 7.6 | 104 |
| Antibody -SATA | 4.42 | 740 | 10 | 750 | 1.3355 | 51.3 | 1.3156 | 50.4 | 5.1 | 0.87 | | | |

### C. Plasma Separation

Aliquots of the resin were added to microcolumns as indicated in Table 2-8 below. The volume of 50% slurry is 2x the final volume of resin. Resin for samples 4 and 5 were added in 2x½ volumes due to the column volume restriction.

**Table 2-8: Plasma and wash volumes for each specific resin volume.**

| **Sample #** | **Resin vol (µl)** | **Final vol of 25 µl plasma (µl)** | **Wash vol (2X) (µl)** | **Final vol (µl)** | **Final dil** |
|---|---|---|---|---|---|
| **1** | 200 of blank | 57 | 57 | 171 | 6.9 |
| **2** | 200 | 57 | 57 | 171 | 6.9 |
| **3** | 350 | 100 | 100 | 300 | 12.0 |
| **4** | 500 | 143 | 143 | 429 | 17.1 |
| **Total** | 1050 | | | | |

The snap off bottoms of the columns were removed, the caps loosened and the columns placed into 2 ml collection tubes. The columns were microcentrifuged at 8,000 rpm for 5 sec. The columns were equilibrated with three washes of 1 column volume of PBS at 8,000 rpm for 5 sec. The final spin was carried out for 30 seconds. Plasma (citrated) was diluted with PBS as indicated in the table below (Table 2-9).

**Table 2-9: Plasma dilution conditions for each specific resin volume.**

| **Sample #** | **Resin vol (µl)** | **Final vol of 25 µl plasma (µl)** | **2x vol of plasma (µl)** | **2x vol of PBS (µl)** |
|---|---|---|---|---|
| **1** | 200 of blank | 57 | 50 | 64 |
| **2** | 200 | 57 | 50 | 64 |
| **3** | 350 | 100 | 50 | 150 |
| **4** | 500 | 143 | 50 | 236 |

An aliquot of the diluted plasma was added to each column and incubated for 10 minutes. The columns were then centrifuged at 10,000 rpm for 1 minute. The depleted plasma was reapplied to the column and incubated for 10 minute. Again, the columns were subsequently centrifuged at 10,000 rpm for 1 minute. An aliquot of PBS equal to the diluted plasma volume was added to each column and then centrifuged at 10K rpm for 1 minute. This wash step was repeated. The pooled, depleted plasma was stored at -20°C. The columns were washed 2 times with 1 column volume of PBS with centrifugation at 10,000 rpm for 1 min, to remove any trace amounts of unbound protein. The wash was then discarded. The columns were not stripped. Instead, the columns were washed 2 times with 1 column volume of 3x PBS. Then, one column volume of PBS was added to each of the columns, and they were stored at 4°C.

### D. Analysis

### ELISA Assay (direct) of all 16 proteins:

The separated plasma samples were diluted with serial 10 fold dilutions of coating buffer (100, 1,000, and 10,000 fold; see Table 2-10). The whole serum samples were diluted with serial 10 fold dilutions of coating buffer (100, 1,000, 10,000, 100,000, and 1,000,000 fold; see Table 2-10). Nine plates were coated by adding 0.1 ml of diluted samples to each well. Plates were incubated at 4 °C overnight.

**Table 2-10: Antibody Dilution Table mapped to a 96 well plate format.**

| | **1** | **2** | **3** | **4** | **5(P)** | **6** | **7(1)** | **8(2)** | **9(3)** | **10 (4)** | **11 (P)** | **12** |
|---|---|---|---|---|---|---|---|---|---|---|---|---|
| **A** | E6 | 10000 | 10000 | 10000 | E6 | | E6 | 10000 | 10000 | 10000 | E6 | |
| **B** | E6 | 10000 | 10000 | 10000 | E6 | | E6 | 10000 | 10000 | 10000 | E6 | |
| **C** | E5 | 1000 | 1000 | 1000 | E5 | | E5 | 1000 | 1000 | 1000 | E5 | |
| **D** | E5 | 1000 | 1000 | 1000 | E5 | | E5 | 1000 | 1000 | 1000 | E5 | |
| **E** | E4 | 100 | 100 | 100 | E4 | | E4 | 100 | 100 | 100 | E4 | |
| **F** | E4 | 100 | 100 | 100 | E4 | | E4 | 100 | 100 | 100 | E4 | |
| **G** | 1000 | Blank | | | 1000 | | 1000 | Blank | | | 1000 | |
| **H** | 1000 | Blank | | | 1000 | | 1000 | Blank | | | 1000 | |

After the incubation, the plates were washed 3 times with TBS-T using a platewasher. Next, the plates were blocked with a 0.3 ml TBS, 1% BSA solution. The blocking solution was incubated with the plates for a minimum of 30 minutes at room temperature, and was then aspirated from the wells.

### For the anti-human plasma protein primary antibodies:

Each anti-human plasma protein antibody was diluted (1-20 mg/ml) 1:1,000 in TBS, 1% BSA, and 0.1 ml of the diluted antibody was added to each well. The anti-human antibody was incubated at 37 °C for 90 minutes. The plates were then washed 3 times with TBS-T using a platewasher.

### For the HRP-conjugated secondary antibodies:

The anti chicken and goat HRP conjugates were diluted 1:40,000 in TBS, 1% BSA. Specifically 5 µl of the anti Chicken HRP conjugate was added to 195 µl of TBS-BSA and then 75 µl of this solution added to 75 ml of buffer. Then 5 µl pf Goat HRP conjugate was added to 195 µl of TBS-BSA and 30 µl of this solution added to 30 ml of TBS-BSA. The anti mouse HRP conjugate was diluted 1:50,000. Specifically 5 µl of the anti mouse HRP conjugate was added to 245 µl of TBS-BSA and then 12 µl of this solution was added to 12 ml of TBS-BSA. Next 0.1 ml of each HRP conjugate was added to each well and incubated at 37 °C for 90 minutes. The plates were washed 3 times with TBS-T using a platewasher. Then 0.1 ml of Tetra Methyl Benzidine (TMB) was added (cold, directly out of the refrigerator) to each well and incubated at room temperature for the amount of time indicated in Table 2-11 below. The reactions were stopped by the addition of 0.1 ml 1M HCl. All of the plates were read at 450 nm. Results are detailed below in Table 2-12 and Figure 3.

A depletion level of greater than 95% of each target protein from 25 µl of plasma using 500 µl of resin was the goal for this experiment. Three of the antibodies (C1q, HDL, and acid-1-glycoprotein) depleted less than 95% using 500 µl of resin. The remaining antibodies depleted at a level >95%. The anti-transferrin Ab depleted >95% using 200 µl of resin, therefore, it can be reduced to 0.3 mg/ml for future runs.

**Table 2-11: TMB development times (in minutes) for discrete antibodies.**

| | **AntiTry** | **Cerul** | **A2M** | **Fibrin** | **Hapto** | **Acid 1glyco** | **ApoA1** | **C1q** | **C3** | **HDL** |
|---|---|---|---|---|---|---|---|---|---|---|
| **HRP** | Chick | Chick | Chick | Chick | Chick | Chick | Chick | Chick | Chick | Chick |
| **time** | 2 | 2 | 1.5 | 1.5 | 1.5 | 1.5 | 1 | 7 | 2 | 2 |
| | IgM | Trans | IgA | IgD | | HAS | IgG | | | |
| **HRP** | Goat | Goat | Goat | Goat | | A9044 | N/A | | | |
| **time** | 9 | 9 | 3 | 10 | | 2 | 10 | | | |

**Table 2-12: ELISA data for discrete antibodies.**

| **IgG** | | | | | | | | **IgA** | | | | | | | |
|---|---|---|---|---|---|---|---|---|---|---|---|---|---|---|---|
| **Sample** | **A₄₀₅** | **Depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.08 | 1 | 1.00E+05 | 1.00E+05 | 0.01 | | | **Plasma** | 0.06 | 1 | 1.00E+06 | 1.00E+06 | 0.06 | | |
| **1** | 0.11 | 6.9 | 1.00E+04 | 6.90E+04 | 0.01 | 100.0 | 0.0 | **1** | 0.08 | 6.9 | 1.00E+05 | 6.90E+05 | 0.06 | 100.0 | 0.0 |
| **2** | 0.08 | 6.9 | 1.00E+03 | 6.90E+03 | 0.00 | 7.1 | 92.9 | **2** | 0.11 | 6.9 | 1.00E+04 | 6.90E+04 | 0.01 | 13.8 | 86.3 |
| **3** | 0.07 | 12 | 1.00E+02 | 1.20E+03 | 0.00 | 1.1 | 98.9 | **3** | 0.25 | 12 | 1.00E+03 | 1.20E+04 | 0.00 | 5.4 | 94.6 |
| **4** | 0.05 | 17.1 | 1.00E+02 | 1.71E+03 | 0.00 | 1.1 | 98.9 | **4** | 0.05 | 17.1 | 1.00E+03 | 1.71E+04 | 0.00 | 1.7 | 98.3 |

| **HAS** | | | | | | | | **Fibrinogen** | | | | | | | |
|---|---|---|---|---|---|---|---|---|---|---|---|---|---|---|---|
| **Sample** | **A₄₀₅** | **Depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.11 | 1 | 1.00E+06 | 1.00E+06 | 0.11 | | | **Plasma** | 0.13 | 1 | 1.00E+06 | 1.00E+06 | 0.13 | | |
| **1** | 0.21 | 6.9 | 1.00E+04 | 6.90E+04 | 0.01 | 100.0 | 0.0 | **1** | 0.18 | 6.9 | 1.00E+05 | 6.90E+05 | 0.13 | 100.0 | 0.0 |
| **2** | 0.18 | 6.9 | 1.00E+03 | 6.90E+03 | 0.00 | 8.8 | 91.2 | **2** | 0.12 | 6.9 | 1.00E+04 | 6.90E+04 | 0.01 | 6.4 | 93.6 |
| **3** | 0.19 | 12 | 1.00E+02 | 1.20E+03 | 0.00 | 1.6 | 98.4 | **3** | 0.04 | 12 | 1.00E+04 | 1.20E+05 | 0.01 | 4.2 | 95.8 |
| **4** | 0.06 | 17.1 | 1.00E+02 | 1.71E+03 | 0.00 | 0.7 | 99.3 | **4** | 0.16 | 17.1 | 1.00E+03 | 1.71E+04 | 0.00 | 2.1 | 97.9 |

| **a2M** | | | | | | | | **antitrypsin** | | | | | | | |
|---|---|---|---|---|---|---|---|---|---|---|---|---|---|---|---|
| **Sample** | **A405** | **Depl dil** | **ELISA dil** | **total dilut** | **A405 x dil** | **% rem** | **% depl** | **Sample** | **A405** | **depl dil** | **ELISA dil** | **total dilut** | **A405 x dil** | **% rem** | **% depl** |
| **Plasma** | 0.07 | 1 | 1.00E+06 | 1.00E+06 | 0.07 | | | **Plasma** | 0.07 | 1 | 1.00E+06 | 1.00E+06 | 0.07 | | |
| **1** | 0.1 | 6.9 | 1.00E+05 | 6.90E+05 | 0.07 | 100.0 | 0.0 | **1** | 0.12 | 6.9 | 1.00E+05 | 6.90E+05 | 0.08 | 100.0 | 0.0 |
| **2** | 0.09 | 6.9 | 1.00E+04 | 6.90E+04 | 0.01 | 8.3 | 91.7 | **2** | 0.09 | 6.9 | 1.00E+04 | 6.90E+04 | 0.01 | 7.6 | 92.4 |
| **3** | 0.2 | 12 | 1.00E+03 | 1.20E+04 | 0.00 | 3.3 | 96.7 | **3** | 0.21 | 12 | 1.00E+03 | 1.20E+04 | 0.00 | 3.1 | 96.9 |
| **4** | 0.11 | 17.1 | 1.00E+03 | 1.71E+04 | 0.00 | 2.5 | 97.5 | **4** | 0.06 | 17.1 | 1.00E+03 | 1.71E+04 | 0.00 | 1.3 | 98.7 |

| **IgM** | | | | | | | | **transferrin** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total ditut** | **A₄₀₅** x dil | **% rem** | **% depl** |
| **Plasma** | 0.2 | 1 | 1.00E+05 | 1.00E+05 | 0.02 | | | **Plasma** | 0.11 | 1 | 1.00E+06 | 1.00E+06 | 0.11 | | |
| **1** | 0.24 | 6.9 | 1.00E+04 | 6.90E+04 | 0.02 | 100.0 | 0.0 | **1** | 0.19 | 6.9 | 1.00E+05 | 6.90E+05 | 0.13 | 100.0 | 0.0 |
| **2** | 0.13 | 6.9 | 1.00E+03 | 6.90E+03 | 0.00 | 5.4 | 94.6 | **2** | 0.05 | 6.9 | 1.00E+04 | 6.90E+04 | 0.00 | 2.9 | 97.1 |
| **3** | 0.06 | 12 | 1.00E+03 | 1.20E+04 | 0.00 | 4.4 | 95.6 | **3** | 0.11 | 12 | 1.00E+03 | 1.20E+04 | 0.00 | 1.1 | 98.9 |
| **4** | 0.12 | 17.11 | 1.00E+02 | 1.71E+03 | 0.00 | 1.3 | 98.7 | **4** | 0.14 | 17.11 | 1.00E+02 | 1.71E+03 | 0.00 | 0.2 | 99.8 |

| **Hapto** | | | | | | | | **Acid1glyco** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅ x dil** | **% rem** | **% depl** |
| **Plasma** | 0.12 | 1 | 1.00E+06 | 1.00E+06 | 0.12 | | | **Plasma** | 0.16 | 1 | 1.00E+05 | 1.00E+05 | 0.016 | | |
| **1** | 0.17 | 6.9 | 1.00E+05 | 6.90E+05 | 0.12 | 100.0 | 0.0 | **1** | 0.13 | 6.9 | 1.00E+04 | 6.90E+04 | 0.009 | 100.0 | 0.0 |
| **2** | 0.12 | 6.9 | 1.00E+04 | 6.90E+04 | 0.01 | 7.1 | 92.9 | **2** | 0.25 | 6.9 | 1.00E+03 | 6.90E+03 | 0.002 | 19.0 | 81.0 |
| **3** | 0.23 | 12 | 1.00E+03 | 1.20E+04 | 0.00 | 2.3 | 97.7 | **3** | 0.1 | 12 | 1.00E+03 | 1.20E+04 | 0.001 | 12.4 | 87.6 |
| **4** | 0.1 | 17.1 | 1.00E+03 | 1.71E+04 | 0.00 | 1.4 | 98.6 | **4** | 0.09 | 17.1 | 1.00E+03 | 1.71E+04 | 0.001 | 15.8 | 84.2 |

| **Ceruloplasmin** | | | | | | | | **lgD** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | depl dil | ELISA dil | total dilut | A**₄₀₅** x dil | % rem | % depl |
| **Plasma** | 0.05 | 1 | 1.00E+06 | 1.00E+06 | 0.05 | | | **Plasma** | 0.29 | 1 | 1.00E+05 | 1.00E+05 | 0.03 | | |
| **1** | 0.07 | 6.9 | 1.00E+05 | 6.90E+05 | 0.04 | 100.0 | 0.0 | **1** | 0.32 | 6.9 | 1.00E+04 | 6.90E+04 | 0.02 | 100.0 | 0.0 |
| **2** | 0.33 | 6.9 | 1.00E+03 | 6.90E+03 | 0.00 | 5.0 | 95.0 | **2** | 0.26 | 6.9 | 1.00E+03 | 6.90E+03 | 0.00 | 8.1 | 91.9 |
| **3** | 0.13 | 12 | 1.00E+03 | 1.20E+04 | 0.00 | 3.4 | 96.6 | **3** | 0.06 | 12 | 1.00E+03 | 1.20E+04 | 0.00 | 3.3 | 96.7 |
| **4** | 0.05 | 17.1 | 1.00E+03 | 1.71E+04 | 0.00 | 1.9 | 98.1 | **4** | 0.2 | 17.1 | 1.00E+02 | 1.71E+03 | 0.00 | 1.5 | 98.5 |

| **ApoA1** | | | | | | | | **C3** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | A**₄₀₅** | **depl dil** | **ELISA dil** | **total dilut** | A**₄₀₅ x dil** | **% rem** | **% depl** |
| **Plasma** | 0.19 | 1 | 1.00E+05 | 1.00E+05 | 0.02 | | | **Plasma** | 0.13 | 1 | 1.00E+06 | 1.00E+06 | 0.13 | | |
| **1** | 0.06 | 6.9 | 1.00E+05 | 6.90E+05 | 0.04 | 100.0 | 0.0 | **1** | 0.2 | 6.9 | 1.00E+05 | 6.90E+05 | 0.14 | 100.0 | 0.0 |
| **2** | 0.11 | 6.9 | 1.00E+04 | 6.90E+04 | 0.01 | 16.4 | 83.6 | **2** | 0.17 | 6.9 | 1.00E+04 | 6.90E+04 | 0.01 | 8.3 | 91.7 |
| **3** | 0.17 | 12 | 1.00E+03 | 1.20E+04 | 0.00 | 4.7 | 95.3 | **3** | 0.05 | 12 | 1.00E+04 | 1.20E+05 | 0.01 | 3.9 | 96.1 |
| **4** | 0.09 | 17.1 | 1.00E+03 | 1.71E+04 | 0.00 | 3.6 | 96.4 | **4** | 0.15 | 17.1 | 1.00E+03 | 1.71E+04 | 0.00 | 1.8 | 98.2 |

| **C1q** | | | | | | | | **HDL** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.15 | 1 | 1.00E+05 | 1.00E+05 | 0.01 | | | **Plasma** | 0.25 | 1 | 1.00E+05 | 1.00E+05 | 0.02 | | |
| **1** | 0.18 | 6.9 | 1.00E+04 | 6.90E+04 | 0.01 | 100.0 | 0.0 | **1** | 0.06 | 6.9 | 1.00E+05 | 6.90E+05 | 0.04 | 100.0 | 0.0 |
| **2** | 0.14 | 6.9 | 1.00E+03 | 6.90E+03 | 0.00 | 8.0 | 92.0 | **2** | 0.15 | 6.9 | 1.00E+04 | 6.90E+04 | 0.01 | 24.2 | 75.8 |
| **3** | 0.06 | 12 | 1.00E+03 | 1.20E+04 | 0.00 | 5.7 | 94.3 | **3** | 0.08 | 12 | 1.00E+04 | 1.20E+05 | 0.01 | 22.2 | 77.8 |
| **4** | 0.38 | 17.1 | 1.00E+02 | 1.71E+03 | 0.00 | 5.4 | 94.6 | **4** | 0.13 | 17.1 | 1.00E+03 | 1.71E+04 | 0.00 | 5.3 | 94.7 |

### Example 3: En masse conjugation of antibodies to the top eighteen proteins based on concentration.

This example demonstrates another attempt at the resin optimization process. Note however that this experiment involves the coupling of 18 antibodies to the resin, not the 16 that had been attempted previously in examples 1 and 2.

Note that the resin(s) generated in this example were made similarly to that described in Example 1. Specifically, activation of the antibodies and solid support was carried out quite similarly to that detailed in Example # 1.

### A. Activation of the Antibodies

Data germane to the activation of the 18 antibodies is shown below in Tables 3-1 through 3-5.

**Table 3-1: Details of antibodies (including concentrations) and activation reagents used to generate the 18-plex resin.**

| **Antibody** | **Species** | **Conc mg/ml** | **mg Ab/ml resin (desired goal)** | **50% increase to account for loss** | **mg Ab for SATA activation for 3 ml resin** | **Volume Ab (ml) used for SATA activation** |
|---|---|---|---|---|---|---|
| **Albumin** | Llama | 9.9 | 2.8 | 4.2 | 12.6 | 1.27 |
| **IgG** | Llama | 9.7 | 0.5 | 0.75 | 2.25 | 0.23 |
| **Total recombinant antibody fragments** | | | 3.3 | | 14.85 | 1.50 |
| | | | | | | |
| **Transferrin** | Goat | 1 | 0.5 | 0.75 | 2.25 | 2.25 |
| **Fibrinogen** | Chicken | 1.77 | 0.32 | 0.48 | 1.44 | 0.81 |
| **lgA** | Goat | 3.64 | 0.35 | 0.525 | 1.58 | 0.43 |
| **alpha-2-Mac** | Chicken | 1.09 | 0.39 | 0.585 | 1.76 | 1.61 |
| **IgM** | Goat | 3.06 | 0.1 | 0.15 | 0.45 | 0.15 |
| **alpha-1-Antitrypsin** | Chicken | 1.41 | 0.75 | 1.125 | 3.38 | 2.39 |
| **Haptoglobin** | Rabbit | 9.1 | 0.18 | 0.27 | 0.81 | 0.09 |
| **Orosomucoid (Acid-1-Glycoprotein)** | Chicken | 1 | 0.35 | 0.525 | 1.575 | 1.58 |
| **Ceruloplasmin** | Chicken | 1 | 0.2 | 0.3 | 0.9 | 0.90 |
| **lgD** | Goat | 1 | 0.1 | 0.15 | 0.45 | 0.45 |
| **Apo A1** | Chicken | 1.55 | 0.25 | 0.375 | 1.125 | 0.73 |
| **C1q** | Chicken | 1.15 | 0.2 | 0.3 | 0.9 | 0.78 |
| **C3** | Chicken | 1.5 | 0.2 | 0.3 | 0.9 | 0.60 |
| **HDL** | Chicken | 2.68 | 0.2 | 0.3 | 0.9 | 0.34 |
| **Apo B** | Goat | 0.6 | 0.2 | 0.3 | 0.9 | 1.50 |
| **Prealbumin** | Goat | 2.77 | 0.2 | 0.3 | 0.9 | 0.32 |
| **Total IgG's** | | | **4.49** | | **20.21** | **14.93** |

**Table 3-2: Calculations showing total concentrations of antibody after concentration.**

| | **A₂₈₀** | **A₂₈₀** | **Ave** | **Dilution** | **A₂₈₀*** dilution | **Extinction coefficient** | **mg/ml** | **Vol (ml)** | **mg** |
|---|---|---|---|---|---|---|---|---|---|
| After concentration | 0.2749 | 0.2746 | 0.275 | 50 | 13.7 | 1.4 | 9.81 | 1.75 | 17.2 |

**Table 3-3: Estimations of activation level based on fluorescamine assay.**

| **Sample** | **0.05 M sodium phosphate, pH 8.2** | **2 mg/ml Fluorescein** | **A₄₈₀** | **A₄₈₀** | **A₄₈₀** | **A₄₈₀** | **Avg** | **% amines remaining** | **% amines coupled** |
|---|---|---|---|---|---|---|---|---|---|
| **Blank** | 1 ml | 10 µl | 0 | | | | 0.0 | | |
| **5 µl ecombinant antibody fragments** | 1 ml | 10 µl | 978 | 988 | 996 | 980 | 985.5 | 100 | 0 |
| **5 µl recombinant antibody fragments - SATA** | 1 ml | 10 µl | 149 | 148 | 150 | 147 | 149 | 15 | 85 |
| **Blank** | 1 ml | 10µl | 0 | | | | 0.0 | | |
| **5 µl IgG antibodies** | 1 ml | 10 µl | 762 | 733 | 678 | 713 | 722 | 100 | 0 |
| **5 µl IgG antibodies - SATA** | 1 ml | 10 µl | 466 | 466 | 443 | 440 | 454 | 63 | 37 |

**Table 3-4: Estimation of total antibody concentration before and after SATA activation of the small recombinant antibody ligands.**

| **Sample** | **Volume (ml)** | **A₂₈₀** | **A₂₈₀** | **Dil** | **A₂₈₀** avg with dil | **mg/ml** | **Total mg** |
|---|---|---|---|---|---|---|---|
| **recombinant antibody fragments before SATA** | 1.5 | 0.1661 | 0.1691 | 50 | 8.38 | 9.9 | 14.85 |
| **recombinant antibody fragments Ab after stir cell** | 2 | 0.1107 | 0.111 | 50 | 5.54 | 6.5 | 13.10 |
| **First flow thru** | 15 | 0.2247 | | 10 | 2.25 | | |
| **2^{nd} flow thru** | 15 | 0.4878 | | 1 | 0.49 | | |
| **3rd flow thru** | 15 | 0.0758 | | 1 | 0.08 | | |

**Table 3-5: Estimation of total lgG antibody concentration after SATA activation.**

| | **A₂₈₀** | **A₂₈₀** | **Ave** | **dil** | **A₂₈₀** * dil | **Extinc** | **mg/ml** | **Vol (ml)** | **mg** |
|---|---|---|---|---|---|---|---|---|---|
| Pool (Fr 5-6) | 0.1027 | 0.0976 | 0.100 | 50 | 5.0 | 1.4 | 3.58 | 4 | 14.3 |

### B. Coupling of the Antibody to the Solid Support

Data germane to the coupling of the 18 antibodies to the solid support is shown below in Tables 3-6 and 3-7.

**Table 3-6: Reagents and quantities employed to couple antibodies to solid support using maleimide chemistry.**

| **Ab type** | **Ab conc (mg/ml)** | **mg Ab/ml resin** | **ml of Resin** | **Vol of Ab (ml)** | **Buffer (0.5M Na P pH 5.6** | **Hydroxyamine (ml)** | **Total vol (ml)** |
|---|---|---|---|---|---|---|---|
| **recombinant antibody fragments** | 6.5 | 3.3 | 3 | 1.52 | | | |
| **IgG/lgY** | 3.58 | 4.49 | 3 | 3.76 | | | |
| **Total** | | 7.79 | 3 | 5.29 | 0 | 0.213 | 8.5 |

| **Ab type** | **ml of resin** | **Vol of PBS- EDTA pH 6.7** | **1 M Hydroxyamine, pH 6.7 (ml)** | **Total vol (ml)** |
|---|---|---|---|---|
| **Blank** | 0.6 | 1.15 | 0.046 | 1.80 |

**Table 3-7: Protein Concentration bound to resin as determined by BCA Assay.**

| **Sample** | **mg Ablml resin** | **Buffer (µl)** | **Sample (µl)** | **BCA reagent (µl)** | **A₅₆₂** | **µg** | **A₅₆₂** | **µg** | **mg/ml (BSA)** | **BSA to ligand mult fact.** | **mg protein / ml resin** | **avg mg/ml - blank** | **avg % coupling - blank** |
|---|---|---|---|---|---|---|---|---|---|---|---|---|---|
| **0 std** | | 750 | 0 | 750 | 0 | | | | | | | | |
| **10 std** | | 740 | 10 | 750 | 0.3132 | | | | | | | | |
| **20 std** | | 730 | 20 | 750 | 0.5669 | | | | | | | | |
| **40 std** | | 710 | 40 | 750 | 0.9855 | | | | | | | | |
| **60 std** | | 690 | 60 | 750 | 1.3702 | | | | | | | | |
| **100 std** | | 650 | 100 | 750 | 2.1487 | | | | | | | | |
| **Blank** | 0 | 740 | 10 | 750 | 0.2839 | 9.8 | 0.3006 | 10.5 | 2.0 | 0.74 | 1.5 | 0.0 | |
| **2a** | 7.79 | 740 | 10 | 750 | 1.1869 | 49.4 | 1.0864 | 44.7 | 9.4 | 0.74 | 6.9 | 5.4 | 70 |
| **antibody - SATA** | 4.41 | 740 | 10 | 750 | 1.3933 | 59.4 | 1.4066 | 60.1 | 6.0 | 0.74 | | | |

### C. Plasma Separation

Aliquots of the resin were added to microcolumns as indicated in Table 3-8, below. The volume of 50% slurry is 2x the final volume of resin. Resin for samples 4 and 5 was added in 2x½ volumes due to the column volume restriction.

**Table 3-8: Plasma and wash volumes for each specific resin volume.**

| **Sample #** | **Resin vol (µl)** | **Final vol of 25 µl Plasma (µl)** | **Wash vol (2X) (µl)** | **Final vol (µl)** | **Final dil** |
|---|---|---|---|---|---|
| **1** | 200 blank | 57 | 57 | 171 | 6.9 |
| **2** | 200 | 57 | 57 | 171 | 6.9 |
| **3** | 350 | 100 | 100 | 300 | 12.0 |
| **4** | 500 | 143 | 143 | 429 | 17.1 |
| **Total** | **1050** | | | | |

The snap off bottoms of the columns were removed, the caps loosened and the columns placed into 2 ml collection tubes. The columns were microcentrifuged at 8,000 rpm for 5 seconds. The columns were equilibrated with three washes of 1 column volume of PBS at 8,000 rpm for 5 seconds. The final spin was carried out for 30 seconds. Plasma (citrated) was diluted with PBS as indicated in the table below (Table 3-9).

**Table 3-9: Plasma dilution conditions for each specific resin volume.**

| **Sample #** | **Resin vol (µl)** | **Final vol of 25 µl Plasma (µl)** | **2x vol of plasma (µl)** | **2x vol of PBS (µl)** |
|---|---|---|---|---|
| **1** | 200 blank | 57 | 50 | 64 |
| **2** | 200 | 57 | 50 | 64 |
| **3** | 350 | 100 | 50 | 150 |
| **4** | 500 | 143 | 50 | 236 |

An aliquot of the diluted plasma was added to each column and incubated for 10 minutes. The columns were then centrifuged at 10,000 rpm for 1 minute. The depleted plasma was reapplied to the column and incubated for 10 minutes. Again, the columns were subsequently centrifuged at 10,000 rpm for 1 minute. An aliquot of PBS equal to the diluted plasma volume was added to each column and then centrifuged at 10,000 rpm for 1 minute. This wash step was repeated. The pooled, depleted plasma was stored at -20 °C. The columns were washed 2 times with 1 column volume of PBS with centrifugation at 10,000 rpm for 1 minute, to remove any trace amounts of unbound protein.

### D. Analysis

### ELISA Assay (direct) of all 18 proteins:

The separated serum samples were diluted with serial 10 fold dilutions of coating buffer (100, 1,000, and 10,000 fold; see Table 3-10). The whole serum samples were diluted with serial 10 fold dilutions of coating buffer (100, 1,000, 10,000, 100,000, and 1,000,000 fold; see Table 3-10). Ten plates were coated by adding 0.1 ml of diluted samples to each well. Plates were incubated at 4 °C overnight.

**Table 3-10: Antibody Dilution Table mapped to a 96 well plate format.**

| | **1** | **2** | **3** | **4** | **5 (P)** | **6** | **7(1)** | **8 (2)** | **9(3)** | **10(4)** | **11 (P)** | **1 2** |
|---|---|---|---|---|---|---|---|---|---|---|---|---|
| **A** | E6 | 10000 | 10000 | 10000 | E6 | | E6 | 10000 | 10000 | 10000 | E6 | |
| **B** | E6 | 10000 | 10000 | 10000 | E6 | | E6 | 10000 | 10000 | 10000 | E6 | |
| **C** | E5 | 1000 | 1000 | 1000 | E5 | | E5 | 1000 | 1000 | 1000 | E5 | |
| **D** | E5 | 1000 | 1000 | 1000 | E5 | | E5 | 1000 | 1000 | 1000 | E5 | |
| **E** | E4 | 100 | 100 | 100 | E4 | | E4 | 100 | 100 | 100 | E4 | |
| **F** | E4 | 100 | 100 | 100 | E4 | | E4 | 100 | 100 | 100 | E4 | |
| **G** | 1000 | BI | | | 1000 | | 1000 | BI | | | 1000 | |
| **H** | 1000 | BI | | | 1000 | | 1000 | Bl | | | 1000 | |

After the incubation, the plates were washed 3 times with TBS-T using a platewasher. Next, the plates were blocked with a 0.3 ml of TBS, 1% BSA solution. The blocking solution was incubated with the plates for a minimum of 30 minutes at room temp, and was then aspirated from the wells.

### For the anti-human plasma protein primary antibodies:

Each anti-human antibody was diluted (1-20 mg/ml) 1:1,000 in TBS, 1% BSA, and 0.1 ml of the diluted antibody was added to each well. The anti-human antibody was incubated at 37°C for 90 minutes. The plates were then washed 3 times with TBS-T using a platewasher.

### For the HRP-conjugated secondary antibodies:

The anti chicken and goat HRP conjugates were diluted 1:40,000 in TBS, 1% BSA. Specifically 5 µl of the anti Chicken HRP conjugate was added to 195 µl of TBS-BSA and then 50 µl of this solution was added to 50 ml of buffer. Next 5 µl Goat HRP conjugate was added to 195 µl of TBS-BSA and then 40 µl of this solution was added to 40 ml of TBS-BSA. The anti mouse HRP conjugate was diluted 1:50,000. Specifically 5 µl of the anti mouse HRP conjugate was added to 245 µl of TBS-BSA and then 12 µl of this solution was added to 12 ml of TBS-BSA. The anti rabbit HRP conjugate was diluted 1:40,000. Specifically 5 µl of the anti rabbit HRP conjugate was added to 195 µl TBS-BSA and then 12 µl of this solution was added to 12 ml of TBS-BSA. Then 0.1 ml of each HRP conjugate was added to each well and incubated at 37 °C for 90 minutes. The plates were washed 3 times with TBS-T using a platewasher. Next 0.1 ml of TMB was added (cold, directly out of the refrigerator) to each well and incubated at room temperature for the amount of time indicated in Table 3-11 below. The reactions were stopped by the addition of 0.1 ml of 1 M HCl. All plates were read at 450 nm. Results were as follows in Table 3-12 and Figure 4.

A depletion level of greater than 95% of each target protein from 25 µl of plasma using 500 µl of resin was the goal for this experiment. The Acid-1-glycoprotein achieved 83% depletion using 500 µl resin. The ApoA1 achieved 91% depletion using 500 µl resin. The C1q achieved 94% depletion using 500 µl resin. The C3 achieved 95% depletion using 500 µl resin. The remaining antibodies depleted at greater than 95% using 500 µl resin. The prealbumin and transferrin antibodies depleted at greater than 98% using 350 µl resin. The relative concentrations of ApoA1, C1q and C3 antibodies will be increased for the next experiment. The concentrations of prealbumin and transferrin antibodies will be decreased for the next experiment.

**Table 3-11: TMB development times (in minutes) for discrete antibodies.**

| | **AntiTry** | **Cerul** | **A2M** | **Fibrin** | **ApoA1** | **C1q** | **C3** | **HDL** | **Acid 1glyco** | |
|---|---|---|---|---|---|---|---|---|---|---|
| **HRP** | Chick | Chick | Chick | Chick | Chick | Chick | Chick | Chick | Chick | |
| **Time** | 4 | 7 | 4.5 | 4.5 | 1.5 | 10 | 2.5 | 1 | 10 | |
| | lgM | Trans | lgA | lgD | ApoB | Prealb | | Hapto | HAS | lgG |
| **HRP** | Goat | Goat | Goat | Goat | Goat | Goat | | Rabbit | A9044 | N/A |
| **Time** | 6 | 4 | 7.5 | 12 | 9 | 9 | | 8 | 5.5 | 2 |

**Table 3-12 : ELISA data for discrete antibodies.**

| **lgG** | | | | | | | | **lgA** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.06 | 1 | 1.00E+06 | 1.00E+06 | 0.06 | | | **Plasma** | 0.12 | 1 | 1.00E+06 | 1.00E+06 | 0.12 | | |
| **1** | 0.08 | 6.9 | 1.00E+05 | 6.90E+05 | 0.05 | 100.0 | 0.0 | **1** | 0.16 | 6.9 | 1.00E+05 | 6.90E+05 | 0.11 | 100.0 | 0.0 |
| **2** | 0.08 | 6.9 | 1.00E+04 | 6.90E+04 | 0.01 | 10.4 | 89.6 | **2** | 0.56 | 6.9 | 1.00E+04 | 6.90E+04 | 0.04 | 34.5 | 65.5 |
| **3** | 0.13 | 12 | 1.00E+03 | 1.20E+04 | 0.00 | 2.8 | 97.2 | **3** | 0.14 | 12 | 1.00E+04 | 1.20E+05 | 0.02 | 15.3 | 84.7 |
| **4** | 0.05 | 17.1 | 1.00E+03 | 1.71E+04 | 0.00 | 1.6 | 98.4 | **4** | 0.22 | 17.1 | 1.00E+03 | 1.71E+04 | 0.00 | 3.4 | 96.6 |

| **HAS** | | | | | | | | **Fibrinogen** | | | | | | | |
|---|---|---|---|---|---|---|---|---|---|---|---|---|---|---|---|
| **Sample** | **A**_{**40**5} | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.1 | 1 | 1.00E+06 | 1.00E+06 | 0.10 | | | **Plasma** | 0.34 | 1 | 1.00E+06 | 1.00E+06 | 0.34 | | |
| **1** | 0.09 | 6.9 | 1.00E+05 | 6.90E+05 | 0.06 | 100.0 | 0.0 | **1** | 0.42 | 6.9 | 1.00E+05 | 6.90E+05 | 0.29 | 100.0 | 0.0 |
| **2** | 0.17 | 6.9 | 1.00E+04 | 6.90E+04 | 0.01 | 19.3 | 80.7 | **2** | 0.44 | 6.9 | 1.00E+04 | 6.90E+04 | 0.03 | 10.5 | 89.5 |
| **3** | 0.21 | 12 | 1.00E+03 | 1.20E+04 | 0.00 | 4.1 | 95.9 | **3** | 0.08 | 12 | 1.00E+04 | 1.20E+05 | 0.01 | 3.2 | 96.8 |
| **4** | 0.05 | 17.1 | 1.00E+03 | 1.71E+04 | 0.00 | 1.4 | 98.6 | **4** | 0.45 | 17.1 | 1.00E+03 | 1.71E+04 | 0.01 | 2.7 | 97.3 |

| **a2M** | | | | | | | | **antitrypsin** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.15 | 1 | 1.00E+06 | 1.00E+06 | 0.15 | | | **Plasma** | 0.15 | 1 | 1.00E+06 | 1.00E+06 | 0.15 | | |
| **1** | 0.25 | 6.9 | 1.00E+05 | 6.90E+05 | 0.17 | 100.0 | 0.0 | **1** | 0.19 | 6.9 | 1.00E+05 | 6.90E+05 | 0.13 | 100.0 | 0.0 |
| **2** | 0.3 | 6.9 | 1.00E+04 | 6.90E+04 | 0.02 | 12.0 | 88.0 | **2** | 0.36 | 6.9 | 1.00E+04 | 6.90E+04 | 0.02 | 19.3 | 80.7 |
| **3** | 0.05 | 12 | 1.00E+04 | 1.20E+05 | 0.01 | 3.4 | 96.6 | **3** | 0.06 | 12 | 1.00E+04 | 1.20E+05 | 0.01 | 5.9 | 94.1 |
| **4** | 0.25 | 17.1 | 1.00E+03 | 1.71E+04 | 0.00 | 2.5 | 97.5 | **4** | 0.17 | 17.1 | 1.00E+03 | 1.71E+04 | 0.00 | 2.2 | 97.8 |

| **IgM** | | | | | | | | **transferrin** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.12 | 1 | 1.00E+05 | 1.00E+05 | 0.01 | | | **Plasma** | 0.06 | 1 | 1.00E+06 | 1.00E+06 | 0.06 | | |
| **1** | 0.18 | 6.9 | 1.00E+04 | 6.90E+04 | 0.01 | 100.0 | 0.0 | **1** | 0.07 | 6.9 | 1.00E+05 | 6.90E+05 | 0.05 | 100.0 | 0.0 |
| **2** | 0.05 | 6.9 | 1.00E+04 | 6.90E+04 | 0.00 | 26.4 | 73.6 | **2** | 0.08 | 6.9 | 1.00E+04 | 6.90E+04 | 0.01 | 10.7 | 89.3 |
| **3** | 0.03 | 12 | 1.00E+03 | 1.20E+04 | 0.00 | 2.9 | 97.1 | **3** | 0.12 | 12 | 1.00E+02 | 1.20E+03 | 0.00 | 0.3 | 99.7 |
| **4** | 0.12 | 17.1 | 1.00E+02 | 1.71E+03 | 0.00 | 1.6 | 98.4 | **4** | 0.08 | 17.1 | 1.00E+02 | 1.71E+03 | 0.00 | 0.3 | 99.7 |

| **Hapto** | | | | | | | | **Acid1glyco** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅ x dil** | **% rem** | **% depl** | **Sample** | **A₄₀₅** | **depl dil** | **ELISA dil** | **total dilut** | **A₄₀₅ x dil** | **% rem** | **% depl** |
| **Plasma** | 0.1 | 1 | 1.00E+06 | 1.00E+06 | 0.10 | | | **Plasma** | 0.22 | 1 | 1.00E+05 | 1.00E+05 | 0.022 | | |
| **1** | 0.12 | 6.9 | 1.00E+05 | 6.90E+05 | 0.08 | 100.0 | 0.0 | **1** | 0.45 | 6.9 | 1.00E+04 | 6.90E+04 | 0.031 | 100.0 | 0.0 |
| **2** | 0.13 | 6.9 | 1.00E+04 | 6.90E+04 | 0.01 | 10.7 | 89.3 | **2** | 0.16 | 6.9 | 1.00E+04 | 6.90E+04 | 0.011 | 35.4 | 64.6 |
| **3** | 0.21 | 12 | 1.00E+03 | 1.20E+04 | 0.00 | 2.9 | 97.1 | **3** | 0.63 | 12 | 1.00E+03 | 1.20E+04 | 0.008 | 24.6 | 75.4 |
| **4** | 0.07 | 17.1 | 1.00E+03 | 1.71E+04 | 0.00 | 1.5 | 98.5 | **4** | 0.31 | 17.1 | 1.00E+03 | 1.71E+04 | 0.005 | 17.2 | 82.8 |

| **Ceruloplasmin** | | | | | | | | **IgD** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.06 | 1 | 1.00E+06 | 1.00E+06 | 0.06 | | | **Plasma** | 0.26 | 1 | 1.00E+05 | 1.00E+05 | 0.03 | | |
| **1** | 0.12 | 6.9 | 1.00E+05 | 6.90E+05 | 0.08 | 100.0 | 0.0 | **1** | 0.35 | 6.9 | 1.00E+04 | 6.90E+04 | 0.02 | 100.0 | 0.0 |
| **2** | 0.16 | 6.9 | 1.00E+04 | 6.90E+04 | 0.01 | 13.7 | 86.3 | **2** | 0.06 | 6.9 | 1.00E+04 | 6.90E+04 | 0.00 | 18.4 | 81.6 |
| **3** | 0.32 | 12 | 1.00E+03 | 1.20E+04 | 0.00 | 4.8 | 95.2 | **3** | 0.06 | 12 | 1.00E+03 | 1.20E+04 | 0.00 | 2.7 | 97.3 |
| **4** | 0.16 | 17.1 | 1.00E+03 | 1.71E+04 | 0.00 | 3.4 | 96.6 | **4** | 0.24 | 17.1 | 1.00E+02 | 1.71E+03 | 0.00 | 1.7 | 98.3 |

| **ApoA1** | | | | | | | | **C3** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.07 | 1 | 1.00E+06 | 1.00E+06 | 0.07 | | | **Plasma** | 0.1 | 1 | 1.00E+06 | 1.00E+06 | 0.10 | | |
| **1** | 0.09 | 6.9 | 1.00E+05 | 6.90E+05 | 0.06 | 100.0 | 0.0 | **1** | 0.16 | 6.9 | 1.00E+05 | 6.90E+05 | 0.11 | 100.0 | 0.0 |
| **2** | 0.29 | 6.9 | 1.00E+04 | 6.90E+04 | 0.02 | 31.3 | 68.7 | **2** | 0.4 | 6.9 | 1.00E+04 | 6.90E+04 | 0.03 | 25.3 | 74.8 |
| **3** | 0.12 | 12 | 1.00E+04 | 1.20E+05 | 0.01 | 22.2 | 77.8 | **3** | 0.08 | 12 | 1.00E+04 | 1.20E+05 | 0.01 | 8.2 | 91.8 |
| **4** | 0.35 | 17.1 | 1.00E+03 | 1.71E+04 | 0.01 | 9.5 | 90.5 | 4 | 0.32 | 17.1 | 1.00E+03 | 1.71E+04 | 0.01 | 4.9 | 95.1 |

| **C1q** | | | | | | | | **HDL** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.14 | 1 | 1.00E+05 | 1.00E+05 | 0.01 | | | **Plasma** | | 1 | 1.00E+05 | 1.00E+05 | 0.00 | | |
| **1** | 0.22 | 6.9 | 1.00E+04 | 6.90E+04 | 0.02 | 100.0 | 0.0 | **1** | | 6.9 | 1.00E+05 | 6.90E+05 | 0.00 | #### | ##### |
| **2** | 0.24 | 6.9 | 1.00E+03 | 6.90E+03 | 0.00 | 10.7 | 89.3 | **2** | | 6.9 | 1.00E+04 | 6.90E+04 | 0.00 | #### | ##### |
| **3** | 0.07 | 12 | 1.00E+03 | 1.20E+04 | 0.00 | 5.2 | 94.8 | **3** | | 12 | 1.00E+04 | 1.20E+05 | 0.00 | #### | ##### |
| **4** | 0.52 | 17.1 | 1.00E+02 | 1.71E+03 | 0.00 | 5.8 | 94.2 | **4** | | 17.1 | 1.00E+03 | 1.71E+04 | 0.00 | #### | ##### |
| **Prealbumin** | | | | | | | | **ApoB** | | | | | | | |

| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
|---|---|---|---|---|---|---|---|---|---|---|---|---|---|---|---|
| **Plasma** | 0.17 | 1 | 1.00E+05 | 1.00E+05 | 0.02 | | | **Plasma** | 0.19 | 1 | 1.00E+05 | 1.00E+05 | 0.02 | | |
| **1** | 0.24 | 6.9 | 1.00E+04 | 6.90E+04 | 0.02 | 100.0 | 0.0 | **1** | 0.31 | 6.9 | 1.00E+04 | 6.90E+04 | 0.02 | 100.0 | 0.0 |
| **2** | 0.05 | 6.9 | 1.00E+04 | 6.90E+04 | 0.00 | 21.4 | 78.6 | **2** | 0.07 | 6.9 | 1.00E+04 | 6.90E+04 | 0.00 | 21.6 | 78.4 |
| **3** | 0.07 | 12 | 1.00E+02 | 1.20E+03 | 0.00 | 0.5 | 99.5 | **3** | 0.1 | 12 | 1.00E+03 | 1.20E+04 | 0.00 | 5.6 | 94.4 |
| **4** | 0.06 | 17.1 | 1.00E+02 | 1.71E+03 | 0.00 | 0.6 | 99.4 | **4** | 0.04 | 17.1 | 1.00E+03 | 1.71E+04 | 0.00 | 3.3 | 96.7 |

### EXAMPLE 4: En masse conjugation of antibodies to the top twenty proteins based on concentration.

This example demonstrates another attempt at the resin optimization process.

Note that the resin(s) generated in this example were made using both the maleimide (as per Examples 1 through 3 above) and bromoacetic acid NHS ester chemistry for comparison purposes.

Also note that this experiment involves the coupling of 20 antibodies to the resin, not the 16 or 18 that had been previously attempted in examples 1 through 3.

### A. Activation of the Antibodies

The A₂₈₀ of the antibodies were read to determine concentration. An extinction coefficient of 1.4 was used. The amount of antibody needed for SATA activation is shown in Table 4-1. Three milliliters of resin were coupled.

The 18 lgG antibodies were pooled and concentrated in four YM-50 Centricons at 5,000 x g to obtain a final volume of 3 ml. The A₂₈₀ was measured again in duplicate to determine the final concentration of the pooled lgG antibodies (Table 4-2). The mixture of concentrated antibodies was adjusted to a final buffer concentration of 50 mM Na phosphate, 0.5 M NaCl, and 1mM EDTA.

The recombinant antibody fragments were combined in a separate tube, using the volumes shown in Table 4-1.

**Table 4-1: Details of antibodies (including concentrations) and activation reagents used to generate the 20-plex resin**

| **Antibody** | **Species** | **Conc mg/ml** | **mg needed per ml resin** | **50% increase to account for loss** | **mg for SATA activation for 4 ml** | **Volume used for SATA activation (ml)** |
|---|---|---|---|---|---|---|
| **Albumin** | Llama | 9.9 | 2.8 | 4.2 | 16.8 | 1.70 |
| **lgG** | Llama | 9.7 | 0.5 | 0.75 | 3 | 0.31 |
| **Total recombinant antibody fragments** | | | 3.3 | | 19.8 | 2.01 |
| **Transferrin** | Chicken | 2.04 | 0.5 | 0.75 | 3 | 1.47 |
| **Fibrinogen** | Chicken | 1.37 | 0.32 | 0.48 | 1.92 | 1.40 |
| **IgA** | Goat | 3.89 | 0.35 | 0.525 | 2.1 | 0.54 |
| **alpha-2-Mac** | Chicken | 1.69 | 0.39 | 0.585 | 2.34 | 1.38 |
| **Igm** | Goat | 3.05 | 0.1 | 0.15 | 0.6 | 0.20 |
| **alpha-1-Antitrypsin** | Chicken | 2.13 | 0.5 | 0.75 | 3 | 1.41 |
| **Haptoglobin** | Rabbit | 9.1 | 0.18 | 0.27 | 1.08 | 0.12 |
| **Acid-1-glycoprotein** | Goat | 1.7 | 0.25 | 0.375 | 1.5 | 0.88 |
| **Ceruloplasmin** | Goat | 1.13 | 0.2 | 0.3 | 1.2 | 1.06 |
| **IgD** | Goat | 1 | 0.1 | 0.15 | 0.6 | 0.60 |
| **Apo A1** | Chicken | 1.43 | 0.35 | 0.525 | 2.1 | 1.47 |
| **C1q** | Chicken | 1.15 | 0.3 | 0.45 | 1.8 | 1.57 |
| **C3** | Chicken | 1.5 | 0.3 | 0.45 | 1.8 | 1.20 |
| **HDL** | Chicken | 2.68 | 0.5 | 0.75 | 3 | 1.12 |
| **Apo B** | Goat | 0.6 | 0.2 | 0.3 | 1.2 | 2.00 |
| **Plasminogen** | Goat | 4.06 | 0.2 | 0.3 | 1.2 | 0.30 |
| **C4 Prealbumin** | Chicken | 0.83 | 0.2 | 0.3 | 1.2 | 1.45 |
| | Goat | 2.9 | 0.15 | 0.225 | 0.9 | 0.31 |
| **Total IgG's** | | | **5.09** | | **30.54** | **18.47** |

**Table 4-2: Calculations showing total concentrations of antibody after concentration.**

| | **A₂₈₀** | **A₂₈₀** | **Ave** | **Dil** | **A₂₈₀** * dil | **Extinc** | **mg/ml** | **Vol (ml)** | **mg** |
|---|---|---|---|---|---|---|---|---|---|
| **After conc** | 0.1976 | 0.1984 | 0.198 | 50 | 9.9 | 1.4 | 7.07143 | 3.15 | 22.275 |

The amount of SATA required for the activation of the antibodies was calculated with an 8 (for the recombinant antibody fragments) or 40 (for the IgG antibodies) molar excess of SATA:
recombinant antibody fragments
   ➢ 12,000 mg/mmole = 0.0000833 mmole/mg
   ➢ 0.0000833 mmole/mg x 8 = 0.00083 mmoles SATA/mg Ab
   ➢ 0.00083 mmole/mg Ab / 0.25 mmoles/ml SATA = 2.6 µl of 0.25 M SATA/mg Ab
IgG antibodies
   ➢ 150,000 mg/mmole Ab = 0.0000067 mmole/mg Ab
   ➢ 0.0000067 mmole/mg Ab x 40 = 0.000267 mmole SATA/mg Ab
   ➢ 0.000267 mmole/mg Ab / 0.25 mmoles/ml SATA = 1.07 µl of 0.25 M SATA/mg Ab

Fresh SATA (Sigma, Product Code A 9043) was prepared with dimethylformamide (DMF) to a concentration of 0.25 M (58 mg / ml; 13.5 mg / 230 µl DMF). A sample (25 µl) of the antibody mixtures was removed for later assays. Then an 8 molar excess of SATA (51.5 µl of 0.25 M) was added to the recombinant antibody fragments and 40 molar excess of SATA (24.3 µl of 0.25 M) was added to the other antibodies. The solutions were mixed and allowed to react for 1 hour at room temperature.

Afterwards, the percent of remaining amines was measured by carrying out a fluorescamine assay. A 2 mg / ml fluorescamine solution was prepared in DMSO. The reagents were combined, as per Table 4-3 below, into individual 2 ml amber tubes and gently mixed. The plate reader was recalibrated, and two 0.2 ml aliquots from each tube were placed into duplicate wells of a fluorescence plate (Corning plate; Product Code 3916). The plate was read on a fluorescence plate reader at an excitation wavelength of 390 nm and an emission wavelength of 480 nm. The percent amines remaining was calculated using the following equation: (fluorescence of SATA activated Ab - blank fluorescence) / (fluorescence of Ab solution - blank fluorescence) x 100 = % amines (Table 4-3). Because of the low percent of coupled amines, 12 µl of additional SATA was added to the IgG reaction, and the mixture was incubated for a second time.

**Table 4-3: Estimations of activation level based on fluorescamine assay.**

| **Sample** | **0.05 M sodium phosphate, pH 8.2** | **2 mg/ml Fluoresc** | **A₄₈₀** | **A₄₈₀** | **A₄₈₀** | **A₄₈₀** | **Ave** | **% amines remaining** | **% amines coupled** |
|---|---|---|---|---|---|---|---|---|---|
| **Blank** | 1 ml | 10 ml | 0 | | | | 0.0 | | |
| **5 µl recombinant antibody fragments** | 1 ml | 10 ml | 593 | 554 | 639 | 562 | 587 | 100 | 0 |
| **5 µl recombinant antibody fragments - SATA** | 1 ml | 10 ml | 88 | 86 | 99 | 90 | 91 | 15 | 85 |
| **Blank** | 1 ml | 10 ml | 0 | | | | 0.0 | | |
| **5 µl IgG antibodies** | 1 ml | 10 ml | 361 | 311 | 375 | 323 | 343 | 100 | 0 |
| **5 µl IgG antibodies - SATA** | 1 ml | 10 ml | 208 | 216 | 272 | 268 | 241 | 70 | 30 |

After SATA activation, the recombinant antibody fragment mix was diluted 10 fold (15 ml) with PBS-EDTA, pH 6.7, and concentrated to the original volume on a stir cell with a 3,000 MW cut off membrane. The recombinant antibody fragment mix was again diluted 10 fold with PBS-EDTA, pH 6.7 (15 ml) and concentrated to approximately 60% of the original volume. The A₂₈₀ of the Ab mix prior to SATA activation and the concentrate after activation was measured in duplicate by diluting 20 fold in PBS-EDTA, pH 6.7 (Table 4-4).

**Table 4-4: Estimation of total antibody concentration before and after SATA activation of the small recombinant antibody ligands.**

| **Sample** | **Volume (ml)** | **A₂₈₀** | **A₂₈₀** | **Dil** | **A₂₈₀** ave with dil | **mg/ml** | **Total mg** |
|---|---|---|---|---|---|---|---|
| **recombinant antibody fragments before SATA** | 2 | 0.1634 | 0.1652 | 50 | 8.22 | 9.9 | 19.8 |
| **recombinant antibody fragments after stir cell** | 2 | 0.1531 | 0.1545 | 50 | 7.69 | 9.3 | 18.53 |
| **First flow thru** | 15 | | | 10 | | | |
| **2^{nd} flow thru** | 15 | | | 1 | | | |
| **3rd flow thru** | 15 | | | 1 | | | |

After SATA activation, the lgG antibody mix was gel filtered. A 20 ml column of Sephadex G25-80 was poured and equilibrated with 3 column volumes of PBS-EDTA pH 6.7. The SATA activated IgG antibodies were added to the top of the column and the column eluted with PBS-EDTA; 2 ml fractions were collected. The A₂₈₀ value of the fractions was read after diluting a 20 µl sample to 1 ml with PBS-EDTA. Fractions containing antibody were pooled. The A₂₈₀ value of the pooled fractions was measured in duplicate (Table 4-5). Samples were diluted 1:50 in PBS-EDTA; an extinction coefficient of 1.4 was used.

**Table 4-5: Estimation of total lgG antibody concentration after SATA activation.**

| | **A₂₈₀** | **A₂₈₀** | **Ave** | **dil** | **A₂₈₀** * dil | **Extinc** | **mg/ml** | **Vol (ml)** | **mg** |
|---|---|---|---|---|---|---|---|---|---|
| **Pool (Fr6-7)** | 0.1184 | 0.1186 | 0.1185 | 50 | 5.925 | 1.4 | 4.23 | 3.6 | 15.2 |

### B. Activation of the Solid Support

### Epoxy activation of resin

Fifty ml of Sepharose 6B was washed with 5 volumes of deionized water and filtered to a damp cake. The gel was transferred to an appropriate size poly container. The following reagents were added to the reaction vessel with mixing: 0.05 L of 1,4-butanediol diglycidyl ether and 0.05 L of 0.6 M NaOH. The reaction was allowed to proceed for 3-4 hours, while mixing on the orbital mixer at 3.5 rpm. When the reaction was complete (≥ 20 µmoles / ml of resin or incubation for 4 hours), the solution was neutralized by adding 974 ml of 1 M potassium phosphate monobasic per liter of resin. The total potassium phosphate added was 48.7 ml. Ethanol (to a final partial volume of 25%) was added to the solution, to help 1,4-butanediol diglycidyl ether become more miscible. The total volume of ethanol = 49.675 ml. The resin solution was transferred to a Buchner filter funnel and washed with: 5 volumes of 0.1 M NaH₂PO₄, pH 7.5, 25% ethanol; 7 volumes of 0.1 M NaH₂PO₄, pH 7.5; and then 15 volumes of deionized H₂O. The total volume of the first wash (0.1M NaH₂PO₄, pH 7.5, 25% ethanol) was 0.250 L. The total volume of the second wash (0.1M NaH₂PO₄, pH 7.5) was 0.350 L. The total volume wash of the third wash (dH₂O) was 0.750 L. The final deionized H₂O was drained through the resin until it cracked and pulled from the side of the Buchner filter funnel. Once drained, the resin was scraped into an appropriate sized poly container, containing 50 ml of 14.5 M ammonium hydroxide. The container was then sealed and mixed overnight at 37 °C.

The resin/ammonium hydroxide slurry was poured into a glass container with slow mixing. The glass container was placed in an ice/water bath and cooled to 5 °C. The amount of glacial acetic acid needed to neutralize the ammonium hydroxide was calculated. Since 50 ml of 14.7 M ammonium hydroxide was used, then, 50 ml of ammonium hydroxide x 14.7 M / 17.4 M = 42.2 ml glacial acetic acid. The glacial acetic acid was slowly added while mixing, keeping the temperature below 40 °C.

After neutralization, the resin was collected on a Buchner filter funnel and washed as follows:
a. 5 volumes of 0.5 M NaCl = 250 ml
b. 12 volumes of deionized water = 600 ml
c. 5 volumes of 0.5 M NaCl = 250 ml
The resin was slurried in an equal volume of 0.5 M NaCl and stored at 2-5 °C.

### TNBS qualitative assay for primary amines on the resin

Three disposable chromatography columns were set up. The columns were labeled: Blank (Sepharose 6B), Old (amine activated resin from six months ago), and New (amine activated resin from current experiment). To each column, 0.5 ml of a 50% suspension of resin (to equal 0.25 ml of resin) was added. Columns were rinsed two times with 0.5 ml of 0.5M NaCl (pre-wash solution). Columns were washed with 0.5 ml of deionized H₂O. The columns were capped at the bottom and 0.5 ml of 0.1 M Borate, pH 10.0 (reaction solution) was added. Then 45 µl of 5% TNBS (Sigma, Product Code P 2297; amine specific reagent) was added. The tops of the columns were sealed with caps and the suspension was mixed by inversion for 30 sec. The suspension was allowed to incubate for 5 minutes to complete reaction with amines. The top and bottom seals were removed, and the columns allowed to drain. The columns were washed with 3 x 0.5 ml of 0.1M NaH₂PO₄ (monobasic, pH not adjusted). To each column, 0.5 ml of deionzed H₂O was added, the resin was resuspended and allowed to drain. The color of the resins was visually inspected and the relative color recorded.

The resin from the old batch of amine activated resin turned bright reddish orange. The resin from the new batch of amine activated resin also turned bright reddish orange. The resin from the blank Sepharose 6B showed no color change. This experiment thus provided qualitative proof that the new batch of resin thus had an adequate amount of free amines attached to its surface.

This experiment provided 50 ml of new amine activated resin to be used in the development of the composition. Also, this experiment examined the resin made six months ago. Based on the qualitative assay the 6 month old resin was still as good as it was on first day it was made. The color of the new resin versus the old, by visual inspection, was essentially identical.

### Maleimidocaproic acid NHS ester activation of resin

Four mls of the epoxy activated resin (see above) were washed with 5 volumes of 0.05 M NaH₂PO₄ pH 7.0. In a poly container, a 50% (1:1) gel suspension was prepared in 0.05 M NaH₂PO₄ pH 7.0. The total volume of buffer used was 4 ml. Three µmoles of maleimidocaproic acid (MA) NHS ester were dissolved per ml of gel in DMF to generate a 25 mg /ml solution.
Volume of gel = 4 ml
Amount of MA NHS ester needed =
   (3 µmol / ml gel) x 4 ml x 1/1000 mmol / µmole x 308.3 mg NHS ester / mmol = 3.7 mg MA NHS ester
Amount of MA NHS ester weighed out 5.7 mg
   5.7 mg MA NHS ester / 25 = 0.228 (ml)
Amount of DMF to add to the weighed out MA NHS ester.
   3.7 mg MA NHS ester needed /25 mg NHS ester /ml = 0.148 ml of 25 mg / ml MA NHS ester in DMF to add to the resin suspension.
One µmole of MA NHS ester per ml of gel was prepared using 25mg / ml MA NHS ester in DMF (Volume of gel = 2ml).

To the mixing gel suspension, the MA NHS ester/DMF solution was slowly added. The suspension was mixed at room temperature for 30 minutes. The unreacted amines on the resin were acetylated by adding acetic anhydride (0.028 x 4 ml resin = 0.112 ml acetic anhydride). The appropriate amount of acetic anhydride was added to the resin slurry slowly and mixed for 15 minutes. The resin slurry was transferred to a Buchner filter funnel and washed with 4 volumes of deionized H₂O. The resin was filtered to a damp cake and stored in a cold room until needed.

### Bromoacetic acid NHS ester activation

Three µmoles of bromoacetic acid NHS ester per ml of gel was prepared using bromoacetic acid NHS ester in DMF at a concentration of 25 mg/ml. To the mixing gel suspension, the NHS ester/DMF solution was slowly added. The suspension was mixed at room temperature for 30 minutes. The unreacted amines on the resin were acetylated by adding acetic anhydride (Sigma, Product Code A 6404; 0.028 x 4 ml resin = 0.112 ml acetic anhydride). The appropriate amount of acetic anhydride was slowly added to the resin slurry and mixed for 15 minutes. The resin slurry was transferred to a Buchner filter funnel and washed with 4 volumes of deionzed H₂O. The resin was filtered to damp cake and stored in a cold room until needed.

### C. Coupling of the Antibody to the Solid Support

Fresh 1 M hydroxylamine, pH 6.7, (10 ml) was made by dissolving 695 mg hydroxylamine and adjusting the pH with 1 M NaOH (approx 8 ml). The SATA activated antibodies were coupled to three different resins (maleimide activated at one or 3 µmoles / ml and bromoacetamide activated). Specifically 1.5 ml maleimide (3µmoles / ml) activated, 0.75 ml (1 µmoles / ml) and 0.75 ml of bromoacetamide activated resin slurry was spun on a centrifuge at 10,000 rpm for approximately 1 minute and the supernatant was removed from the resin bed. The reagents in Table 4-6 were added to the resin bed in 15 ml Corning tubes. The antibodies were added to the resin bed first and mixed, followed by the addition of the hydroxylamine. The tubes were mixed overnight in the cooler. Specifically 54 and 50 µl of 1M hydroxylamide was added to the bromoacetamide-antibody resin mix and the bromoacetamide blank resin respectively and mixed at room temperature for one hour. The resin was washed twice by resuspending with 3 column volumes of 3x PBS and centrifuging for 1 minute at 2,000 rpm in a clinical centrifuge. The resin was then washed twice with 3 column volumes of 0.1M Bis-Tris propane, (BTP), pH 8.5. One column volume of 0.1 M Bis-Tris propane, pH 8.5, 30 mM lodoacetamide was added to all tubes to block excess sulfhydryl moieties. This blocking step was carried out at room temperature on an orbital mixer for 30 minutes. (Buffer preparation: A 0.5 M stock solution of iodoacetamide was prepared with 600 µl of BTP buffer. This stock solution was added to 9.4 ml of BTP, pH 8.5.) The resin was washed three times with three column volumes of water, and three times with three column volumes of 2x PBS, pH 7.5. Sufficient glycerol was added to generate a final solution containing 50% glycerol / 50% PBS, and the resin was stored at 4 °C.

**Table 4.6: Reagents and quantities employed to couple antibodies to solid support using maleimide and bromoacetic acid NHS ester chemistries.**

| **Ab type** | **Ab conc (mg/ml)** | **Ab mg/ml resin** | **ml of 3mM Maleimide resin** | **Vol of Ab (ml)** | **Buffer (0.5M Na P pH 5.6** | **Hydroxyamine (ml)** | **Total vol (ml)** |
|---|---|---|---|---|---|---|---|
| **recombinant antibody fragments** | 9.3 | 3.3 | 1.5 | 0.53 | | | |
| **IgG/IgY** | 4.23 | 5.09 | 1.5 | 1.80 | | | |
| **Total** | | 8.39 | 1.5 | 2.34 | 0 | 0.099 | 3.9 |

| **Ab type** | **Ab conc (mg/ml)** | **Ab mg/ml resin** | **ml of 1mM Maleimide resin** | **Vol of Ab (ml)** | **Buffer (0.5M Na P pH 5.6** | **Hydroxyamine (ml)** | **Total vol (ml)** |
|---|---|---|---|---|---|---|---|
| **recombinant antibody fragments** | 9.3 | 3.3 | 0.75 | 0.27 | | | |
| **IgG/IgY** | 4.23 | 5.09 | 0.75 | 0.90 | | | |
| **Total** | | 8.39 | 0.75 | 1.17 | 0 | 0.049 | 2.0 |

| **Ab type** | **Ab conc (mglml)** | **Ab mg/ml resin** | **ml of Bromoacetamide resin** | **Vol of Ab (ml)** | **Buffer (0.5M Na P pH 5.6** | **1 M BTP pH 9.5 (ml)** | **Total vol (ml)** |
|---|---|---|---|---|---|---|---|
| **recombinant antibody fragments** | 9.3 | 3.3 | 0.75 | 0.27 | | | |
| **IgG/lgY** | 4.23 | 5.09 | 0.75 | 0.90 | | | |
| Total | | 8.39 | 0.75 | 1.17 | 0 | 0.221 | 2.1 |

| **Ab type** | **ml of 1 mM or 3 mM Maleimide resin** | **Vol of PBS-EDTA pH 6.7 (ml)** | **1M Hydroxyamine, pH 6.7 (ml)** | **Total vol (ml)** |
|---|---|---|---|---|
| **Blank** | 0.6 | 1.15 | 0.046 | 1.80 |

| **Ab type** | **ml of Bromoacetamide resin** | **Vol of PBS-EDTA pH 6.7 (ml)** | **1M BTP pH 9.5 (ml)** | **Total vol (ml)** |
|---|---|---|---|---|
| **Blank** | 0.6 | 1.15 | 0.207 | 1.96 |

Duplicate 10 µl aliquots of each of the resins, and 10µl aliquots of the antibodies, were assayed by the BCA-1 assay (reagents: 25 ml Reagent A (Sigma, Product Code B 9643) and 0.5 ml Reagent B (Sigma, Product Code C 2284)). The BCA assay was carried out overnight at room temp on a rotating wheel. The tubes were microcentrifuged at 8,000 rpm for 1 minute and 1 ml was transferred to disposable cuvets and read at A₅₆₂ (Table 4-7).

**Table 4-7: Protein Concentration bound to resin as determined by BCA Assay.**

| **Sample** | **mg anti/ml resin** | **Buffer (ml)** | **Sample (ml)** | **BCA reagent (µl)** | **A₅₆₂** | **mg** | **A₅₈₂** | **mg** | **mg/ml (BSA)** | BSA **to ligand mult fact.** | **mg protein /ml resin** | **avg mg/ml - blank** | **avg % coupling - blank** |
|---|---|---|---|---|---|---|---|---|---|---|---|---|---|
| **0 std** | | 750 | 0 | 750 | 0 | | | | | | | | |
| **10 std** | | 740 | 10 | 750 | 0.244 | | | | | | | | |
| **20 std** | | 730 | 20 | 750 | 0.4994 | | | | | | | | |
| **40 std** | | 710 | 40 | 750 | 0.8847 | | | | | | | | |
| **60 std** | | 690 | 60 | 750 | 1.1968 | | | | | | | | |
| **100 std** | | 650 | 100 | 750 | 1.8989 | | | | | | | | |
| **Blank** | 0 | 740 | 10 | 750 | 0.135 | 5.0 | 0.1783 | 6.9 | 1.2 | 0.79 | 0.9 | 0.0 | |
| **Mal 1** | 8.39 | 740 | 10 | 750 | 0.8175 | 37.5 | 0.8607 | 39.7 | 7.7 | 0.79 | 6.1 | 5.2 | 62 |
| **Blank** | 0 | 740 | 10 | 750 | 0.2698 | 11.1 | 0.2615 | 10.7 | 2.2 | 0.79 | 1.7 | 0.0 | |
| **Mal3** | 8.39 | 740 | 10 | 750 | 1.1664 | 55.9 | 1.0848 | 51.5 | 10.7 | 0.79 | 8.5 | 6.8 | 81 |
| **Blank** | 0 | 740 | 10 | 750 | 0.0495 | 1.2 | 0.0342 | 0.5 | 0.2 | 0.79 | 0.1 | 0.0 | |
| **BrAcet** | 8.39 | 740 | 10 | 750 | 0.8642 | 39.9 | 0.8426 | 38.8 | 7.9 | 0.79 | 6.2 | 6.1 | 73 |
| **antibody-SATA** | 5.36 | 740 | 10 | 750 | 1.1389 | 68.4 | 1.3613 | 66.9 | 6.8 | 0.79 | | | |

### Yellowing Study

An aliquot of the blank resins which were exposed to the coupling conditions were incubated in the 37 °C incubator overnight (viz. 18 hours). The resins were removed from the incubator and photographs were taken (Figure 5). The blank bromoacetamide activated resin remained white at 37 °C for 18 hours whereas the maleimide-activated resin turned yellow. Because of the reduced yellowing of the bromoacetamide activated resin, this change will be instituted into the protocol.

### D. Plasma Separation

Aliquots of the maleimide (3 µmole) resin were added to microcolumns as indicated in Table 4-8 below. The volume of 50% slurry was 2x the final volume of resin.

**Table 4-8: Plasma and wash volumes for each specific resin volume (maleimide chemistry).**

| **Sample #** | **Resin vol (µl)** | **Final vol of 25 µl Plasma (µl)** | **Wash vol (2X) (µl)** | **Final vol (µl)** | **Final dil** |
|---|---|---|---|---|---|
| **1** | 200 blank | 57 | 57 | 171 | 6.9 |
| **2** | 250 | 72 | 72 | 215 | 8.6 |
| **3** | 500 | 143 | 143 | 429 | 17.1 |
| **Total** | **1050** | | | | |

The snap off bottoms of the columns were removed, the caps loosened and the columns placed into 2 ml collection tubes. The columns were microcentrifuged at 5,000 rpm for 5 seconds. The columns were equilibrated with three washes of 1 column volume of PBS at 5,000 rpm for 5 seconds. The final spin was carried out for 30 seconds. Plasma (citrated) was diluted with PBS as indicated in the table below (Table 4-9).

**Table 4-9: Plasma dilution conditions for each specific resin volume (maleimide chemistry).**

| **Sample #** | **Resin vol (µl)** | **Final vol of 25 µl Plasma (µl)** | **2x vol of plasma (µl)** | **2x vol of PBS (µl)** |
|---|---|---|---|---|
| **1** | 200 blank | 57 | 50 | 64 |
| **2** | 250 | 72 | 50 | 93 |
| **3** | 500 | 143 | 50 | 236 |

An aliquot of the diluted plasma was added to each column and incubated for 10 minutes. The columns were then centrifuged at 10,000 rpm for 1 minute. The depleted plasma was reapplied to the column and incubated for 10 minutes. Again, the columns were subsequently centrifuged at 10,000 rpm for 1 minute. An aliquot of PBS equal to the diluted plasma volume was added to each column and then centrifuged at 10,000 rpm for 1 minute. This wash step was repeated. The pooled, depleted plasma was stored at -20 °C.

Aliquots of the bromoacetamide resin were added to microcolumns as indicated in Table 4-10 below. The volume of 50% slurry was 2x the final volume of resin.

**Table 4-10: Plasma and wash volumes for each specific resin volume (bromoacetic acid NHS ester chemistry).**

| **Sample** | **#Resin vol (µl)** | **Final vol of 25 µl Plasma (µl)** | **Wash vol (2X)(µl)** | **Final vol (µl)** | **Final dil** |
|---|---|---|---|---|---|
| **1** | 200 blank | 57 | 57 | 171 | 6.9 |
| **2** | 500 | 143 | 143 | 429 | 17.1 |

The snap off bottoms of the columns were removed, the caps loosened and the columns placed into 2 ml collection tubes. The columns were microcentrifuged at 5,000 rpm for 5 seconds. The columns were equilibrated with three washes of 1 column volume of PBS at 5,000 rpm for 5 seconds. The final spin was carried out for 30 seconds. Plasma (citrated) was diluted with PBS as indicated in the table below (Table 4-11).

**Table 4-11: Plasma dilution conditions for each specific resin volume (bromoacetic acid NHS ester chemistry).**

| **Sample #** | **Resin vol (µl)** | **Final vol of 25 µl Plasma (µl)** | **2x vol of plasma (µl)** | **2x vol of PBS (µl)** |
|---|---|---|---|---|
| **1** | 200 blank | 57 | 50 | 64 |
| **2** | 500 | 143 | 50 | 236 |

An aliquot of the diluted plasma was added to each column and incubated for 10 minutes. The columns were then centrifuged at 10,000 rpm for 1 minute. The depleted plasma was reapplied to the column and incubated for 10 minutes. Again, the columns were subsequently centrifuged at 10,000 rpm for 1 minute. An aliquot of PBS equal to the diluted plasma volume was added to each column and then centrifuged at 10,000 rpm for 1 minute. This wash step was repeated. The pooled, depleted plasma was stored at -20°C.

### Bradford Assay of Depleted Plasma

The Bradford was carried out in the cuvets with 50 µl of sample and 1.5 ml of Bradford reagent. The whole plasma (5 µl) was diluted 10-fold into PBS (45µl). Next, 45 µl of PBS was added to each of the sample cuvets, and 5 µl of either the diluted plasma or the depleted plasma was added. Then 1.5 ml of Bradford reagent was added to each. Samples were incubated for 25 minutes and read at A₅₉₅. The protein concentration of each sample was calculated. See representative table in Example 5.

### E. Analysis

### ELISA Assay (direct) of all 20 proteins:

The separated serum samples were diluted with serial 10 fold dilutions of coating buffer (100, 1,000, and 10,000 fold; see Table 4-12). The whole serum samples were diluted with serial 10 fold dilutions of coating buffer (100, 1000, 10,000, 100,000, and 1,000,000 fold; see Table 4-12). Ten plates were coated by adding 0.1 ml of diluted samples to each well. Plates were incubated at 4 °C overnight.

**Table 4-12: Antibody dilution table mapped to a 96 well plate format.**

| | **1 (1)** | **2 (2)** | **3 (3)** | **4 (P)** | **5 (1)** | **6 (2)** | **7 (3)** | **8 (P)** | **9(1)** | **10 (2)** | **11 (3)** | **12 (P)** |
|---|---|---|---|---|---|---|---|---|---|---|---|---|
| **A** | E6 | 10000 | 10000 | E6 | E6 | 10000 | 10000 | E6 | E6 | 10000 | 10000 | E6 |
| **B** | E6 | 10000 | 10000 | E6 | E6 | 10000 | 10000 | E6 | E6 | 10000 | 10000 | E6 |
| **C** | E5 | 1000 | 1000 | E5 | E5 | 1000 | 1000 | E5 | E5 | 1000 | 1000 | E5 |
| **D** | E5 | 1000 | 1000 | E5 | E5 | 1000 | 1000 | E5 | E5 | 1000 | 1000 | E5 |
| **E** | E4 | 100 | 100 | E4 | E4 | 100 | 100 | E4 | E4 | 100 | 100 | E4 |
| **F** | E4 | 100 | 100 | E4 | E4 | 100 | 100 | E4 | E4 | 100 | 100 | E4 |
| **G** | 1000 | BI | | 1000 | 1000 | Bl | | 1000 | 1000 | Bl | | 1000 |
| **H** | 1000 | Bl | | 1000 | 1000 | Bl | | 1000 | 1000 | Bl | | 1000 |

After incubation, the plates were washed 3 times with TBS-T using a platewasher. Next, the plates were blocked with a 0.3 ml TBS, 1% BSA solution. The blocking solution was incubated with the plates for a minimum of 30 minutes at room temperature, and was then aspirated from the wells.

### For the anti-human plasma protein primary antibodies:

Each anti-human antibody was diluted (1-20 mg/ml) 1:1,000 in TBS, 1% BSA, and 0.1 ml of the diluted antibody was added to each well. The anti-human antibody was incubated at 37 °C for 90 minutes. The plates were then washed 3 times with TBS-T using a platewasher.

### For the HRP-conjugated secondary antibodies:

The anti chicken and goat HRP conjugates were diluted 1:40,000 in TBS, 1% BSA. Specifically 5 µl of the anti Chicken HRP conjugate was added to 195 µl of TBS-BSA and then 50 µl of this solution was added to 50 ml of buffer. Next 5 µl of Goat HRP was conjugated to 195 µl of TBS-BSA and then 40 µl of this solution was added to 40 ml of TBS-BSA. The anti mouse HRP conjugate was diluted 1:50,000. Specifically 5 µl of the anti mouse HRP conjugate was added to 245 µl of TBS-BSA and then 12 µl of this solution was added to 12 ml of TBS-BSA. The anti rabbit HRP conjugate was diluted 1:40,000. Specifically 5 µl of the anti rabbit HRP conjugate was added to 195 µl of TBS-BSA and then 12 µl of this solution was added to 12 ml of TBS-BSA. Next 0.1 ml of each HRP conjugate was added to each well and incubated at 37 °C for 90 minutes. The plates were washed 3 times with TBS-T using a platewasher. Then 0.1 ml of TMB was added (cold, directly out of the refrigerator) to each well and incubated at room temperature for the amount of time indicated in Table 4-13, below. The reactions were stopped by addition of 0.1 ml 1M HCl. The plates were read at 450 nm.

**Table 4-13: TMB development times (in minutes) for discrete antibodies.**

| | **Transf** | **Fibrin** | **A2M** | **ApoA1** | **C3** | **HDL** | **Antitry** | **C1q** | **C4** | **IgG** |
|---|---|---|---|---|---|---|---|---|---|---|
| **HRP** | Chick | Chick | Chick | Chick | Chick | Chick | Chick | Chick | Chick | N/A |
| **Time** | 3.5 | 5 | 6(Nil) | Nil | 4 | Nil | 30 | 7 | 7 | 5 |
| | lgD | ApoB | Prealb | lgA | lgM | Acid1 | Cerulo | Plasm | Hapto | Albu |
| **HRP** | Goat | Goat | Goat | Goat | Goat | Goat | Goat | Goat | Rabbit | A9044 |
| **Time** | 15 | 13 | 16 | 2.5 | 8 | 10 | 10 | 25 | 5 | 4 |

The ELISA assays for alpha-2-mac, HDL and ApoA1 were repeated because the whole plate turned blue. The primary antibody of each was reduced by ½ (viz. 1:2000 dil.) The assays for the alpha-1-antitrypsin, C1q and C3 were also repeated. The amount of alpha-1-antitrypsin antibody was doubled (1:500 dil) because of the long incubation time. Two plates were coated overnight at 4 °C. With the exceptions noted immediately above, the protocol was the same as described. The plates were incubated with Tetra Methyl Benzidine (TMB) for the amount of time indicated in Table 4-14.

**Table 4-14: TMB development times (in minutes) for discrete antibodies.- Repeat Experiment.**

| | **A2M** | **Antitryp** | **C1q** | **ApoA1** | **C3** | **HDL** |
|---|---|---|---|---|---|---|
| **HRP** | Chick | Chick | Chick | Chick | Chick | Chick |
| **Time** | 2.25 | 65 | 16 | Nil | Nil | Nil |

Following the above protocol, the bromoacetamide and maleimide-depleted plasma was analyzed by ELISA. The dilutions were as depicted in Tables 4-15.

**Table 4-15: Antibody Dilution Table mapped to a 96 well plate format.**

| | **1 (1)** | **2 (2)** | **3 (P)** | **4(1)** | **5 (2)** | **6 (P)** | **7 (1)** | **8 (2)** | **9(P)** | **10(1)** | **11 (2)** | **12 (P)** |
|---|---|---|---|---|---|---|---|---|---|---|---|---|
| **A** | E6 | 10000 | E6 | E6 | 10000 | E6 | E6 | 10000 | E6 | E6 | 10000 | E6 |
| **B** | E6 | 10000 | E6 | E6 | 10000 | E6 | E6 | 10000 | E6 | E6 | 10000 | E6 |
| **C** | E5 | 1000 | E5 | E5 | 1000 | E5 | E5 | 1000 | E5 | E5 | 1000 | E5 |
| **D** | E5 | 1000 | E5 | E5 | 1000 | E5 | E5 | 1000 | E5 | E5 | 1000 | E5 |
| **E** | E4 | 100 | E4 | E4 | 100 | E4 | E4 | 100 | E4 | E4 | 100 | E4 |
| **F** | E4 | 100 | E4 | E4 | 100 | E4 | E4 | 100 | E4 | E4 | 100 | E4 |
| **G** | 1000 | BI | 1000 | 1000 | BI | 1000 | 1000 | BI | 1000 | 1000 | BI | 1000 |
| **H** | 1000 | BI | 1000 | 1000 | BI | 1000 | 1000 | BI | 1000 | 1000 | BI | 1000 |

Results were as follows in Table 4-16, Table 4-17 and Figures 6-8. The coupling efficiency was 62% for the maleimide (1 µmole), 73% for the bromoacetamide and 81 % for the maleimide (3 µmole) resins. The ELISA assays turned out poorly for the alpha-2-mac, ApoA1 and HDL antibodies / proteins in which the whole plate turned yellow. Reducing the amount of primary antibody in these assays helped but the concentrations must be reduced even more for the next experiment. The primary antibodies for alpha-2-mac, ApoA1 and HDL will have to be reduced from a 1:1000 dil to a 1:10,000 dil for the ELISA.

**Table 4-16: ELISA data from 25 µl of Plasma on 500 µL of Maleimide Activated Resin**

| **IgG** | | | | | | | | **IgA** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.10 | 1 | 1.00E+06 | 1.00E+06 | 0.10 | | | **Plasma** | 0.3 | 1 | 1.00E+05 | 1.00E+05 | 0.030 | | |
| 1 | 0.15 | 6.9 | 1.00E+05 | 6.90E+05 | 0.11 | 100.0 | 0.0 | **1** | 0.37 | 6.9 | 1.00E+04 | 6.90E+04 | 0.026 | 100.0 | 0.0 |
| **2** | | | | | | | | **2** | | | | | | | |
| **3** | 0.06 | 8.6 | 1.00E+04 | 8.60E+04 | 0.01 | 5.0 | 95.0 | **3** | 0.05 | 8.6 | 1.00E+04 | 8.60E+04 | 0.00 | 17.4 | 82.6 |
| **4** | 0.09 | 17.1 | 1.00E+03 | 1.71E+04 | 0.00 | 1.4 | 98.6 | **4** | 0.05 | 17.1 | 1.00E+03 | 1.71E+04 | 0.00 | 3.5 | 96.5 |

| **HAS** | | | | | | | | **Fibrinogen** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.05 | 1 | 1.00E+06 | 1.00E+06 | 0.05 | | | **Plasma** | 0.23 | 1 | 1.00E+06 | 1.00E+06 | 0.23 | | |
| **1** | 0.08 | 6.9 | 1.00E+05 | 6.90E+05 | 0.05 | 100.0 | 0.0 | **1** | 0.25 | 6.9 | 1.00E+05 | 6.90E+05 | 0.17 | 100.0 | 0.0 |
| **2** | | | | | | | | **2** | | | | | | | |
| **3** | 0.09 | 8.6 | 1.00E+04 | 8.60E+04 | 0.01 | 14.6 | 85.4 | **3** | 0.21 | 8.6 | 1.00E+04 | 8.60E+04 | 0.02 | 10.7 | 89.3 |
| **4** | 0.06 | 17.1 | 1.00E+03 | 1.71E+04 | 0.00 | 2.0 | 98.0 | **4** | 0.35 | 17.1 | 1.00E+03 | 1.71E+04 | 0.01 | 3.5 | 96.5 |

| **a2M** | | | | | | | | **Antitrypsin** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.11 | 1 | 1.00E+05 | 1.00E+05 | 0.011 | | | **Plasma** | 0.07 | 1 | 1.00E+05 | 1.00E+05 | 0.007 | | |
| **1** | 0.04 | 6.9 | 1.00E+05 | 6.90E+05 | 0.024 | 100.0 | 0.0 | **1** | 0.09 | 6.9 | 1.00E+04 | 6.90E+04 | 0.006 | 100.0 | 0.0 |
| **2** | | | | | | | | **2** | | | | | | | |
| **3** | 0.11 | 8.6 | 1.00E+03 | 8.60E+03 | 0.00 | 3.8 | 96.2 | **3** | 0.14 | 8.6 | 1.00E+03 | 8.60E+03 | 0.00 | 20.1 | 79.9 |
| **4** | 0.08 | 17.1 | 1.00E+02 | 1.71E+03 | 0.00 | 0.6 | 99.4 | **4** | 0.3 | 17.1 | 1.00E+02 | 1.71E+03 | 0.00 | 8.5 | 91.5 |

| **IgM** | | | | | | | | **transferrin** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.17 | 1 | 1.00E+05 | 1.00E+05 | 0.02 | | | **Plasma** | 0.27 | 1 | 1.00E+06 | 1.00E+06 | 0.27 | | |
| **1** | 0.02 | 6.9 | 1.00E+05 | 6.90E+05 | 0.02 | 100.0 | 0.0 | **1** | 0.04 | 6.9 | 1.00E+06 | 6.90E+06 | 0.28 | 100.0 | 0.0 |
| **2** | | | | | | | | **2** | | | | | | | |
| **3** | 0.13 | 8.6 | 1.00E+03 | 8.60E+03 | 0.00 | 6.9 | 93.1 | **3** | 0.25 | 8.6 | 1.00E+04 | 8.60E+04 | 0.02 | 7.5 | 92.5 |
| **4** | 0.03 | 17.1 | 1.00E+03 | 1.71E+04 | 0.00 | 3.0 | 97.0 | **4** | 0.14 | 17.1 | 1.00E+03 | 1.71E+04 | 0.00 | 0.8 | 99.2 |

| **Hapto** | | | | | | | | **Acid1glyco** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.06 | 1 | 1.00E+06 | 1.00E+06 | 0.06 | | | **Plasma** | 0.12 | 1 | 1.00E+05 | 1.00E+05 | 0.012 | | |
| **1** | 0.08 | 6.9 | 1.00E+05 | 6.90E+05 | 0.06 | 100.0 | 0.0 | **1** | 0.16 | 6.9 | 1.00E+04 | 6.90E+04 | 0.011 | 100.0 | 0.0 |
| **2** | | | | | | | | **2** | | | | | | | |
| **3** | 0.04 | 8.6 | 1.00E+04 | 8.60E+04 | 0.00 | 6.2 | 93.8 | **3** | 0.09 | 8.6 | 1.00E+03 | 8.60E+03 | 0.001 | 7.3 | 92.7 |
| **4** | 0.06 | 17.1 | 1.00E+03 | 1.71E+04 | 0.00 | 1.7 | 98.3 | **4** | 0.11 | 17.1 | 1.00E+02 | 1.71E+03 | 0.000 | 1.8 | 98.2 |

| **Ceruloplasmin** | | | | | | | | **IgD** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.11 | 1 | 1.00E+06 | 1.00E+06 | 0.11 | | | **Plasma** | 0.3 | 1 | 1.00E+05 | 1.00E+05 | 0.03 | | |
| **1** | 0.16 | 6.9 | 1.00E+05 | 6.90E+05 | 0.11 | 100.0 | 0.0 | **1** | 0.42 | 6.9 | 1.00E+04 | 6.90E+04 | 0.03 | 100.0 | 0.0 |
| **2** | | | | | | | | **2** | | | | | | | |
| **3** | 0.07 | 8.6 | 1.00E+04 | 8.60E+04 | 0.01 | 5.7 | 94.3 | **3** | 0.17 | 8.6 | 1.00E+03 | 8.60E+03 | 0.00 | 5.2 | 94.8 |
| **4** | 0.1 | 17.1 | 1.00E+03 | 1.71E+04 | 0.00 | 1.5 | 98.5 | **4** | 0.2 | 17.1 | 1.00E+02 | 1.71E+03 | 0.00 | 1.2 | 98.8 |

| **ApoA1** | | | | | | | | **C3** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.06 | 1 | 1.00E+06 | 1.00E+06 | 0.06 | | | **Plasma** | 0.09 | 1 | 1.00E+06 | 1.00E+06 | 0.09 | | |
| **1** | 0.11 | 6.9 | 1.00E+05 | 6.90E+05 | 0.07 | 100.0 | 0.0 | **1** | 0.15 | 6.9 | 1.00E+05 | 6.90E+05 | 0.10 | 100.0 | 0.0 |
| **2** | | | | | | | | **2** | | | | | | | |
| **3** | 0.14 | 8.6 | 1.00E+04 | 8.60E+04 | 0.01 | 15.6 | 84.4 | **3** | 0.23 | 8.6 | 1.00E+04 | 8.60E+04 | 0.02 | 18.5 | 81.5 |
| **4** | 0.1 | 17.1 | 1.00E+03 | 1.71 E+04 | 0.00 | 2.3 | 97.7 | **4** | 0.11 | 17.1 | 1.00E+03 | 1.71E+04 | 0.00 | 1.8 | 98.2 |

| **C1q** | | | | | | | | **HDL** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.29 | 1 | 1.00E+05 | 1.00E+05 | 0.03 | | | **Plasma** | 0.1 | 1 | 1.00E+05 | 1.00E+05 | 0.01 | | |
| **1** | 0.04 | 6.9 | 1.00E+05 | 6.90E+05 | 0.02 | 100.0 | 0.0 | **1** | 0.07 | 6.9 | 1.00E+04 | 6.90E+04 | 0.01 | 100.0 | 0.0 |
| **2** | | | | | | | | **2** | | | | | | | |
| **3** | 0.04 | 8.6 | 1.00E+04 | 8.60E+04 | 0.00 | 15.3 | 84.7 | **3** | 0.09 | 8.6 | 1.00E+03 | 8.60E+03 | 0.00 | 14.9 | 85.1 |
| **4** | 0.07 | 17.1 | 1.00E+03 | 1.71E+04 | 0.00 | 4.8 | 95.2 | **4** | 0.07 | 17.1 | 1.00E+02 | 1.71E+03 | 0.00 | 2.4 | 97.6 |
| **Prealbumin** | | | | | | | | **ApoB** | | | | | | | |

| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
|---|---|---|---|---|---|---|---|---|---|---|---|---|---|---|---|
| **Plasma** | 0.24 | 1 | 1.00E+05 | 1.00E+05 | 0.02 | | | **Plasma** | 0.24 | 1 | 1.00E+05 | 1.00E+05 | 0.02 | | |
| **1** | 0.04 | 6.9 | 1.00E+05 | 6.90E+05 | 0.02 | 100.0 | 0.0 | **1** | 0.04 | 6.9 | 1.00E+05 | 6.90E+05 | 0.02 | 100.0 | 0.0 |
| **2** | | | | | | | | **2** | | | | | | | |
| **3** | 0.15 | 8.6 | 1.00E+03 | 8.60E+03 | 0.00 | 5.3 | 94.7 | **3** | 0.04 | 8.6 | 1.00E+04 | 8.60E+04 | 0.00 | 15.0 | 85.0 |
| **4** | 0.14 | 17.1 | 1.00E+02 | 1.71E+03 | 0.00 | 1.0 | 99.1 | **4** | 0.06 | 17.1 | 1.00E+03 | 1.71E+04 | 0.00 | 4.5 | 95.5 |

| **Plasminogen** | | | | | | | | **C4** | | | | | | | |
|---|---|---|---|---|---|---|---|---|---|---|---|---|---|---|---|
| **Sample** | **A₄₀₅** | **depl dil** | **ELISA dil** | **total dilut** | **A₄₀₅** x dil | **% rem** | **% depl** | **Sample** | **A₄₀₅** | **depl dil** | **ELISA dil** | **total dilut** | **A₄₀₅** x dil | **% rem** | **% depl** |
| **Plasma** | 0.05 | 1 | 1.00E+06 | 1.00E+06 | 0.05 | | | **Plasma** | 0.51 | 1 | 1.00E+05 | 1.00E+05 | 0.051 | | |
| **1** | 0.5 | 6.9 | 1.00E+04 | 6.90E+04 | 0.03 | 100.0 | 0.0 | **1** | 0.06 | 6.9 | 1.00E+05 | 6.90E+05 | 0.041 | 100.0 | 0.0 |
| **2** | | | | | | | | **2** | | | | | | | |
| **3** | 0.09 | 8.6 | 1.00E+03 | 8.60E+03 | 0.00 | 2.3 | 97.7 | **3** | 0.12 | 8.6 | 1.00E+03 | 8.60E+03 | 0.00 | 2.4 | 97.6 |
| **4** | 0.14 | 17.1 | 1.00E+02 | 1.71E+03 | 0.00 | 0.7 | 99.3 | **4** | 0.03 | 17.1 | 1.00E+03 | 1.71E+04 | 0.00 | 1.1 | 98.9 |

**Table 4-17: ELISA data from 25 µl of Plasma on 500 µL of Bromoacetamide-Activated Resin**

| IgG | | | | | | | | IgA | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| **Plasma** | 0.80 | 1 | 1.00E+05 | 1.00E+05 | 0.080 | | | **Plasma** | 0.11 | 1 | 1.00E+06 | 1.00E+06 | 0.113 | | |
| **1** | 0.78 | 6.9 | 1.00E+04 | 6.90E+04 | 0.054 | 100.0 | 0.0 | **1** | 0.12 | 6.9 | 1.00E+05 | 6.90E+05 | 0.085 | 100.0 | 0.0 |
| **2** | | | | | | | | **2** | | | | | | | |
| **3** | | | | | | | | **3** | | | | | | | |
| **4** | 0.35 | 17.1 | 1.00E+03 | 1.71E+04 | 0.006 | 11.3 | 88.7 | **4** | 0.11 | 17.1 | 1.00E+04 | 1.71E+05 | 0.02 | 23.0 | 77.0 |

| HAS | | | | | | | | Fibrinogen | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.06 | 1 | 1.00E+06 | 1.00E+06 | 0.059 | | | Plasma | 0.15 | 1 | 1.00E+06 | 1.00E+06 | 0.149 | | |
| 1 | 0.09 | 6.9 | 1.00E+05 | 6.90E+05 | 0.059 | 100.0 | 0.0 | 1 | 0.17 | 6.9 | 1.00E+05 | 6.90E+05 | 0.119 | 100.0 | 0.0 |
| **2** | | | | | | | | 2 | | | | | | | |
| 3 | | | | | | | | 3 | | | | | | | |
| 4 | 0.34 | 17.1 | 1.00E+03 | 1.71E+04 | 0.006 | 9.9 | 90.1 | 4 | 0.13 | 17.1 | 1.00E+04 | 1.71E+05 | 0.022 | 18.6 | 81.4 |

| a2M | | | | | | | | antitrypsin | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.19 | 1 | 1.00E+05 | 1.00E+05 | 0.019 | | | Plasma | 0.36 | 1 | 1.00E+05 | 1.00E+05 | 0.036 | | |
| 1 | 0.26 | 6.9 | 1.00E+04 | 6.90E+04 | 0.018 | 100.0 | 0.0 | 1 | 0.27 | 6.9 | 1.00E+04 | 6.90E+04 | 0.018 | 100.0 | 0.0 |
| 2 | | | | | | | | 2 | | | | | | | |
| 3 | | | | | | | | 3 | | | | | | | |
| 4 | 0.07 | 17.1 | 1.00E+03 | 1.71E+04 | 0.001 | 6.1 | 93.9 | 4 | 0.07 | 17.1 | 1.00E+03 | 1.71E+04 | 0.001 | 6.2 | 93.8 |

| IgM | | | | | | | | transferrin | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.14 | 1 | 1.00E+05 | 1.00E+05 | 0.014 | | | Plasma | 0.22 | 1 | 1.00E+06 | 1.00E+06 | 0.217 | | |
| 1 | 0.19 | 6.9 | 1.00E+04 | 6.90E+04 | 0.013 | 100.0 | 0.0 | 1 | 0.03 | 6.9 | 1.00E+06 | 6.90E+06 | 0.221 | 100.0 | 0.0 |
| 2 | | | | | | | | 2 | | | | | | | |
| 3 | | | | | | | | 3 | | | | | | | |
| 4 | 0.06 | 17.1 | 1.00E+03 | 1.71E+04 | 0.001 | 7.6 | 92.4 | 4 | 0.1 | 17.1 | 1.00E+04 | 1.71E+05 | 0.017 | 7.7 | 92.3 |

| Hapto | | | | | | | | Acid1glyco | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.08 | 1 | 1.00E+06 | 1.00E+06 | 0.080 | | | Plasma | 0.19 | 1 | 1.00E+05 | 1.00E+05 | 0.0194 | | |
| 1 | 0.1 | 6.9 | 1.00E+05 | 6.90E+05 | 0.069 | 100.0 | 0.0 | 1 | 0.03 | 6.9 | 1.00E+05 | 6.90E+05 | 0.0193 | 100.0 | 0.0 |
| 2 | | | | | | | | 2 | | | | | | | |
| 3 | | | | | | | | | | | | | | | |
| 4 | 0.05 | 17.1 | 1.00E+04 | 1.71E+05 | 0.009 | 13.1 | 86.9 | 4 | 0.1 | 17.1 | 1.00E+03 | 1.71E+04 | 0.0017 | 8.9 | 91.1 |

| Ceruloplasmin | | | | | | | IgD | | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.43 | 1 | 1.00E+05 | 1.00E+05 | 0.043 | | | Plasma | 0.05 | 1 | 1.00E+06 | 1.00E+06 | 0.049 | | |
| 1 | 0.05 | 6.9 | 1.00E+05 | 6.90E+05 | 0.035 | 100.0 | 0.0 | 1 | 0.55 | 6.9 | 1.00E+04 | 6.90E+04 | 0.038 | 100.0 | 0.0 |
| 2 | | | | | | | | 2 | | | | | | | |
| 3 | | | | | | | | 3 | | | | | | | |
| 4 | 0.24 | 17.51 | 1.00E+03 | 1.75E+04 | 0.004 | 11.6 | 88.4 | 4 | 0.17 | 17.1 | 1.00E+03 | 1.71E+04 | 0.003 | 7.5 | 92.5 |

| ApoA1 | | | | | | | | C3 | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.11 | 1 | 1.00E+06 | 1.00E+06 | 0.110 | | | **Plasma** | 0.09 | 1 | 1.00E+06 | 1.00E+06 | 0.085 | | |
| 1 | 0.1 | 6.9 | 1.00E+05 | 6.90E+05 | 0.071 | 100.0 | 0.0 | 1 | 0.11 | 6.9 | 1.00E+05 | 6.90E+05 | 0.074 | 100.0 | 0.0 |
| 2 | | | | | | | | 2 | | | | | | | |
| 3 | | | | | | | | 3 | | | | | | | |
| 4 | 0.09 | 17.1 | 1.00E+04 | 1.71E+05 | 0.016 | 22.6 | 77.4 | 4 | 0.09 | 17.1 | 1.00E+04 | 1.71E+05 | 0.015 | 19.9 | 80.1 |

| C1q | | | | | | | | HDL | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.34 | 1 | 1.00E+05 | 1.00E+05 | 0.034 | | | Plasma | 0.03 | 1 | 1.00E+06 | 1.00E+06 | 0.032 | | |
| 1 | 0.34 | 6.9 | 1.00E+04 | 6.90E+04 | 0.024 | 100.0 | 0.0 | 1 | 0.04 | 6.9 | 1.00E+05 | 6.90E+05 | 0.025 | 100.0 | 0.0 |
| 2 | | | | | | | | 2 | | | | | | | |
| 3 | | | | | | | | 3 | | | | | | | |
| 4 | 0.14 | 17.1 | 1.00E+03 | 1.71E+04 | 0.002 | 10.5 | 89.5 | 4 | 0.04 | 17.1 | 1.00E+04 | 1.71E+05 | 0.007 | 26.8 | 73.2 |

| Prealbumin | | | | | | | ApoB | | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.16 | 1 | 1.00E+05 | 1.00E+05 | 0.016 | | | Plasma | 0.05 | 1 | 1.00E+06 | 1.00E+06 | 0.051 | | |
| 1 | 0.03 | 6.9 | 1.00E+05 | 6.90E+05 | 0.019 | 100.0 | 0.0 | 1 | 0.05 | 6.9 | 1.00E+05 | 6.90E+05 | 0.035 | 100.0 | 0.0 |
| 2 | | | | | | | | 2 | | | | | | | |
| 3 | | | | | | | | 3 | | | | | | | |
| 4 | 0.08 | 17.1 | 1.00E+03 | 1.71E+04 | 0.001 | 6.8 | 93.2 | 4 | 0.04 | 17.1 | 1.00E+04 | 1.71 E+05 | 0.006 | 18.8 | 81.2 |

| Plasminogen | | | | | | | C4 | | | | | | | | |
|---|---|---|---|---|---|---|---|---|---|---|---|---|---|---|---|
| Sample | A405 | depl dil | ELISA dil | total dilut | A405 x dil | % rem | % depl | Sample | A405 | depl dil | ELISA dil | total dilut | A405 x dil | % rem | % depl |
| Plasma | 0.37 | 1 | 1.00E+05 | 1.00E+05 | 0.037 | | | Plasma | 0.73 | 1 | 1.00E+05 | 1.00E+05 | 0.073 | | |
| 1 | 0.42 | 6.9 | 1.00E+04 | 6.90E+04 | 0.029 | 100.0 | 0.0 | 1 | 1.01 | 6.9 | 1.00E+04 | 6.90E+04 | 0.070 | 100.0 | 0.0 |
| 2 | | | | | | | | 2 | | | | | | | |
| 3 | | | | | | | | 3 | | | | | | | |
| 4 | 0.04 | 17.1 | 1.00E+03 | 1.71E+04 | 0.001 | 2.6 | 97.4 | 4 | 0.15 | 17.1 | 1.00E+03 | 1.71E+04 | 0.002 | 3.6 | 96.4 |

### EXAMPLE 5: Multiplexed conjugation of 20 antibodies to the resin and subsequent ELISA assay.

This example demonstrates an optimized resin production process, coupling 20 antibodies to the agarose support.

### A. Activation of the Antibodies

The A₂₈₀ of the antibodies were read to determine concentration. An extinction coefficient of 1.4 was used. The amount of antibody needed for SATA activation is shown in Table 5-1 below. Five milliliters of resin were coupled.

**Table 5-1: Details of antibodies (including concentrations) and activation reagents used to generate an optimized 20-plex resin.**

| **5 ml batch Antibody** | **Species** | **Conc mg/ml** | **mg needed/ml resin** | **20% increase to account for losses** | **mg for SATA activation for 5 ml** | **Volume (ml) for SATA activation** |
|---|---|---|---|---|---|---|
| **Albumin** | Llama | 9.9 | 2.33 | 2.80 | 14.00 | 1.41 |
| **IgG** | Llama | 9.7 | 0.48 | 0.58 | 2.88 | 0.30 |
| **Total recombinant antibody fragments** | | | 2.81 | | 16.88 | 1.71 |
| **Transferrin** | Goat | 1.20 | 0.38 | 0.45 | 2.25 | 1.88 |
| **Fibrinogen** | Chicken | 2.37 | 0.56 | 0.68 | 3.38 | 1.42 |
| **IgA** | Goat | 2.90 | 0.56 | 0.68 | 3.38 | 1.16 |
| **alpha-2-Mac** | Chicken | 1.27 | 0.29 | 0.35 | 1.75 | 1.38 |
| **lgM** | Goat | 2.50 | 0.29 | 0.35 | 1.75 | 0.70 |
| **alpha-1-Antitrypsin** | Goat | 2.73 | 0.27 | 0.32 | 1.60 | 0.59 |
| **Haptoglobin** | Rabbit | 8.78 | 0.21 | 0.25 | 1.25 | 0.14 |
| **Acid-1-glyco** | Goat | 2.81 | 0.33 | 0.40 | 2.00 | 0.71 |
| **Ceruloplasm** | Goat | 1.05 | 0.24 | 0.29 | 1.44 | 1.37 |
| **IgD** | Goat | 1.05 | 0.08 | 0.10 | 0.50 | 0.47 |
| **Apo A1** | Goat | 1.32 | 0.40 | 0.48 | 2.40 | 1.82 |
| **C1q** | Sheep | 0.52 | 0.33 | 0.40 | 1.98 | 3.81 |
| **C3** | Sheep | 0.95 | 0.50 | 0.60 | 3.00 | 3.16 |
| **ApoA2** | Goat | 0.53 | 0.40 | 0.48 | 2.40 | 4.53 |
| **Apo B** | Goat | 0.77 | 0.50 | 0.60 | 3.00 | 3.90 |
| **Plasminogen** | Goat | 2.44 | 0.08 | 0.10 | 0.50 | 0.20 |
| **C4** | Goat | 10.20 | 0.17 | 0.20 | 1.00 | 0.10 |
| **Prealbumin** | Goat | 0.57 | 0.17 | 0.20 | 1.00 | 1.76 |
| **Total IgG's** | | | **5.76** | | **34.57** | **29.10** |

### Activation of single chain recombinant antibody fragments

The single chain recombinant antibody fragments were mixed together (volumes were multiplied by 1.5 to give 2.12 + 0.45 and 0.15 ml was removed for assays so that the final volume was 2.42 ml in order to have enough volume to process) and SATA (0.25 M in DMF) was added to obtain a final SATA concentration of 6.5 mM. Freshly prepared 0.25 M SATA in DMF was employed. The volume of 0.25 M SATA = total volume of antibody mix (2.42 ml) x 6.5 / 250 = 0.063 ml SATA (Sigma Product Code A 9043). The SATA was freshly dissolved in DMF to generate a 0.25 M solution (viz. 58 mg/ml; or 20 mg / 345 µl DMF) and stored at -20 °C. The reaction was then allowed to react for 60 to 90 minutes at room temperature with no mixing during the reaction. The percent of amines remaining on the proteins was measured by carrying out a fluorescamine assay on the starting material and the reaction mix. The percent of remaining amines must be less than 25%. The solution was "Diafiltered" (3,000 MW cutoff) against 10 mM sodium phosphate, 0.5 M NaCl, 1 mM EDTA pH 7.4 (> 100 fold) and concentrated or diluted to 8 - 10 mg/ml by measuring the A₂₈₀ (see Table 5-2) (Note that the Bradford Protein concentration assay does not work with SATA-conjugated ligands). The solution was then diluted to 14 ml and then concentrated to approximately 1 ml. This process was repeated three times or until the flow thru (containing excess SATA) obtained an A₂₈₀ of less than 0.04.

**Table 5-2: Estimation of total antibody concentration before and after SATA activation of the small recombinant antibody ligands.**

| **Sample** | **Volume (ml)** | **A₂₈₀** | **A₂₈₀** | Dilution | **A₂₈₀** avg with dil | **mg/ml** | **Total mg** |
|---|---|---|---|---|---|---|---|
| **recombinant antibody fragments before SATA** | 2.4 | 0.1766 | 0.1781 | 50 | 8.87 | 9.90 | 23.76 |
| **recombinant antibody fragments after stir cell** | 2 | 0.2108 | 0.211 | 50 | 10.55 | 11.77 | 23.55 |
| **First flow thru** | 15 | 0.2758 | | 10 | 2.758 | | |
| **2^{nd} flow thru** | 15 | 0.7911 | | 1 | 0.791 | | |
| **3rd flow thru** | 15 | 0.1157 | | 1 | 0.116 | | |
| **4th flow thru** | 15 | 0.0362 | | 1 | 0.036 | | |

### Activation of the IgG antibodies

The protein concentrations of each antibody were determined and the volume necessary to produce the desired amount of resin calculated (Table 5-1). The IgG ligands were mixed together and the concentrations of sodium phosphate, NaCl and EDTA were adjusted to generate a final buffer concentration of 50 mM sodium phosphate, pH 7.5, 0.5 M NaCl, 1 mM EDTA, pH 7.4. A 0.1 ml sample was removed prior to adding the SATA for the fluorescamine assay. The SATA (0.25 M in DMF) was added to generate a final concentration of 1.8 mM. Freshly prepared 0.25 M SATA was employed. The solution was briefly mixed and then reacted for 60 - 90 minutes at room temperature with no mixing during the reaction proper. The percent of amines remaining on the protein was measured by carrying out a fluorescamine assay on the starting material and the reaction mix (see Table 5-3). Ideally, the percent of remaining amines should be 50 - 70%.

**Table 5-3: Estimations of activation level based on fluorescamine assay.**

| **Sample** | **0.05 M sodium phosphate, pH 8.2** | **2 mg / ml Fluoresc** | **A₄₈₀** | **A₄₈₀** | **A₄₈₀** | **A₄₈₀** | **Avg** | **% amines remaining** | **% amines coupled** |
|---|---|---|---|---|---|---|---|---|---|
| **Blank** | 1 ml | 10 µl | 0 | | | | 0 | | |
| **10 µl recombinant antibody fragments** | 1 ml | 10 µl | 1335 | 1329 | 1393 | 1325 | 1346 | 100 | 0 |
| **10 µl recombinant antibody fragments-SATA** | 1 ml | 10 µl | 208 | 216 | 223 | 222 | 217 | 16 | 84 |
| **Blank** | 1 ml | 10 µl | 0 | | | | 0 | | |
| **20 µl lgG antibodies** | 1 ml | 10 µl | 360 | 359 | 345 | 340 | 351 | 100 | 0 |
| **20 µl lgG antibodies-SATA** | 1 ml | 10 µl | 166 | 155 | 183 | 174 | 170 | 48 | 52 |

The SATA-activated antibody solution was "Diafiltered" (30,000 MW cutoff) against 10 mM sodium phosphate, 0.5 M NaCl, 1 mM EDTA pH 7.4 (> 100 fold) and concentrated or diluted to 6 - 8 mg/ml by measuring the A₂₈₀ (see Table 5-4) (Note, that the Bradford protein quantitation assay does not work with SATA-conjugated ligands). The antibody mixture was diluted to 14 ml and then re-concentrated to approximately 1 ml. Then 14 ml of buffer was added by way of dilution and then the solution re-concentrated. This process was repeated three times or until the flow thru (containing excess SATA) obtained an A₂₈₀ of less than 0.04. The eluate was then centrifuged at 2,000 rpm for 2 minutes in the clinical centrifuge.

**Table 5-4: Estimation of total antibody concentration before and after SATA activation of the lgG antibodies.**

| **Sample** | **Volume (ml)** | **A₂₈₀** | **A₂₈₀** | **Dil** | **A₂₈₀** avg with dil | **mg/ml** | **Total mg** |
|---|---|---|---|---|---|---|---|
| **lgG Ab after stir cell** | 3.3 | 0.2291 | 0.2314 | 50 | 11.51 | 8.22 | 27.14 |
| **First flow thru** | 20 | 0.6668 | | 10 | 6.67 | | |
| **2^{nd} flow thru** | 20 | 1.3243 | | 1 | 1.32 | | |
| **3^{rd} flow thru** | 20 | 0.1833 | | 1 | 0.18 | | |
| **4th flow thru** | 20 | 0.021 | | 1 | 0.02 | | |

### B. Activation of the Solid Support

### Epoxy activation of resin

Sepharose 6B (50 ml) was washed with 5 volumes of deionized water and filtered to a damp cake. The gel was transferred to an appropriate size poly container. The following reagents were added to the reaction vessel with mixing: 0.05 L of 1,4-butanediol diglycidyl ether and 0.05 L of 0.6 M NaOH. The reaction was allowed to proceed for 3-4 hours, while mixing on the orbital mixer at 3.5 rpm. When the reaction was complete (≥ 20 µmoles / ml of resin or incubation for 4 hours), the solution was neutralized by adding 974 ml of 1 M potassium phosphate monobasic per liter of resin. The total volume of potassium phosphate added was 48.7 ml. Ethanol (to a final partial volume of 25%) was added to the solution, to help 1,4-butanediol diglycidyl ether become more miscible. The total volume of ethanol used was 49.675 ml. The resin solution was transferred to a Buchner filter funnel and washed with: 5 volumes of 0.1 M NaH₂PO₄, pH 7.5, 25% ethanol; 7 volumes of 0.1 M NaH₂PO₄, pH 7.5; and then 15 volumes of deionized H₂O. The total volume of the first wash (0.1 M NaH₂PO₄, pH 7.5, 25% ethanol) was 0.250 L. The total volume of the second wash (0.1 M NaH₂PO₄, pH 7.5) was 0.350 L. The total volume wash of the third wash (dH₂O) was 0.750 L. The final deionized H₂O was drained through the resin until it cracked and pulled from the side of the Buchner filter funnel. Once drained, the resin was scraped into an appropriate sized poly container, containing 50 ml of 14.5 M ammonium hydroxide. The container was then sealed and mixed overnight at 37 °C.

The resin/ammonium hydroxide slurry was poured into a glass container with slow mixing. The glass container was placed in an ice/water bath and cooled to 5 °C. The amount of glacial acetic acid needed to neutralize the ammonium hydroxide was calculated. Since 50 ml of 14.7 M ammonium hydroxide was used, then, 50 ml of ammonium hydroxide x 14.7 M / 17.4 M = 42.2 ml glacial acetic acid. The glacial acetic acid was slowly added while mixing, keeping the temperature below 40 °C.

After neutralization, the resin was collected on a Buchner filter funnel and washed as follows:
a. 5 volumes of 0.5 M NaCl = 250 ml
b. 12 volumes of deionized water = 600 ml
c. 5 volumes of 0.5 M NaCl = 250 ml
The resin was slurried in an equal volume of 0.5 M NaCl and stored at 2-8 °C.

### TNBS qualitative assay for primary amines on the resin

Three disposable chromatography columns were set up. The columns were labeled: Blank (Sepharose 6B), Old (amine activated resin from six months ago), and New (amine activated resin from current experiment). To each column, 0.5 ml of a 50% suspension of resin (to equal 0.25 ml of resin) was added. Columns were rinsed two times with 0.5 ml of 0.5M NaCl (pre-wash solution). Columns were washed with 0.5 ml of deionized H₂O. The columns were capped at the bottom and 0.5 ml of 0.1 M Borate, pH 10.0 (reaction solution) was added. Then 45 µl of 5% TNBS (Sigma, Product Code P 2297; amine specific reagent) was added. The tops of the columns were sealed with caps and the suspension was mixed by inversion for 30 seconds. The suspension was allowed to incubate for 5 minutes to complete reaction with amines. The top and bottom seals were removed, and the columns allowed to drain. The columns were washed with 3 x 0.5 ml of 0.1 M NaH₂PO₄ (monobasic, pH not adjusted). To each column, 0.5 ml of deionized H₂O was added, the resin was resuspended and allowed to drain. The color of the resins was visually inspected and the relative color recorded.

The resin from the old batch of amine activated resin turned bright reddish orange. The resin from the new batch of amine activated resin turned bright reddish orange. The resin from the blank Sepharose 6B showed no color change. This experiment thus provided qualitative proof that the new batch of resin thus has adequate amounts of free amines attached to its surface.

This experiment provided 50 ml of new amine activated resin to be used in the development of the composition. Also, this experiment examined the resin made six months ago. Based on the qualitative assay the 6 month old resin was still as good as it was on first day it was made. The color of the new resin versus the old, by visual inspection, was practically identical.

### Bromoacetic acid NHS ester activation

A 25 mg / ml bromoacetic acid NHS ester stock solution (106 mM) in DMF was prepared. The aminated resin was washed with 5 resin volumes of 50 mM sodium phosphate, pH 7.0. The resin was transferred to a suitably sized container and 1 resin volume of 50 mM sodium phosphate, pH 7.0 was added. To the mixing gel suspension, the 106 mM bromoacetic NHS ester/DMF solution was added to generate a final concentration of 6 mM [e.g. 56.6 µl / ml gel (6mM)]. The suspension was mixed at room temperature for 30 minutes. The unreacted amines on the resin were acetylated by adding acetic anhydride (Sigma Product Code A6404). For example 0.028 x 100 ml resin = 2.8 ml acetic anhydride. The appropriate amount of acetic anhydride was slowly added to the resin slurry and mixed for 15 minutes. The resin slurry was transferred to a Buchner filter funnel and washed with 6 volumes of 0.5 M NaCl. The slurry was filtered to damp cake and stored at 2 - 8°C until it was needed.

### C. Coupling of the Antibody to the Solid Support

The washed bromoacetamide-activated resin was transferred to a suitably sized container. Both the SATA activated single chain recombinant antibody fragments and IgG antibodies were added as shown in Table 5-5 to the resin and briefly mixed to suspend the resin.

**Table 5-5: Reagents and quantities employed to couple antibodies to solid support using bromoacetic acid NHS ester chemistries.**

| **Ab type** | **Ab conc (mg/ml)** | **mg Ab/ml resin** | **6 mM Bromoacetamide resin (ml)** | **Vol of Ab (ml)** | **5M NaCl (ml)** | **1M BTP pH 9.5 (ml)** | **Total vol (ml)** |
|---|---|---|---|---|---|---|---|
| **recombinant antibody fragments** | 11.8 | 2.81 | 4.50 | 1.07 | | | |
| **IgG/IgY** | 8.22 | 5.76 | 4.50 | 3.15 | | | |
| **Total** | 9.12 | 8.57 | 4.50 | 4.23 | 0.10 | 0.99 | 9.8 |

A 1 M Bis-Tris Propane (BTP)-HCl pH 9.5 buffer (56.4 g BTP was dissolved in 100 ml water and the pH adjusted with 1 M HCl; the volume was then adjusted to 200 ml with water) and 5 M NaCl was added to the resin mix to give final concentrations of 0.1 M BTP, pH 9.5 and 0.5 M NaCl. The solution was mixed overnight at 2- 8 °C. The mixing rate was such that the resin remained suspended. The mixture was then allowed to incubate at room temperature for 1 hour with mixing. The resin slurry was centrifuged at 2,000 rpm for 2 minutes and the supernatant removed. Next 1.5 resin volumes of 0.1 M Bis-Tris propane, pH 7.5, 0.5 M NaCl, 30 mM lodoacetamide were added to the resin to block excess sulfhydryl moieties. This blocking step was carried out at room temperature on an orbital mixer for 1 hour. The final pH was approximately 8.5.

To the bromoacetamide-antibody resin a 1/10 volume of 1 M hydroxylamide pH 8.5 (final 0.1 M) was added and the solution was then mixed at room temperature for 1 hour. Specifically, 10 ml of 1 M hydroxylamine, pH 8.5, was made by titrating 695 mg hydroxylamine with 1.7 ml of 5 M NaOH. The resin was filtered and washed with 10 resin volumes of 0.1M BTP, 0.5 M NaCl, pH 8.5.and then washed with 10 resin volumes of water. The slurry was filtered to a moist cake. The resin was washed with 10 resin volumes of 0.1 M glycine, pH 2.5. The resin was then washed with 10 resin volumes of water and 10 resin volumes of 2 x PBS. The packed resin was transferred to a suitably sized container and 1 resin volume of glycerol was added to the resin bed. Next, Kathon (1.5% w/v) was added at a 1:1000 dilution. The resin was stored at 2-8°C. Triplicate 10 µl aliquots of the resin, and 10 µl aliquots of the antibody, were assayed by the BCA-1 assay [reagents: 25 ml Reagent A (Sigma Product Code B 9643) and 0.5 ml Reagent B (Sigma Product Code C 2284)]. The BCA assay was carried out overnight at room temperature on a rotating wheel. The tubes were microcentrifuged at 8,000 rpm for 1 min and 1 ml was transferred to disposable cuvets and read at A₅₆₂ (Table 5-6).

**Table 5-6: Protein Concentration bound to resin as determined by BCA Assay.**

| **Sample** | **mg Ab/ ml resin** | **Buffer (µl)** | **Sample (µl)** | **BCA reagent (µl)** | **A₅₆₂** | **mg** | **A₅₆₂** | **mg** | **A₅₆₂** | **mg** | **mg/ ml (BSA)** | **BSA to ligand mult fact.** | **mg protein /ml resin** | **Avg % coupling** |
|---|---|---|---|---|---|---|---|---|---|---|---|---|---|---|
| **0 std** | | 750 | 0 | 750 | 0 | | | | | | | | | |
| **10 std** | | 740 | 10 | 750 | 0.2887 | | | | | | | | | |
| **20 std** | | 730 | 20 | 750 | 0.5348 | | | | | | | | | |
| **40 std** | | 710 | 40 | 750 | 0.9611 | | | | | | | | | |
| **60 std** | | 690 | 60 | 750 | 1.3453 | | | | | | | | | |
| **100 std** | | 650 | 100 | 750 | 2.0724 | | | | | | | | | |
| **100%** resin | 8.57 | 740 | 10 | 750 | 1.4324 | 63.3 | 1.36 | 59.5 | 1.423 | 62.8 | 12.4 | 0.80 | 9.8 | 115 |
| **Antibody -SATA** | 9.12 | 740 | 10 | 750 | 2.2728 | 114 | 2.37 | 121 | 2.197 | 108.7 | 11.5 | 0.80 | 9.1 | |

### D. Plasma Separation

Columns were prepared by pipetting 7.5 ml of resin slurry (to equal 3.75 ml resin) into columns. The columns were equilibrated with 16 ml of 1x PBS (Sigma Product Code P 5493) using a syringe. The columns were used to deplete 100 µl, 200 µl, and 300 µl of plasma. The plasma (citrated;100 µl, 200 µl, 300 µl) was diluted to 1.25 ml with (1.15 ml, 1.05 ml, 0.95 ml) of PBS, respectively and filtered through a 0.2 µm spin filter. Note that 3.5 ml of diluted plasma was generated. The diluted plasma solutions (1.25 ml) were added to the columns and incubated for 20 minutes. The columns were centrifuged at 5,000 rpm for 1 minute. The pull through was kept and stored at 2 - 8°C. PBS equal to the diluted plasma volume (1.25 ml) was added to the columns and then centrifuged at 5,0000 rpm for 1 minute. The pull through was kept and stored at 2 - 8 °C. This wash step was repeated one time. The columns were washed 1 time with 1 column volume (3.75 ml) of PBS with centrifugation at 5,000 rpm for 1 minute, to remove any trace amounts of unbound protein. The wash was then discarded. The columns were stripped with 12 ml of 1x ProteoPrep 20 Elution Solution. The elution was neutralized with 500 µl of 1M Trizma. The columns were immediately reequilibrated with 16 ml of PBS. A Bradford Protein Quantitation assay was then run on the three saved washes, to determine protein content (Table 5-7).

**Table 5-7: Bradford Protein Quantitation assay run on three washes.**

| **Value** | **PBS (µl)** | **1 mg/ml BSA (µl)** | **Sample** | **A₅₉₅** | **A₅₉₅** | **Conc (mg/ml)** | **Cone (mg/ml)** | **Avg conc** | **Conc with dilution** |
|---|---|---|---|---|---|---|---|---|---|
| | 50 | 0 | | 0 | | | | | |
| | 43 | 6.3 | | 0.0892 | | | | | |
| | 37.5 | 12.5 | | 0.1872 | | | | | |
| | 25 | 25 | | 0.4123 | | | | | |
| | 12.5 | 37.5 | | 0.5768 | | | | | |
| | 0 | 50 | | 0.7045 | | | | | |
| **100 ul 1st** | | | 50 | 0.0824 | 0.0842 | 0.1063 | 0.1084 | 0.10735 | 0.11 |
| **100 ul 2nd** | | | 50 | 0.0912 | 0.0837 | 0.1163 | 0.1079 | 0.1121 | 0.11 |
| **100 ul 3rd** | | | 50 | 0.0397 | 0.0408 | 0.0589 | 0.06 | 0.05945 | 0.06 |
| **300 ul 1st** | 40 | | 10 | 0.1818 | 0.1861 | 0.2212 | 0.2263 | 0.22375 | 1.12 |
| **300 ul 2nd** | 40 | | 10 | 0.3097 | 0.3076 | 0.3794 | 0.3767 | 0.37805 | 1.89 |
| **300 ul 3rd** | 40 | | 10 | 0.1354 | 0.1404 | 0.1668 | 0.1726 | 0.1697 | 0.85 |

1.5 ml of Bradford reagent was added and incubated for 20 - 30 minutes. The solutions were assayed at A₅₉₅.

### E. Analysis

### ELISA Assay (direct) of all 20 proteins:

The depleted plasma samples that had only been separated once (unpolished) were subjected to an 80, 160 or 240 fold dilution [50 µl with 4 (100 µl), 8 (200 µl) or 12 ml (300 µl) coating buffer] and a 400, 800 and 1200 fold dilution (10 µl with 4, 8 and 12 ml coating buffer). The depleted plasma samples separated twice (polished) were subjected to a 40, 80, 120 fold dilution [100 µl with 4 (100 µl), 8 (200 µl) or 12 ml (300 µl) coating buffer] and a 200, 400, 600 fold dilution (20 µl with 4, 8 and 12 ml coating buffer). The whole plasma samples were subjected to serial 10 fold dilutions starting from 400 (20 µl with 8 ml coating buffer) and 4,000 (0.80 ml with 7.2 ml). The final dilutions were made as follows: 8,000 (2 ml with 2 ml) and 40,000 fold (0.40 ml with 3.6 ml). Four plates were coated by adding 0.1 ml of diluted samples to the wells of the plates as detailed in the table below. Plates were stored at 4 °C overnight.

**Table 5-8: Antibody Dilution Table mapped to a 96 well plate format.**

| | 1 | 2 | 3 | 4 | 5 | 6 | 7 | 8 | 9 | 10 | 11 | 12 |
|---|---|---|---|---|---|---|---|---|---|---|---|---|
| **A** | **Plasma** | **Plasma** | **Plasma** | **Plasma** | **Plasma** | **Plasma** | **Plasma** | **Plasma** | **Plasma** | **Plasma** | **Plasma** | **Plasma** |
| **B** | BI | BI | BI | BI | BI | BI | BI | BI | BI | BI | BI | BI |
| **C** | 100 | 200 | 300 | 100 | 200 | 300 | 100 | 200 | 300 | 100 | 200 | 300 |
| **D** | 100 | 200 | 300 | 100 | 200 | 300 | 100 | 200 | 300 | 100 | 200 | 300 |
| **E** | 100 | 200 | 300 | 100 | 200 | 300 | 100 | 200 | 300 | 100 | 200 | 300 |
| **F** | 100p | 200p | 300p | 100p | 200p | 300p | 100p | 200p | 300p | 100p | 200p | 300p |
| **G** | 100p | 200p | 300p | 100p | 200p | 300p | 100p | 200p | 300p | 100p | 200p | 300p |
| **H** | 100p | 200p | 300p | 100p | 200p | 300p | 100p | 200p | 300p | 100p | 200p | 300p |

After incubation, the plates were washed 3 times with TBS-T using a platewasher. Next, the plates were blocked with a 0.3 ml TBS, 1% BSA solution. The blocking solution was incubated with the plates for a minimum of 30 minutes at room temperature, and was then aspirated from the wells.

### For the human plasma protein primary antibodies:

Each anti-human plasma protein antibody was diluted (1-20 mg / ml) 1:1,000 in TBS, 1% BSA, and 0.1 ml of the diluted antibody was added to each well. The anti-human antibody was incubated at 37 °C for 90 minutes.

### For the HRP Conjugate secondary antibodies:

The anti chicken and goat HRP conjugates were diluted 1:40,000 in TBS, 1% BSA. Specifically 5 µl of the anti Chicken HRP conjugate to was added to 195 µl of TBS-BSA and then 50 µl of this solution was added to 50 ml of buffer. Also 5 µl of Goat HRP conjugate was added to 195 µl of TBS-BSA and then 40 µl of this solution was added to 40 ml of TBS-BSA. The anti mouse HRP conjugate was diluted 1:50,000. Specifically 5 µl of the anti mouse HRP conjugate was added to 245 µl of TBS-BSA and then 12 µl of this solution was added to 12 ml of TBS-BSA. The anti rabbit HRP conjugate was diluted 1:40,000. Specifically 5 µl of the anti rabbit HRP conjugate was added to 195 µl of TBS-BSA and then 12 µl of this solution was added to 12 ml of TBS-BSA. Next 0.1 ml of each HRP conjugate was added to each well and incubated at 37 °C for 90 minutes. The plates were washed 3 times with TBS-T using a platewasher. Then 0.1 ml of TMB was added (cold, directly out of the refrigerator) to each well and incubated at room temperature for the amount of time detailed in Table 4-13, below. The reactions were stopped by the addition of 0.1 ml 1M HCl. The plates were read at 450 nm. Results are shown in Figure 9.

**Table 5-9: TMB development times (in minutes) for discrete antibodies. Dilution values (1st value = depleted plasma dilution; 2^{nd} value = whole plasma dilution).**

| | **400 and 40,000** | | | | **80 and 8,000** | | | |
|---|---|---|---|---|---|---|---|---|
| **Plate** | **Primary** | | **Sec** | **time** | **Primary** | | **Sec** | **time** |
| **1** | Alpha-2-mac | ICL | Ch | 9.5 | Acid-1-glyco | ICL | Go | 3 |
| **1** | Fibrinogen | ICL | Ch | 1.5 | IgM | Sigma | Go | 1.5 |
| **1** | C3 | Chris | Sh | 8.5 | Apo A1 | BiosPacific | Go | 1 |
| **1** | Transferrin | Bethyl | Go | 3 | Haptoglobin | Athens | Ra | 3 |
| | | | | | | | | |
| **2** | Albumin | Sigma | Mo | 3.5 | Apo All | Chris | Go | 2 |
| **2** | IgG | Sigma | Mo | 11 | IgD | Bethyl | Go | 6 |
| **2** | IgA | Sigma | Go | 2.5 | Apo B | USB | Go | 2 |
| **2** | Antitrypsin | ICL | Go | 3 | Plasminogen | USB | Go | 8 |
| | | | | | | | | |
| **3** | C4 | BiosPacific | Go | 10.5 | Prealbumin | Chris | Go | 11 |
| **3** | Ceruloplasmin | Bethyl | Go | 10.5 | C1q | Chris | Sh | 18 |
| | | | | | | | | |

**Table 5-10: Percent depletions for each plasma volume. The "p" designation is for the polished sample after the second depletion.**

| | 100 | 100p | 200 | 200p | 300 | 300p |
|---|---|---|---|---|---|---|
| Albumin | 97.0 | 99.8 | 90.6 | 99.9 | 87.7 | 99.3 |
| IgG | 98.0 | 99.9 | 83.6 | 99.8 | 69.1 | 99.7 |
| Transferrin | 98.0 | 100.0 | 90.5 | 100.0 | 85.7 | 99.7 |
| Fibrinogen | 92.8 | 99.4 | 80.7 | 98.2 | 75.5 | 96.1 |
| IgA | 98.3 | 99.9 | 87.8 | 100.0 | 79.5 | 99.8 |
| Alpha-2-Macroglobulin | 98.7 | 99.3 | 91.5 | 99.9 | 81.5 | 100.0 |
| lgM | 96.5 | 98.0 | 95.1 | 97.4 | 93.8 | 97.0 |
| Alpha-1-Antitrypsin | 98.3 | 99.9 | 82.2 | 100.0 | 68.5 | 99.5 |
| Haptoglobin | 98.0 | 99.6 | 97.1 | 99.6 | 96.3 | 99.5 |
| C3 Complement | 99.7 | 100.0 | 96.1 | 100.0 | 89.3 | 100.0 |
| Apolipoprotein A1 | 96.1 | 99.6 | 95.7 | 99.2 | 94.8 | 98.4 |
| Apolipoprotein B | 87.0 | 93.9 | 87.2 | 93.2 | 83.6 | 92.3 |
| Alpha-1-acid Glycoprotein | 98.6 | 99.9 | 97.0 | 100.0 | 94.9 | 99.9 |
| Ceruloplasmin | 99.1 | 99.9 | 93.4 | 99.9 | 85.8 | 99.7 |
| Prealbumin | 98.3 | 99.7 | 96.1 | 99.9 | 92.6 | 99.8 |
| lgD | 98.5 | 100.0 | 96.8 | 100.0 | 94.8 | 99.9 |
| ApoA2 | 94.3 | 99.8 | 94.4 | 99.3 | 91.6 | 98.1 |
| Plasminogen | 98.9 | 100.0 | 95.5 | 100.0 | 92.0 | 100.0 |
| C4 | 98.1 | 98.8 | 95.6 | 98.5 | 92.5 | 98.3 |
| C1q | 99.5 | 99.8 | 98.6 | 99.7 | 97.4 | 99.8 |
| **Average** | 97.2 | 99.4 | 92.3 | 99.2 | 87.4 | 98.8 |

### Example 6. One step separation of high abundance proteins and fractionation of low abundance proteins using liquid chromatography

### A. Column Chromatography

The study of the human plasma proteome is especially important for the identification of disease biomarkers. Many proteins of interest appear at low concentrations (pg/ml - ng/ml) in the plasma, and are, therefore, difficult to detect. Identification of potential biomarkers is especially difficult due to the presence of higher abundance proteins. Removal of the high abundance proteins allows for detection of low abundance proteins that co-migrate with, and are masked by, the higher abundance proteins during separation procedures. The purpose of this experiment was to test a liquid chromatography (LC) procedure using an antibody-based resin for the depletion of 20 high abundance plasma proteins and the concomitant size fractionation of the low abundance proteins

A column (10 x 120 mm) was prepared with 10 ml of PROT20 immunodepletion resin, which comprises Sepharose® 6B resin coupled to antibodies against the following human plasma proteins: albumin, IgGs, transferrin, fibrinogen, IgAs, alpha-2-macroglobulin, IgMs, alpha-1-antitrypsin, complement C3, haptoglobin, apolipoprotein A1, apolipoprotein A2, apolipoprotein B, alpha-1-acid glycoprotein, ceruloplasmin, complement C4, complement C1q, IgDs, prealbumin, and plasminogen. While Sepharose® 6B is a matrix for coupling affinity ligands, it also is a size exclusion matrix that fractionates globular proteins that range from 1 x 10⁴ to 4 x 10⁶ Daltons. The column was connected to an AKTA FPLC (GE Healthcare) LC unit, which was programmed as indicated in Table 6.1.

**Table 6.1. LC Workflow**

| Step | Flow rate (ml/min) | % Elution Buffer | % Elution Buffer | Volume (ml) | Time (min) |
|---|---|---|---|---|---|
| 1 | 0.3 | 100 | 0 | 0 | 0 |
| 2 | 0.3 | 100 | 0 | 16 | 53 |
| 3 | 3.0 | 0 | 100 | 30 | 63 |
| 4 | 3.0 | 100 | 0 | 30 | 73 |

Citrated human plasma (100 µl) was diluted to 500 µl with equilibration buffer (1x PBS) and injected into the column. Fractions (1 ml) were collected during step 2. The bound proteins were eluted with elution buffer (0.1 M glycine-HCl, pH 2.5, and 0.1 % (w/v) octyl β-D-glucopyranoside) in step 3. Figure 10 presents a chromatogram of a typical separation. Several peaks of protein, as monitored by A₂₈₀ absorption, are detected in the depleted plasma flow-through. The protein concentration of the depleted plasma was determined using a Bradford assay.

### B. Characterization of depleted fractions using SDS-PAGE

SDS-PAGE analysis was used to visualize the proteins present in each fraction of the depleted plasma. An aliquot of each fraction was pooled for comparison. Aliquots of nine individual factions (#4-12) and the pooled fraction equivalent to 4.7 µl of original plasma were loaded onto the gel. A sample of whole plasma equivalent to 0.19 µl of the original plasma was also loaded. Figure 11 presents Coomassie and silver stained gels. Many more proteins can be visualized in the individual fractions than in the pooled fraction, in which proteins appear to be masked by other proteins.

### C. Identification of proteins in the depleted fractions

Mass spectrometry was used to determine whether fractionation increased the number of proteins that could be identified. The depleted fractions from two depletion runs were each collected in 1 ml fractions and pooled (2 ml). An aliquot (0.7 ml) from each of the combined fractions (Fractions #4-12) was removed. Another 0.7 ml aliquot from each fraction was pooled together (6.3 ml total volume). The samples were processed by adding 1/20 volume of 1M ammonium bicarbonate, pH 8.4, to each sample. The samples were reduced and alkylated with tributylphosphine and iodoacetamide (Cat. No. PROTRA). The samples were then concentrated and the buffer was exchanged to 50 mM ammonium bicarbonate, pH 8.3 using 5,000 NMWL filters (Amicon Ultra, Millipore). Protein concentration was determined using a Bradford Assay (Cat. No. B6916). The samples were digested with trypsin (Cat. No. T6567) at a concentration of 3.3% (w/w) and allowed to incubate at 37 °C overnight. The digests were dried down in a SpeedVac and dissolved in 30 µl of 5% acetonitrile and 0.1 % formic acid, and then further diluted 5-fold. Samples of the digests (5 µl) were injected onto a nano-LC column (PepMap C18, 3 µm, 100A) coupled to a QSTAR XL Q-TOF Mass Spectrometer. Protein identifications, probability scores (MOWSE), and the number of peptides were determined from a Mascot search of the data (Table 6.2).

This analysis revealed that many more proteins were identified in the fractioned samples. Forty proteins were identified with two or more peptides and 68 proteins were identified with at least one peptide in the different fractions. In contrast, only 17 proteins were identified in the pooled, unfractionated sample, and only 13 proteins were identified in the whole plasma (WP).

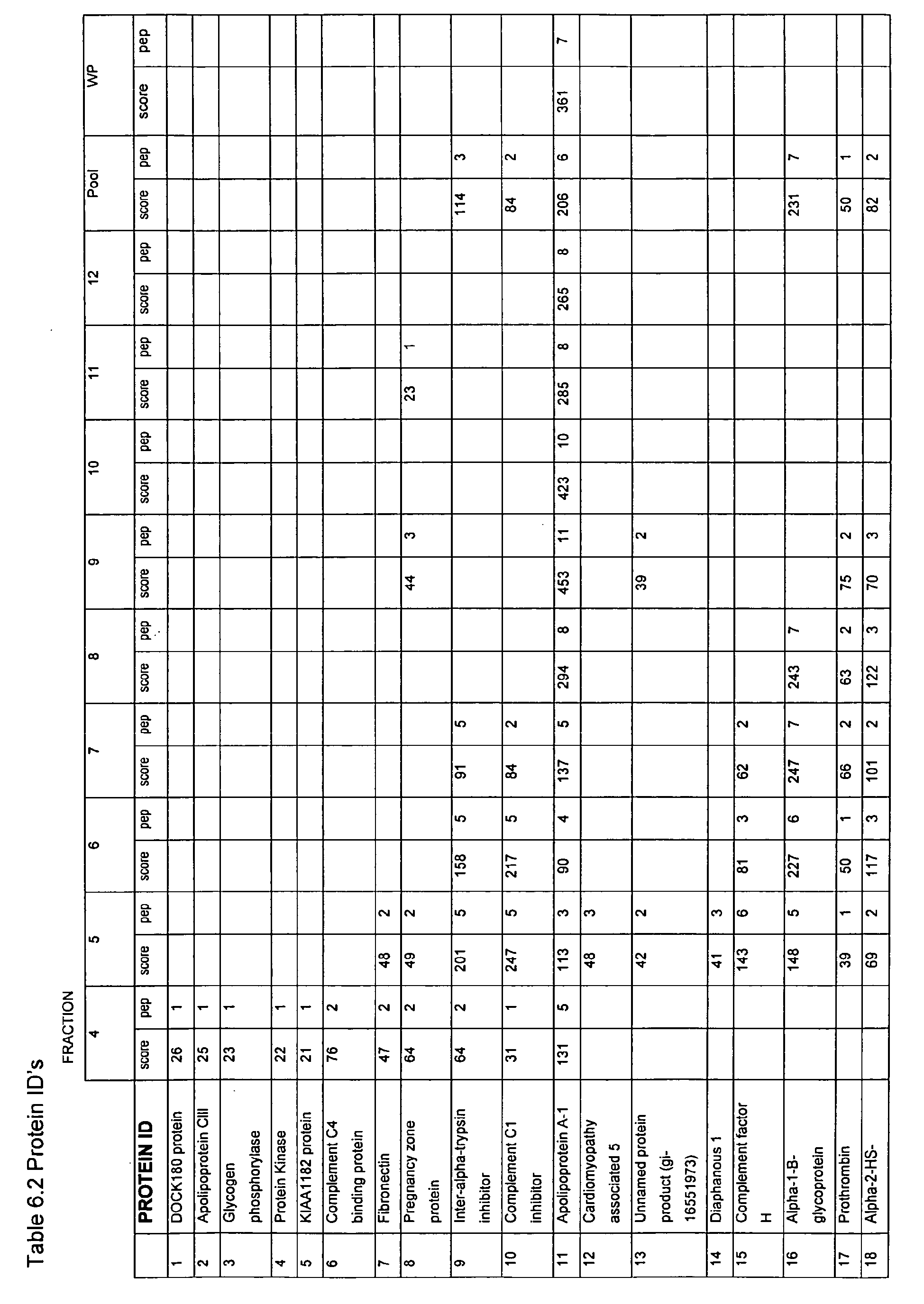

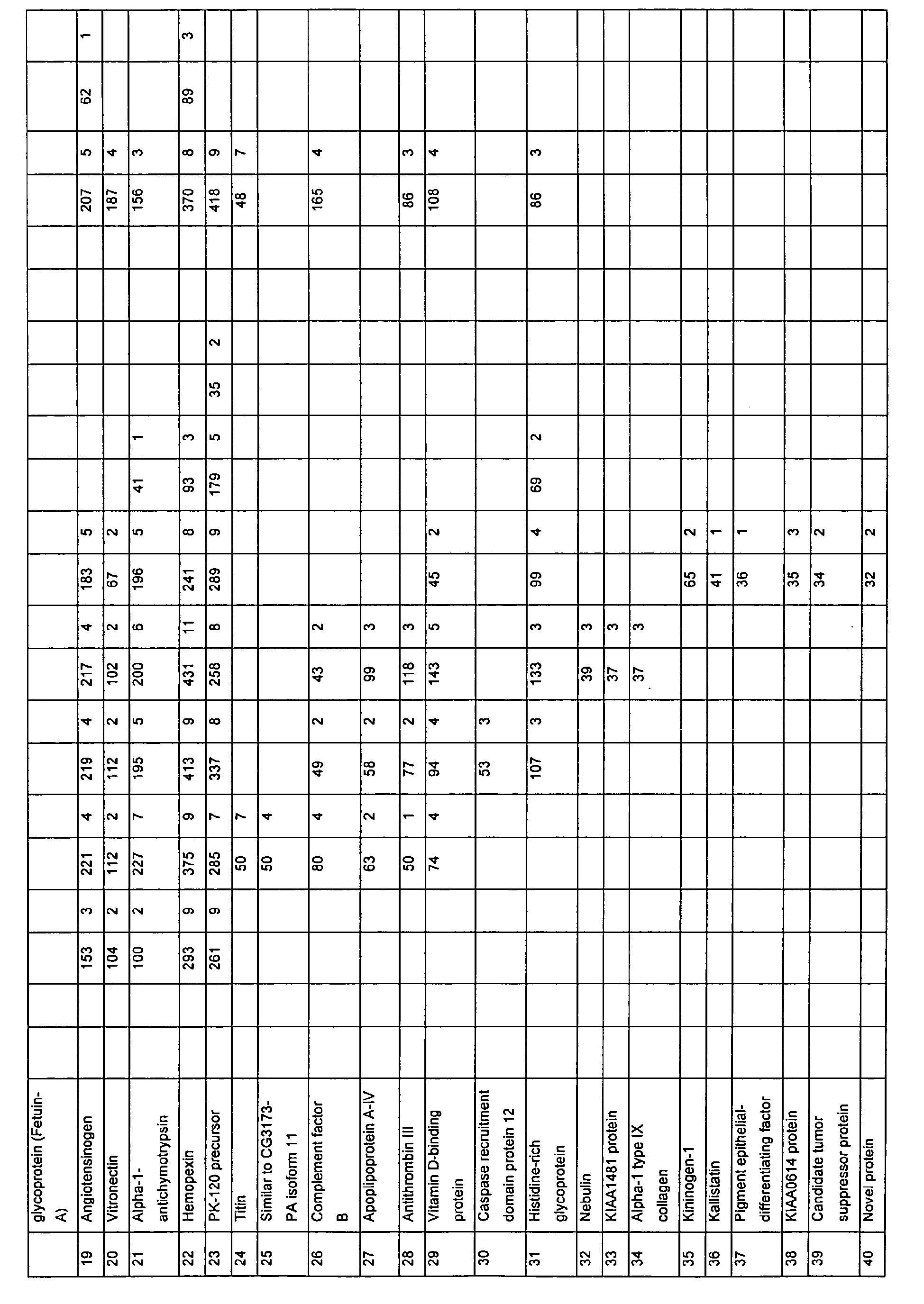

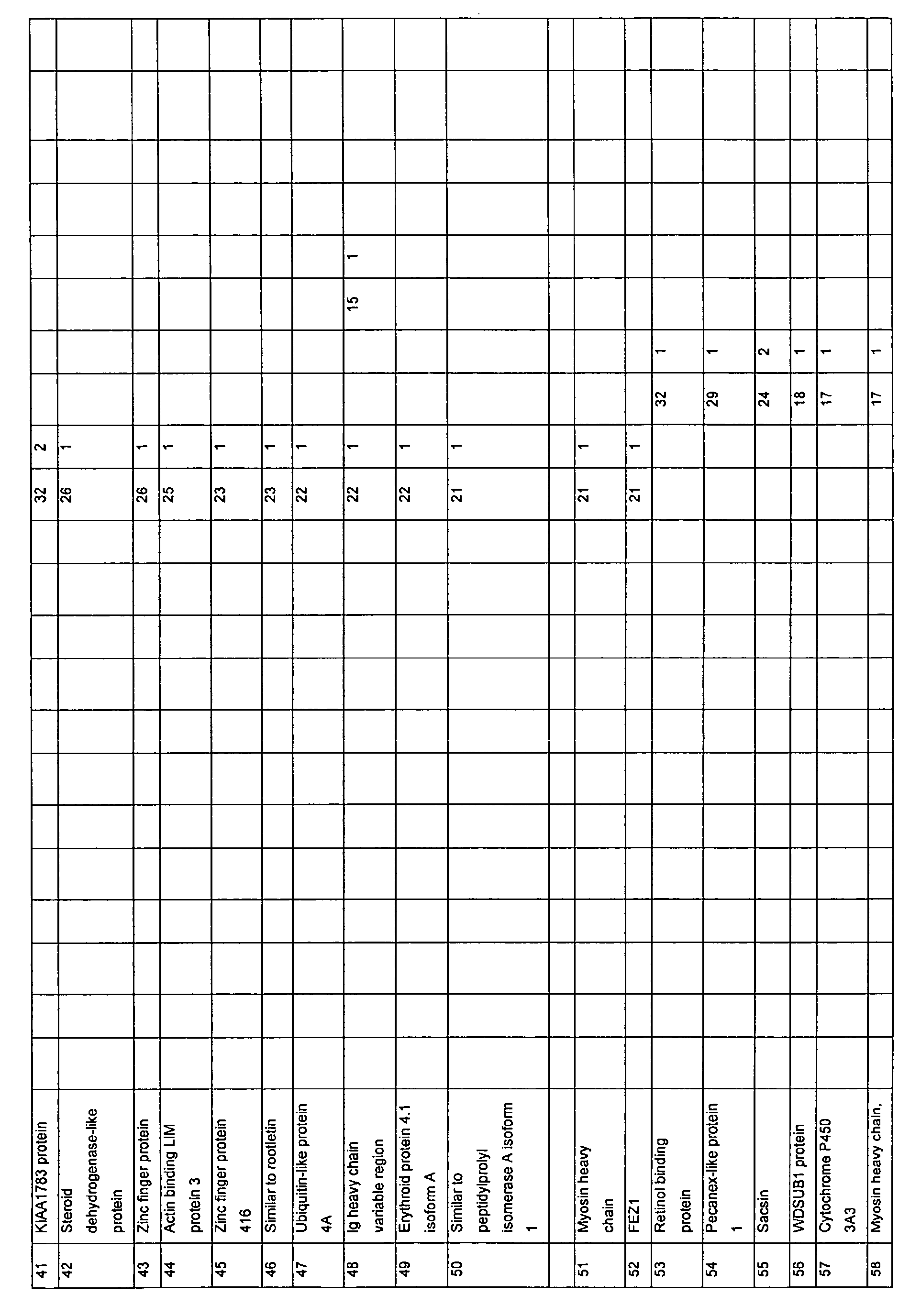

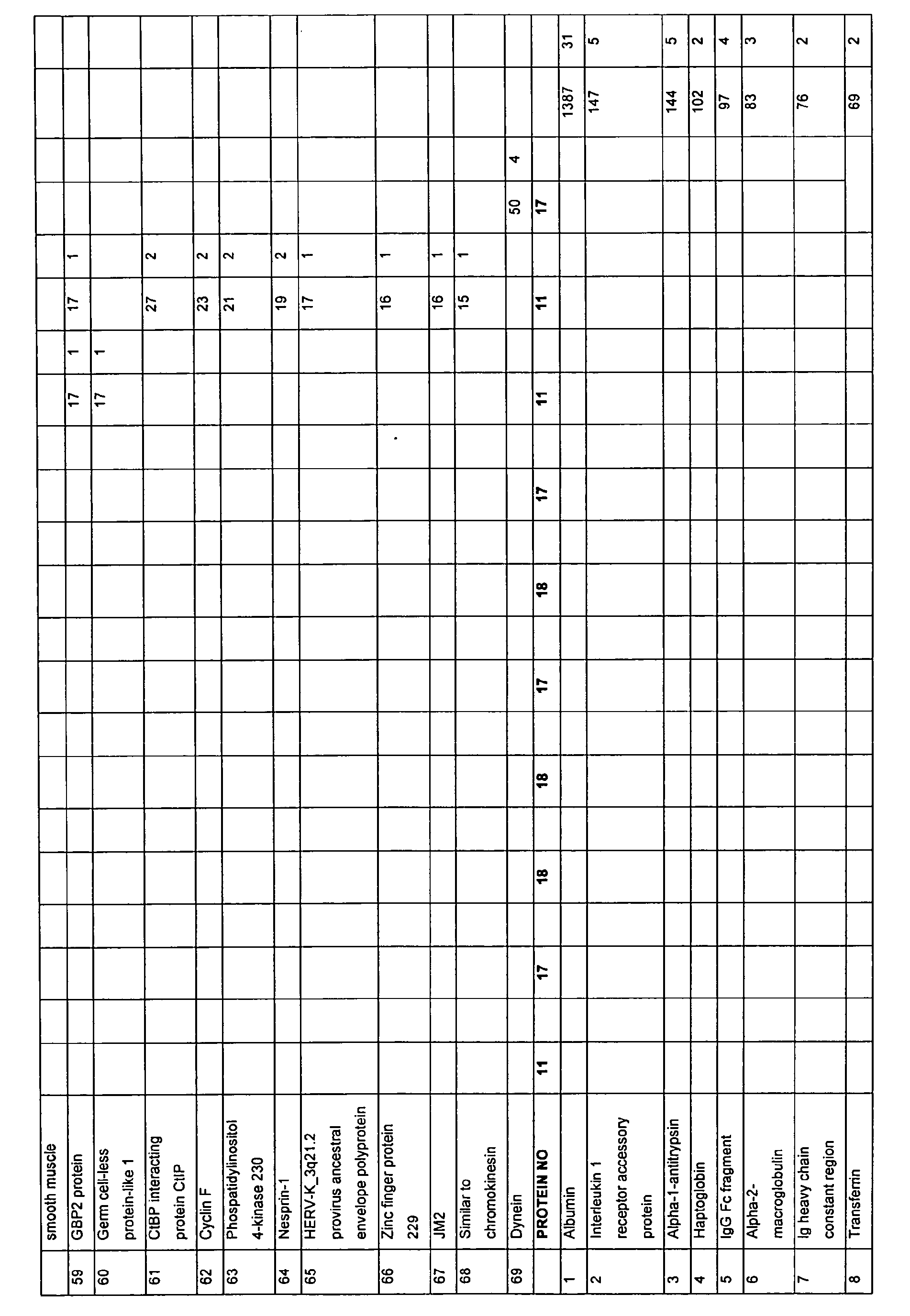

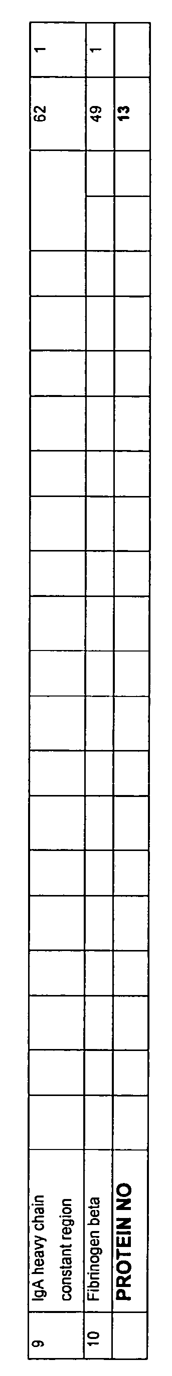

### D. Confirmation of depletion of the high abundance proteins

An ELISA assay was used to determine the percent depletion of the high abundance proteins from human plasma. Whole citrated plasma and depleted plasma samples were directly coated onto 96 well ELISA plates overnight following dilution in carbonate buffer (Product Code C3041). The plates were washed with TBS-Tween 20 and then incubated with 20 primary antibodies in TBS-BSA for 2 hr at 37 °C. The plates were washed and then incubated with HRP-conjugated secondary antibodies in TBS-BSA for 2 hr at 37 °C. The plates were washed and developed with TMB substrate (Product Code T0440), stopped with an equal volume of 1 M HCl and the absorption was measured at 450 nm.

As shown in Figure 13, 17 of the 20 high abundance proteins were removed with an average depletion of 99.8%, and three apoliproteins were removed with an average depletion of 95.5% from 100 µl of plasma. Thus, this antibody resin displays high depletion capacity for 20 of the most abundant proteins in human plasma. Furthermore, this resin provides a size exclusion fractionation of the depleted plasma, which increases the number of proteins that can be identified in the depleted plasma.

## Claims

1. A composition comprising a plurality of heterogeneous epitope binding agents stochastically conjugated *en masse* to a solid support.

2. The composition of claim 1, wherein the composition further comprises a plurality of linkers, one or more linkers being disposed between an epitope binding agent and the solid support, the linkers coupling the epitope binding agents to the solid support.

3. The composition of claim 2, wherein each linker is substantially hydrophilic.

4. The composition of claim 2, wherein each linker is substantially uncharged along the entire length of the linker.

5. The composition of any of claims 2 to 4, wherein each linker is from about 5 to about 50 atoms in length.

6. The composition of any of claims 2 to 5, wherein each linker comprises substantially carbon, hydrogen, and oxygen atoms.

7. The composition of claim 1, wherein the epitope binding agents are conjugated to the solid support by covalent bonding.

8. The composition of claim 7, wherein the density of epitope binding agents conjugated to the solid support comprises from about 5.0 mg/ml to about 20.0 mg/ml.

9. The composition of any preceding claim, wherein the epitope binding agents bind from about 2 to about 1000 distinct targets.

10. The composition of claim 9, wherein the target is selected from the group consisting of a protein, peptide, protein-containing complex, nucleic acid and small metabolite.

11. The composition of claim 9 or claim 10, wherein the target is derived from *Homo sapiens* or *H. sapiens* cells.

12. The composition of claim 9 or claim 10, wherein the target is derived from a species selected from the group consisting of mouse, rat, cow, Dictyostelium, Drosophila, zebrafish, dog, chicken, rhesus monkey, chimpanzee, yeast, Arabidopsis and rice.

13. The composition of any of claims 9 to 12, wherein the target is separated from a sample selected from the group consisting of whole blood, plasma, serum, cerebrospinal fluid, urine, tears, nipple aspirate, lactation fluids, vaginal fluids, semen, perspiration, faeces, peritoneal fluids, lavages, cell lysates, cell culture supernatants and cell culture lysates.

14. The composition of any preceding claim, wherein the epitope binding agents are selected from the group consisting of monoclonal antibodies, polyclonal antibodies, recombinant antibodies, single chain antibodies, peptide epitopes and antibody fragments.

15. The composition of claim 14, wherein the antibodies are a mixture of IgG's, IgY's, and single chain antibodies.

16. The composition of any preceding claim, wherein the solid support is selected from the group consisting of resins, beads, wells, emulsions, glass supports, silica supports, magnetic supports, powders, polymer supports, copolymer supports, nano-capillaries and other nano-materials.

17. The composition of claim 16, wherein the solid support is selected from the group consisting of agarose resin, agarose beads, silica resin, and silica beads.

18. The composition of any preceding claim, wherein the composition has a binding capacity from about 0.5 mg/ml to about 20 mg/ml.

19. A process for preparing a composition capable of separating at least two distinct targets from a sample comprising a mixture of targets and non-targets, the process comprising:
(a) combining a plurality of heterogeneous epitope binding agents to form an epitope binding agent mixture; and
(b) contacting the epitope binding agent mixture with a solid support under conditions such that the plurality of epitope binding agents are stochastically conjugated *en masse* to the solid support.

20. The process of claim 19, wherein the solid support is selected from the group consisting of resins, beads, emulsions, glass supports, silica supports, magnetic supports, powders, polymer supports, copolymer supports, nano-capillaries and other nano-materials.

21. The process of claim 18 or claim 19, wherein substantially each epitope binding agent is conjugated to the solid support via one or more linkers.

22. The process of claim 21, wherein each linker is substantially hydrophilic.

23. The process of claim 21, wherein each linker is substantially neutrally charged throughout the entire length of the linker.

24. The process of claim any of claims 21 to 23, wherein each linker is from about 5 to about 50 atoms in length.

25. The process of any of claims 21 to 24, wherein each linker comprises substantially carbon, hydrogen, and oxygen.

26. The process of any of claim 21 to 25, further comprising reacting the epitope binding agents with a substance that stochastically adds multiple sulfur-containing groups to each epitope binding agent prior to contacting the epitope binding agent mixture with the solid support.

27. The process of claim 26, wherein the sulfur-containing group is sulfhydryl.

28. The process of claim 26, wherein the substance comprises S-acetylthioglycolic acid NHS ester.

29. The process of claim 26 further comprising contacting the solid support with reagents under conditions such that a chemical moiety is added to the solid support that is capable of coupling to sulfhydryl groups on the epitope binding agents.

30. The process of claim 29, wherein the chemical moiety is formed by a process comprising:
(a) contacting the solid support with a first reagent under conditions such that from about 5 to about 50 atoms are added to the solid support to form a solid support having a substantially hydrophilic arm;
(b) contacting the solid support having a substantially hydrophilic arm with a second reagent that adds an amine group to the end of the arm; and
(c) contacting the product of (b) with a third reagent that forms the chemical moiety on the solid support that is capable of binding to sulfhydryl groups.

31. The process of claim 30, wherein the first reagent is 1,4 butanediol diglycidyl ether, the second reagent is ammonium hydroxide, and the third reagent is selected from bromoacetic acid NHS ester or maleimide spacer NHS ester.

32. The process of any of claims 19 to 31, further comprising iteratively selecting the relative concentration of each type of epitope binding agent present in the mixture.

33. The process of claim 32, wherein the concentration of each type of epitope binding agent present in the mixture is optimized based on the relative molar ratio of each target to be separated from the sample.

34. The process of claim 33, wherein the concentration of each epitope binding agent present in the mixture is iteratively selected by a process comprising:
(a) contacting a fixed volume of sample comprising targets and non-targets with various volumes of the composition of claim 1;
(b) separating the targets from the non-targets for all the various volumes used in step (a);
(c) determining the level of separation for the targets from non-targets for all the various volumes used in (a); and
(d) adjusting the concentration of each antibody based upon the level of target separation for each of the volumes of the composition of in step (a) and the desired level of target separation.

35. The process of any of claims 19 to 34, wherein the density of antibodies conjugated to the solid support comprises from about 5.0 mg/ml to about 20.0 mg/ml.

36. The process of any of claims 19 to 35, wherein the composition has a binding capacity from about 0.5 mg/ml to about 20.0 mg/ml.

37. The process of any of claims 19 to 36, wherein the epitope binding agent is an antibody.

38. A method for separating at least two distinct targets from a sample, the sample comprising a mixture of targets and non-targets, the method comprising contacting the sample with a composition as defined in any of claims 1 to 18, the epitope binding agents having affinity for the distinct targets such that when the epitope binding agents contact the targets they bind to the targets thereby separating the targets from the non-targets.

39. The method of claim 38, further comprising the step of simultaneously fractionating at least two distinct non-targets from the sample by collecting at least two fractions of the target-depleted sample, such that each fraction comprises at least one distinct non-target.

40. The method of claims 38 or 39, wherein the solid support is selected from the group consisting of resins, beads, wells, emulsions, glass supports, silica supports, polymer supports, copolymer supports, magnetic supports, powders, nano-capillaries, and other nano-materials.

41. The method of claims 38 or 39, wherein the solid support comprises resins, beads, or emulsions which are contained in a column.

42. The method of claims 40 or 41, wherein the beads are selected from the group consisting of agarose beads, polyacrylamide beads, polyacrylic beads, copolymer beads, silica beads, and magnetic beads.

43. The method of any of claims 38 to 42, wherein the fractions are collected during a procedure selected from the group consisting of spin-column chromatography, fast protein liquid chromatography (FPLC), low pressure liquid chromatography, and high performance liquid chromatography (HPLC).

44. The method of any of claims 38 to 43, wherein the sample is derived from a species selected from the group consisting of human, mouse, rat, cow, dog, cat, chicken, rhesus monkey, chimpanzee, zebrafish, Drosophila, Dictyostelium, yeast, Arabidopsis, and rice.

45. The method of any of claims 38 to 44, wherein the sample is selected from the group consisting of whole blood, serum, plasma, cerebrospinal fluid, tears, urine, feces, saliva, vaginal fluid, nipple aspirate, lactation fluid, semen, perspiration, faeces, peritoneal fluid, lavages, cell lysates, cell culture supernatants, and cell culture lysates.

46. The method of any of claims 38 to 45, wherein the target and the non-target are selected from the group consisting of proteins, peptides, protein-containing complexes, nucleic acids, and small metabolites.

47. The method of any of claims 38 to 46, wherein the epitope binding agents bind from about 5 to about 100 different targets.

48. The method of any of claims 38 to 47, wherein the epitope binding agents are antibodies.

49. The method of claim 48, wherein the antibodies are selected from the group consisting of monoclonal antibodies, polyclonal antibodies, recombinant antibodies, single chain antibodies, peptide epitopes, and antibody fragments.

50. The method of claims 48 or 49, wherein the antibodies are a mixture of IgG's, IgY's, and single chain antibodies.

51. The method of any of claims 38 to 50, wherein the sample is human plasma, the targets and the non-targets are proteins, the solid support comprises size exclusion agarose beads, such that the non-targets are separated on the basis of size, and the fractions of the target-depleted sample are collected during liquid chromatography.

52. A kit for separating a plurality of distinct targets from a sample comprising targets and non-targets, the kit comprising the composition of any of claims 1 to 18 and instruction for separation of the distinct targets from the sample.
